# EUROPEAN PATENT APPLICATION

(11) **EP 2 423 323 A1**
(43) Date of publication of application: **29.02.2012**
(21) Application number: 10767100.0
(22) Date of filing: 21.04.2010
(51) Int. Cl.: C12Q 1/68, C12N 15/09, C12Q 1/02

(54) **METHOD FOR EVALUATING CANCER**

(30) Priority: 21.04.2009 JP 2009103332
(71) Applicant: NEC Corporation, Tokyo 108-8001 (JP); TOKYO MEDICAL UNIVERSITY, Shinjyuku-ku Tokyo 160-8402 (JP)
(72) Inventor: KURODA, Masahiko, Tokyo 167-0052 (JP); SAITO, Akira, Tokyo 108-8001 (JP); SANO, Maki, Tokyo 108-8001 (JP)
(74) Representative: Glawe, Delfs, Moll
(86) International application number: PCT/JP2010/057102
(87) International publication number: WO 2010/123043

(57) **Abstract**

Provided is a cancer evaluation method using a novel cancer marker for evaluating the onset, the preclinical stage, the clinical stage, or the prognosis of a cancer in a subject. At least one miRNA selected from hsa-miR-92 and hsa-miR-494 is used as the novel cancer marker in cancer evaluation. The cancer marker in a sample of a cell or a tissue is detected, and the possibility of a cancer in the sample is evaluated based on the expression level of the cancer marker. According to this evaluation method, by detecting the miRNA as the cancer marker, it becomes possible to evaluate the possibility of a cancer in the sample with excellent reliability. As a method for detecting the cancer marker, it is preferable to perform an in situ hybridization method using a labeled probe with respect to the sample that has been immobilized, for example.

## Description

### Technical Field

The present invention relates to a method for evaluating the possibility of cancers by detecting a novel cancer marker.

### Background Art

In the field of clinical medical practice, it is required to easily determine the presence or absence of a disease, the degree of progression of the disease, the effect obtained after a treatment, etc. Under these circumstances, as a method for determining them indirectly, detecting a marker whose expression amount changes specifically accompanying the onset or progression of each disease has been proposed, and attempts actually are made to put this into practical use.

Among various diseases, detecting malignant tumors, which are so-called cancers, early and selecting and changing a treatment strategy therefor appropriately are particularly important. Thus, in order to realize indirect judgment by the detection of a marker as described above, various cancer markers have been reported. The cancer markers also are referred to as tumor markers. Specific examples of the cancer markers include PSA (Prostate-Specific Antigen), CEA (Carcinoembryonic Antigen), CA 19-9 (Carcinoembryonic Antigen 19-9), and CA 72-4 (Carcinoembryonic Antigen 72-4). Furthermore, it is described in Non-Patent Documents 1 and 2 that the expression of miRNAs such as has-mir-15, has-mir-16, miR-143, and miR-145 is downregulated in lymphocytic leukemia, colon cancer, and the like (Non-Patent Documents 1 and 2).

### [Citation List]

### [Non-Patent Document(s)]

[Non-Patent Document 1] Calin GA, Dumitru CD, Shimizu M et al., Frequent deletions and down-regulation of micro-RNA genes miR15 and miR16 at 13q14 in chronic lymphocytic leukemia, Proc Nat1 Acad Sci USA, 2002, vol. 99, pp.15524-9
[Non-Patent Document 2] Michael MZ, SM OC, van Holst Pellekaan NG, Young GP, James RJ, Reduced accumulation of specific microRNAs in colorectal neoplasia, Mol Cancer Res, 2003, vol. 1, pp. 882-91

### Brief Summary of the Invention

### Problem to be Solved by the Invention

However, in the field of clinical medical practice, cancer markers with which the onset of cancers and their progression can be determined with excellent reliability are necessary. Therefore, there still is a demand for the provision of a novel cancer marker. Thus, with the foregoing mind, it is an object of the present invention to provide an evaluation method using a novel cancer marker for evaluating a cancer, and an evaluation reagent to be used in the evaluation method.

### Means for Solving Problem

The present invention provides an evaluation method for evaluating the possibility of a cancer, including the steps of: detecting a cancer marker in a sample; and evaluating the possibility of a cancer in the sample based on an expression level of the cancer marker. In this evaluation method, the sample is a cell or a tissue, and the cancer marker includes at least one miRNA selected from hsa-miR-92 and hsa-miR-494.

### Effects of the Invention

The inventors of the present invention conducted a diligent study, and as a result, they found that the expression levels of hsa-miR-92 and hsa-miR-494 in a cell or a tissue change accompanying the development of cancers, thereby achieving the present invention. According to the evaluation method of the present invention, by detecting the expression level of at least one of the above-described miRNAs in a sample, it is possible to determine the presence or absence of cancer development or cancer progression with excellent reliability, for example. Furthermore, a significant difference in the expression of these miRNAs is seen between negative and positive for canceration, for example. Thus, according to the present invention, it becomes possible to detect cancers easily at an initial stage whereas such detection is difficult by general palpation and the like. Still further, by associating the evaluation method of the present invention with, for example, cancer evaluation utilizing conventional HE staining or the like, cancer evaluation with still higher reliability becomes possible.

### Brief Description of Drawings

[FIG. 1] FIG. 1 is a block diagram showing a cancer pathological image diagnosis support system according to Embodiment 1B of the present invention.
[FIG. 2] FIG. 2 illustrates the contents of a stained image database in Embodiment 1B of the present invention.
[FIG. 3] FIG. 3 is a flowchart illustrating an example of the operation of the cancer pathological image diagnosis support system according to Embodiment 1B of the present invention, which is shown in FIG. 1.
[FIG. 4] FIG. 4 is a flowchart illustrating another example of the operation of the cancer pathological image diagnosis support system according to Embodiment 1B of the present invention, which is shown in FIG. 1.
[FIG. 5] FIG. 5 is a flowchart illustrating still another example of the operation of the cancer pathological image diagnosis support system according to Embodiment 1B of the present invention, which is shown in FIG. 1.
[FIG. 6] FIG. 6 is a flowchart illustrating yet another example of the operation of the cancer pathological image diagnosis support system according to Embodiment 1B of the present invention, which is shown in FIG. 1.
[FIG. 7] FIG. 7 is a block diagram showing a cancer pathological image diagnosis support system according to Embodiment 1C of the present invention.
[FIG. 8] FIG. 8 is a flowchart illustrating an example of the operation of the cancer pathological image diagnosis support system according to Embodiment 1C of the present invention, which is shown in FIG. 7.
[FIG. 9] FIG. 9 is a flowchart illustrating another example of the operation of the cancer pathological image diagnosis support system according to Embodiment 1C of the present invention, which is shown in FIG. 7.
[FIG. 10] FIG. 10 is a flowchart illustrating still another example of the operation of the cancer pathological image diagnosis support system according to Embodiment 1C of the present invention, which is shown in FIG. 7.
[FIG. 11] FIG. 11 is a block diagram showing a cancer pathological image diagnosis support system according to Embodiment 1D of the present invention.
[FIG. 12] FIG. 12 is a block diagram showing a cancer pathological image diagnosis support system according to Embodiment 1E of the present invention.
[FIG. 13] FIG. 13 is a block diagram showing the configuration of a system that performs a feature selection method according to Embodiment 2A of the present invention.
[FIG. 14] FIG. 14 is a flowchart illustrating a feature selection method according to Embodiment 2B of the present invention.
[FIG. 15] FIG. 15 is a flowchart showing an example of processing to create a category table in Embodiment 2B of the present invention.
[FIG. 16] FIG. 16 shows an example of a category table created in Embodiment 2B of the present invention.
[FIG. 17] FIG. 17 is a block diagram showing the configuration of a system to conduct diagnosis in Embodiment 2B of the present invention.
[FIG. 18] FIG. 18 is a flowchart showing an example of processing to extract subimages in Embodiment 2B of the present invention.
[FIG. 19] FIG. 19 is a block diagram showing the configuration of a cancer pathological image diagnosis support system according to Embodiment 1A of the present invention.
[FIG. 20] FIG. 20 is a block diagram showing the cancer pathological image diagnosis support system according to Embodiment 1A of the present invention.
[FIG. 21] FIG. 21 is a flowchart illustrating an example of the operation of the cancer pathological image diagnosis support system according to Embodiment 1A of the present invention, which is shown in FIG. 20.
[FIG. 22] FIG. 22 shows photographs showing the results of miRNA staining of leukocyte cells in Example 1 of the present invention.
[FIG. 23] FIG. 23 shows photographs showing the results of miRNA staining of breast tissues in Example 2 of the present invention.
[FIG. 24] FIG. 24 shows photographs showing the results of miRNA staining of hepatocytes in Example 3 of the present invention.

### Mode for Carrying out the Invention

The meanings of the respective terms used in the present invention are as follows. The term "cancer" generally means malignant tumor. The term "canceration" generally means the onset of a cancer and also encompasses "malignant transformation". Regarding the term "onset", for example, the time point at which one is diagnosed as having a specific disease through synthetic judgment based on disease-specific clinical symptoms, test data, or the like is referred to as the onset of the disease. The term "preclinical stage" generally refers to a condition before the onset of a disease where disease-specific clinical symptoms have not appeared yet but in an early stage of the disease in which a trace amount of malignant tumor cells are present already. The term "prognosis" means, for example, a course of a disease after the treatment of the disease, such as operation. Since the cancer marker used in the present invention can provide useful information for predicting prognosis, foreseeing the course of a disease, and selecting an appropriate treatment method, for example, it also can be referred to as a "prognostic factor". The "stage of cancer progression" can be determined as appropriate based on the kind of cancerous tissues or the like, for example. In general, Stage 0 and Stage I can be classified as an initial cancer, Stage 11 can be classified as an early cancer, and Stage III and Stage IV can be classified as an advanced cancer.

In the present invention, "the possibility of a cancer" encompasses the possibility that the subject may develop a cancer, whether or not canceration has occurred, the stage of cancer progression such as a preclinical stage or a clinical stage, a prognosis state, or the like, for example.

### <Cancer Marker>

The cancer marker miRNA in the present invention is, as described above, at least one miRNA selected from hsa-miR-92 and hsa-miR-494. Hereinafter, the cancer marker also is referred to as the "cancer marker miRNA".

In the present invention, the cancer marker miRNA may be a single strand (monomer) or a double strand (dimer), for example. Furthermore, in the present invention, the cancer marker miRNA may be immature miRNA or mature miRNA, for example. Examples of the immature miRNA include primary transcript initial miRNA (pri-miRNA) and precursor miRNA (pre-miRNA). The pri-miRNA has a hairpin loop structure as a result of intramolecular binding. The pri-miRNA is cleaved with Drosha, whereby it is converted into the short pre-miRNA having a stem-loop structure. Hereinafter, the pre-miRNA also is referred to as stem-loop miRNA. The pre-miRNA is cleaved with Dicer, whereby a still shorter double-stranded RNA (miRNA-miRNA*) is generated. This double-stranded RNA is unwound on RISC, whereby two single-stranded RNAs are generated. The single-stranded RNAs are mature miRNAs. Hereinafter, one of the mature miRNAs is referred to as functional miRNA, and the other is referred to as Minor miRNAs*.

In the present invention, the cancer marker miRNA is not particularly limited, and preferably is stem-loop miRNA or mature miRNA, particularly preferably mature miRNA.

Examples of hsa-miR-92 include hsa-miR-92a and hsa-miR-92b.

hsa-miR-92a may be either immature miRNA or mature miRNA, as described above. Hereinafter, the former also is referred to as immature hsa-miR-92a, and the latter also is referred to as mature hsa-miR-92a.

Examples of the immature hsa-miR-92a include hsa-miR-92a-1 and hsa-miR-92a-2, and the immature hsa-miR-92a may be either one of them. hsa-miR-92a-1 and hsa-miR-92a-2 are stem-loop miRNAs. Hereinafter, the former also is referred to as stem-loop hsa-miR-92a-1, and the latter also is referred to as stem-loop hsa-miR-92a-2. They are transcription products derived from different genomic regions, but the sequences of them in their mature forms are identical. The sequence of the stem-loop hsa-miR-92a-1 is registered under Accession No. MI0000093, for example, and the sequence of the stem-loop hsa-miR-92a-2 is registered under Accession No. MI0000094, for example.

The mature hsa-miR-92a may be a functional miRNA, for example, and the sequence thereof is registered under Accession No. MIMAT0000092, for example. The sequence of this functional hsa-miR-92a is shown in SEQ ID NO: 1 below.
Functional hsa-miR-92a (SEQ ID NO: 1)
   5'-uauugcacuugucccggccugu-3'

Other examples of the mature hsa-miR-92a include Minor miRNA*. Examples of Minor miRNA* include hsa-miR-92a-1* and hsa-miR-92a-2*. The sequence of hsa-miR-92a-1* is registered under Accession No. MIMAT0004507, for example. This sequence is shown in SEQ ID NO: 6. The sequence of hsa-miR-92a-2* is registered under Accession No. MIMAT0004508. This sequence is shown in SEQ ID NO: 7.
Minor hsa-miR-92a-1* (SEQ ID NO: 6)
   5'-agguugggaucgguugcaaugcu-3'
Minor hsa-miR-92a-2* (SEQ ID NO: 7)
   5'-ggguggggauuuguugcauuac-3'

Although hsa-miR-92b is a transcription product derived from a different genomic region from that of hsa-miR-92a, the seed sequence of hsa-miR-92b is similar to that of hsa-miR-92a. Thus, hsa-miR-92b can be used as a cancer marker, similarly to hsa-miR-92a. hsa-miR-92b may be either immature miRNA or mature miRNA, as described above. Hereinafter, the former also is referred to as immature hsa-miR-92b, and the latter also is referred to as mature hsa-miR-92b.

Among various kinds of immature hsa-miR-92b, stem-loop miRNA hereinafter also is referred to as stem-loop hsa-miR-92b. The sequence of the stem-loop hsa-miR-92b is registered under Accession No. MI0003560, for example.

The mature hsa-miR-92b may be a functional miRNA, for example, and the sequence thereof is registered under Accession No. MIMAT0003218. The sequence of this functional hsa-miR-92b is shown in SEQ ID NO: 3.
Functional hsa-miR-92b (SEQ ID NO: 3)
   5'-uauugcacucgucccggccucc-3'

Other examples of the mature hsa-miR-92b include Minor miRNA*. Examples of Minor miRNA* include hsa-miR-92b*. Although hsa-miR-92b* is a transcription product derived from a different genomic region from that of hsa-miR-92a-1* or hsa-miR-92a-2*, the seed sequence thereof is similar to that of hsa-miR-92a-1* or hsa-miR-92a-2*. Thus, hsa-miR-92b* can be used as a cancer marker, similarly to hsa-miR-92a-1* or hsa-miR-92a-2*. The sequence of hsa-miR-92b* is registered under Accession No. MIMAT0004792, for example. This sequence is shown in SEQ ID NO: 4.
Minor hsa-miR-92b* (SEQ ID NO: 4)
   5'-agggacgggacgcggugcagug-3'

hsa-miR-494 may be either immature miRNA or mature miRNA, as described above. Hereinafter, the former also is referred to as immature hsa-miR-494, and the latter also is referred to as mature hsa-miR-494.

Examples of the immature hsa-miR-494 include stem-loop miRNA. Hereinafter, this also is referred to as stem-loop hsa-miR-494. The sequence of the stem-loop hsa-miR-494 is registered under Accession No. MI0003134, for example.

Examples of the mature hsa-miR-494 include functional miRNA, and the sequence thereof is registered under Accession Mo. MIMAT0002816, for example. The sequence of this functional hsa-miR-494 is shown in SEQ ID NO: 2.
Functional hsa-miR-494 (SEQ ID NO: 2)
   5'-ugaaacauacacgggaaaccuc-3'

As disclosed in the documents listed below, the 5' end and the 3' end of each of the miRNAs respectively have some variations, for example. Therefore, each of the miRNAs in the present invention also encompasses variants each having a sequence different from the sequence thereof in its mature form by a few bases.
Wu H. et al., 2007, PLoS ONE 2 (10): e1020 miRNA profiling of naive, effector and memory CD8 T cells.
Pablo Landgraf et al., 2007, Cell, vol. 129, pp. 1401-1414 A Mammalian microRNA Expression Atlas Based on Small RNA Library Sequencing.
Neilson et al., 2007, Genes Dev, vol. 21, pp. 578-589 Dynamic regulation of miRNA expression in order to stage of cellular development.
Ruby et al., 2006, Cell, vol. 127, pp. 1193-1207 Large-scale sequencing reveals 21U-RNAs and additional microRNAs and endogeneous siRNAs in C. elegans.
Obemoster et al., RNA 2006 12: pp. 1161-1167 Post-transcriptional regulation of microRNA expression.
Lagos-Quintana et al., 2002, Curr Biol, vol. 12, pp. 735-739 Identification of tissue-specific microRNAs from mouse.

The cancer marker miRNAs in the present invention encompass, for example, polynucleotides having a base sequence with a homology to the base sequences of the respective sequence identification numbers, and polynucleotides having base sequences complementary thereto. The "homology" refers to the degree of identity between sequences to be compared with each other when they are aligned appropriately, and represents the occurrence ratio (%) of perfect match of nucleotides between these sequences. When it is described that the base sequence of a polynucleotide "has a homology" to the base sequences of the miRNAs of the present invention, it means that the polynucleotide is similar enough to the miRNAs to be able to maintain the function as the miRNAs of the present invention. The alignment can be achieved by using an arbitrary algorithm such as BLAST, for example. Even when the base sequences differ from each other by, for example, point mutation such as substitution, deletion, or addition, it can be said that they are homologous as long as such a difference does not affect the function of the miRNAs. The number of bases different between the base sequences is, for example, 1 to 20, 1 to 15, 1 to 10, 1 to 5, 1 to 3, 1 to 2, or 1. Furthermore, when base sequences of two polynucleotides to be compared with each other have an identity of, for example, 80%, 85%, 90%, 91%, 92%, 93%, 94%, 95%, 96%, 97%, 98%, 99%, or 100%, it can be said that they are homologous. Furthermore, for example, when one of the two polynucleotides hybridizes to a polynucleotide having a base sequence complementary to the other polynucleotide under stringent conditions, it can be said that the two polynucleotides are homologous. The stringent conditions are not particularly limited, and may be such that, for example, the two polynucleotides are kept at a temperature of "Tm(°C) - 25°C" overnight in a solution containing 6 × SSC, 0.5% SDS, 5 × Denhardt's solution, and 0.01% denatured salmon sperm nucleic acid.

### <Evaluation Method>

As described above, the evaluation method according to the present invention includes the steps of: detecting a cancer marker in a sample; and evaluating the possibility of a cancer in the sample based on an expression level of the cancer marker. In this evaluation method, the sample is a cell or a tissue, and the cancer marker includes at least one miRNA selected from hsa-miR-92 and hsa-miR-494. According to the present invention, for example, by detecting the cancer marker in a sample of a subject, it becomes possible to evaluate the possibility that the subject may develop a cancer, whether or not canceration has occurred, the stage of cancer progression such as a preclinical stage (initial stage) or a clinical stage, a prognosis state, or the like, for example. The evaluation method of the present invention also can be referred to as a method for evaluating whether or not a subject has a cancer, for example.

The cancer marker in the present invention is as described above. In the present invention, the cancer marker to be detected may be either one of hsa-miR-92 and hsa-miR-494, or may be both of them, for example.

When the cancer marker to be detected is hsa-miR-92, for example, it may be either one of mature hsa-miR-92 and immature hsa-miR-92, or may be both of them. The kinds of the mature hsa-miR-92 and immature hsa-miR-92 also are not limited, and any one kind of them, two kinds of them, or all the kinds of them may be used, for example.

When the cancer marker to be detected is mature hsa-miR-92a, it may be: any one of functional hsa-miR-92, Minor hsa-miR-92a-1*, and Minor hsa-miR-92a-2*; any two of them; or all of them, for example. When the cancer marker to be detected is immature hsa-miR-92a, it may be either one of stem-loop hsa-miR-92a-1 and stem-loop hsa-miR-92a-2, or both of them, for example. When the cancer marker to be detected is mature hsa-miR-92b, it may be either one of functional hsa-miR-92b and Minor hsa-miR-92b*, or both of them, for example.

When the cancer marker to be detected is hsa-miR-494, it may be either one of mature hsa-miR-494 and immature hsa-miR-494, or may be both of them, for example. The kinds of the mature hsa-miR-494 and immature hsa-miR-494 also are not limited, and any one kind of them, two kinds of them, or all the kinds of them may be used, for example. In the case of mature hsa-miR-494, examples thereof include functional hsa-miR-494.

The present invention is **characterized in that** the expression level of the cancer marker is detected, as described above. The method for detecting the cancer marker is by no means limited, and a known method can be used. The detection method preferably is a method including visualization of the cancer marker, for example. The method for achieving the visualization is not particularly limited, and the visualization preferably is achieved by color development, fluorescence, autoradiography, or the like, for example. When the cancer marker is visualized by color development, the cancer marker can be detected by visual observation, absorbance measurement, image processing, or the like, for example. When the cancer marker is visualized by fluorescence, the cancer marker can be detected by visual observation, fluorescence intensity measurement, image processing, or the like, for example. Hereinafter, visualization of the cancer marker miRNA by color development or fluorescence also is referred to as miRNA staining. When the cancer marker is visualized by, for example, autoradiography, the cancer marker miRNA can be detected by visual observation of the autoradiograph, image processing of the autoradiograph, or the like, for example.

In the present invention, cancers to be evaluated are not particularly limited. Examples of the cancers include, as described above, colon cancer, rectal cancer, gallbladder cancer, stomach cancer, breast cancer, leukemia, pancreas cancer, liver cancer, brain tumor, and osteosarcoma.

In the present invention, the sample is a biological sample, and is not particularly limited as long as it is a cell or a tissue. Specific examples of the sample include: tissues and cells of large intestine, rectum, gallbladder, stomach, breast, blood cells, liver, brain, bone, and the periphery of bone; and cells in blood, such as leukocytes.

In the present invention, a subject from which the sample is derived is not particularly limited, and may be a human, for example. Other examples of the subject include nonhuman mammals including: primates excluding humans; rodents; dogs; and cats.

In the present invention, the cancer marker miRNA in the sample may be detected directly from the sample, or may be detected indirectly from RNAs collected from the sample, for example. According to the present invention, the region where the cancer marker is expressed in the sample can be specified, for example. Thus, it is preferable to detect the cancer marker directly from the sample.

First, an example of a method for detecting the cancer marker miRNA in the sample using RNAs collected from the sample will be described below.

The method for collecting RNAs from the sample is not particularly limited, and a known method can be employed. Specifically, a guanidine-CsCl ultracentrifugation method, AGPC (Acid Guanidinium-Phenol-Chloroform), or the like can be used. Also, it is possible to use a commercially available reagent or kit.

When RNAs are collected from the sample, it is also possible to detect the cancer marker miRNA indirectly by synthesizing cDNAs using the collected RNAs as templates and then detecting cDNA of the cancer marker miRNA therefrom, for example.

When the cDNAs synthesized using the RNAs as the template are used, detection of the cancer marker miRNA can be performed utilizing a nucleic acid amplification method, for example. The nucleic acid amplification method is not particularly limited, and examples thereof include a polymerase chain reaction (PCR) method, a reverse transcription PCR (RT-PCR) method, a real-time PCR method, and a real-time RT- PCR method. Among them, the real-time RT- PCR method is preferable.

When the nucleic acid amplification method is utilized, for example, first, total RNAs are extracted from the sample, and cDNAs are synthesized with the total RNAs as templates using random primers. Next, with the thus-obtained cDNAs as templates, an amplification reaction is caused using a primer that can amplify cDNA of the target cancer marker miRNA, and the amplification product is detected. By detecting the presence or absence of the amplification product or the amount of the amplification product, it is possible to detect the expression level of the cancer marker miRNA in the sample, i.e., the presence or absence of the expression of the cancer marker miRNA or the amount of the cancer marker miRNA expressed in the sample.

The random primers to be used in the reaction of synthesizing the cDNAs are not particularly limited, and commercially available random primers can be used, for example. Furthermore, the primer to be used in the amplification reaction is by no means limited, and it may be, for example a primer that can hybridize to the cDNA of the cancer marker miRNA or a sequence complementary thereto, or a primer that can hybridize to cDNA of a peripheral region of the cancer marker miRNA or a sequence complementary thereto. The primer can be designed as appropriate based on the base sequence of the cancer marker miRNA and common general technical knowledge, for example. Specifically, the primer may be, for example, a primer composed of the cDNA of the target cancer marker miRNA or a sequence complementary thereto or a primer composed of cDNA of a peripheral region of the cancer marker miRNA or a sequence complementary thereto. It is preferable that the sequence of the primer is at least about 70% complementary to, for example, the cDNA of the target cancer marker miRNA or a sequence complementary thereto or cDNA of a peripheral region of the cancer marker miRNA or a sequence complementary thereto, preferably at least 80% complementary to the same, more preferably at least 90% complementary to the same, still more preferably 95% complementary to the same, and particularly preferably 100% complementary to the same, for example.

The constitutional unit of the primer is not particularly limited, and known constitutional units can be employed. Specific examples of the constitutional units include nucleotides such as deoxyribonucleotide and ribonucleotide. Examples of the constitutional units also include PNA (Peptide Nucleic Acid) and LNA (Locked Nucleic Acid). Bases in the constitutional unit are not particularly limited. The constitutional unit may include natural bases (inartificial base) such as A, C, G, T, and U, or may include unnatural bases (artificial bases), for example. The length of the primer is not particularly limited, and may be a general length.

The method for detecting the amplification product is not particularly limited, and a known method can be employed. When the amplification product is detected in real time, it is preferable to cause a fluorescent reagent to be present in a reaction solution of the amplification reaction, for example. Examples of the fluorescent reagent include: a fluorescent substance that specifically binds to a double-stranded nucleic acid; and a fluorescent substance that intercalates into a double-stranded nucleic acid. In the amplification reaction, when a double-stranded nucleic acid is formed by extension from the primer that has annealed to the template cDNA, the fluorescent substance present in the reaction solution binds to or intercalates into the double-stranded nucleic acid. Then, by checking the fluorescence of the fluorescent substance that has bound to or intercalated into the double-stranded nucleic acid, it is possible to check the presence or absence of the amplification product, whereby the presence or absence of the target cancer marker miRNA can be checked indirectly. Furthermore, by measuring the fluorescence intensity of the fluorescent substance, the amplification product can be quantified, whereby the target cancer marker miRNA can be quantified indirectly. Examples of the fluorescent reagent include SYBR (trademark) Green. The detection using the fluorescent reagent can be carried out by a known method, for example. Specifically, in the case of real-time RT-PCR, the detection can be carried out using, for example, a commercially available reagent such as SYBR (trademark) Green PCR Master Mix (trade name, Perkin-Elmer Applied Biosystems) and a commercially available detector such as ABI Prism 7900 Sequence Detection System (trade name, Perkin-Elmer Applied Biosystems) in accordance with their manuals.

When the amplification product is detected in real time, other examples of the method for detecting the amplification product include causing a labeled probe to be present in a reaction solution of the amplification reaction. The labeled probe may be a probe having a fluorescent substance and a quencher, for example. Specific examples of such a probe include TaqMan (trademark) probes and cycling probes to be used with RNase. When the labeled probe is present alone, for example, fluorescence of the fluorescent substance is quenched by the quencher. When the labeled probe forms a double-stranded nucleic acid, the quenching effect is removed, whereby fluorescence is emitted. Such a labeled probe can be used according to a known method, for example.

Also, it is possible to detect the cDNA of the cancer marker miRNA using a probe. The probe may be, for example, a primer that can hybridize to the cDNA of the cancer marker miRNA or a sequence complementary thereto. In this method, hybridization is caused between the cDNA of the cancer marker and the probe, and the probe that has hybridized to the cDNA of the cancer marker is detected. The presence or absence or the amount of the probe that has hybridized to the cDNA of the cancer marker corresponds to the presence or absence or the amount of the cancer marker miRNA in the RNAs collected from the sample. Thus, by detecting the probe, it is possible to detect the presence or absence or the amount of the cancer marker miRNA in the sample indirectly. The detection of the probe can be carried out by a known method, which may be the same as a method to be described below, for example.

Furthermore, when RNAs are collected from the sample, for example, the cancer marker miRNA may be detected directly from the collected RNAs. In this case, the detection method may be a hybridization method using a probe, for example. As the probe, for example, a probe that can specifically hybridize to the cancer marker miRNA can be used. In this method, hybridization is caused between the cancer marker miRNA and the probe, and the probe that has hybridized to the cancer marker miRNA is detected. The presence or absence or the amount of the probe that has hybridized to the cancer marker corresponds to the presence or absence or the amount of the cancer marker miRNA in the RNAs collected from the sample. Thus, by detecting the probe, it is possible to detect the presence or absence or the amount of the cancer marker miRNA in the sample indirectly.

The method for detecting the probe is not particularly limited. A specific example of the detection method is such that, for example, a labeled probe labeled with a labeling substance is used as the probe, and the detection can be carried out by detecting the labeling substance.

As the labeling substance, a substance detectable *per se* can be used, for example. The substance may be, for example, a color-developing substance, a fluorescent substance that emits fluorescence, a radioactive material, or the like. In the case of the color-developing substance, for example, the presence or absence and the amount of the color-developing substance can be determined based on the presence or absence and the intensity of the color development. The color-developing substance may be, for example: a substance that develops color *per se*; a substance that releases a substance that develops color by an enzyme reaction or the like; or a substance that turns into a substance that develops color by an enzyme reaction or an electron transfer reaction. In the case of the fluorescent substance, for example, the presence or absence and the amount of the fluorescent substance can be determined based on the presence or absence and the intensity of the fluorescence. The fluorescent substance may be, for example: a substance that emits fluorescence *per se*; a substance that releases a substance that emits fluorescence by an enzyme reaction or the like; or a substance that turns into a substance that emits fluorescence by an enzyme reaction or an electron transfer reaction. In the case of the radioactive material, for example, the presence or absence and the amount of the labeling substance can be determined by measuring the radiation level with a scintillation counter or based on the presence or absence and the color density of an image obtained by autoradiography. Examples of the hybridization method using a probe labeled with the radioactive material include Northern blotting and microarray analysis.

The labeling substance may be, for example, a labeling substance detectable with another reagent. Examples of such a labeling substance include enzymes such as alkaline phosphatase (AP) and horseradish peroxidase (HRP). When the labeling substance is an enzyme, for example, a substrate that develops color or emits fluorescence through a reaction with the enzyme, electron transfer accompanying the enzyme reaction, or the like may be added as the above-described another reagent, and the presence or absence of color development or fluorescence by the reaction with the enzyme, the absorbance, or the fluorescence intensity may be detected. The substrate is not particularly limited, and can be set as appropriate depending on the kind of the enzyme or the like. Specific examples are as follows: in the case of AP, for example, bromo-chloro-indolyl-phosphate (BCIP), combination of the BCIP and nitroblue tetrazolium (NBT), or the like can be used; and in the case of HRP, for example, 3,3'-diaminobenzidine tetrahydrochloride (DAB) or the like can be used.

Other examples of the labeling substance detectable with another reagent include biotin and avidin. Among them, biotin is preferable. When the probe is labeled with biotin, it is preferable to add, as the above-described another reagent, the above-described enzyme, color-developing substance, fluorescent substance, radioactive material, or the like each having avidin bound thereto for example. Since biotin as the labeling substance of the probe binds to avidin, the enzyme or the like bound to avidin may be detected by any of the above-described methods. Furthermore, the above-described another reagent may be, for example, a complex of avidin and biotin with any of the enzymes and the like, which is a so-called avidin-biotin complex. This method is a so-called ABC (avidin-biotin complex) method. When such complexes are used, for example, avidin in a certain complex can bind to biotin that is present in another complex, so that it is possible to increase the number of molecules of the enzyme or the like that bind to a single molecule probe. Thus, detection with still higher sensitivity becomes possible. The biotin may be a biotin derivative, for example, and the avidin may be an avidin derivative such as streptavidin, for example.

Furthermore, examples of the method for detecting the probe that has hybridized to the cancer marker miRNA include a method in which a labeled probe labeled with an antigen is used as the probe and an antigen-antibody reaction is utilized. A specific example of such a method is a method that uses, in addition to the antigen-labeled probe, a labeled primary antibody that can specifically binds to the antigen and is labeled with a labeling substance. Another example is a method that uses a primary antibody that can specifically binds to the antigen and a labeled secondary antibody that can specifically binds to the primary antibody and is labeled with a labeling substance.

When the labeled primary antibody in the former is used, for example, first, hybridization is caused between the cancer marker miRNA and the antigen-labeled probe. Subsequently, to the antigen-labeled probe that has bound to the cancer marker miRNA, the labeled primary antibody is bound via the antigen. Then, the labeling substance of the labeled primary antibody bound to the probe is detected. In this manner, the probe that has hybridized to the cancer marker miRNA can be detected, whereby the cancer marker miRNA can be detected indirectly. The kind of the antigen labeling the probe is not particularly limited, and examples thereof include digoxigenin (DIG). The primary antibody is not particularly limited, and can be set as appropriate depending on the kind of the antigen, for example. When the antigen is DIG, for example, an anti-DIG antibody or the like can be used. The labeling substance of the labeled primary antibody is not particularly limited, and may be the same as described above.

The latter method using the labeled secondary antibody is a so-called sandwich method. In this method, for example, first, hybridization is caused between the cancer marker miRNA and the antigen-labeled probe. Subsequently, to the antigen-labeled probe that has bound to the cancer marker miRNA, the primary antibody is bound via the antigen. Further, the labeled secondary antibody is bound to the primary antibody that has bound to the probe. As a result, the labeled secondary antibody is bound to the cancer marker miRNA via the primary antibody. Then, the labeling substance of the labeled secondary antibody is detected. In this manner, the probe that has hybridized to the cancer marker miRNA can be detected, whereby the cancer marker miRNA can be detected indirectly. The kind of the antigen labeling the probe and the primary antibody are not particularly limited, and may be the same as described above. The labeling substance of the secondary antibody is not particularly limited, and may be the same as described above.

Next, an illustrative example where the cancer marker miRNA is detected directly from the sample will be described.

According to the present invention, the region where the cancer marker miRNA is expressed in the sample can be specified, for example. Thus, it is preferable to detect the cancer marker miRNA directly from the sample. In this case, it is preferable to immobilize the sample. The method for detecting the cancer marker miRNA preferably is a hybridization method using a probe, for example. In particular, for example, it is preferable to detect the cancer marker by performing an in situ hybridization method with respect to an immobilized sample, and it is particularly preferable to apply immunohistochemistry (IHC) utilizing an antigen-antibody reaction. According to such a method, it is possible to stain the inside of cytoplasm or nucleus with the cancer marker miRNA, for example. As the probe, for example, a labeled probe is preferable, and the same probes as those described above can be used.

The in situ hybridization method can be carried out according to a known procedure, for example. Also, the in situ hybridization method can be carried out using a commercially available kit or the like in accordance with its instructions for use. Examples of the kit include a RiboMap in situ hybridization kit (trade name) available from Ventana.

In the in situ hybridization method, for example, preparation of a section slide of the sample, a pretreatment of the section slide, hybridization of the labeled probe, and detection of a hybridization signal are performed. A specific example of the in situ hybridization method will be given below. It is to be noted, however, that the present invention is not limited thereto.

First, a section slide of a cell or a tissue is prepared as the sample. The section slide can be prepared by immobilizing the sample using an immobilizing solution, embedding the sample, then cutting the sample into a piece having a desired thickness, and placing the thus-obtained cut piece on a slide. Examples of the immobilizing solution include: crosslinking agents such as formaldehyde and paraformaldehyde; PLP (periodate-lysine-paraformaldehyde); Zamboni's solution; glutaraldehyde; and coagulating and precipitating agents, such as ethanol and acetone. The immobilization may be achieved by, for example, any preparation method selected from freeze sectioning with respect to an unimmobilized sample, freeze sectioning with respect to an immobilized sample, and paraffin embedding with respect to an immobilized sample, for example. The conditions for the immobilization are not particularly limited, and the immobilization preferably is performed at room temperature for at least 1 hour, more preferably at least 6 hours, for example.

Next, prior to the hybridization, the section slide is pretreated. The pretreatment may be, for example, deparaffinization, rehydration, reimmobilization, an acid treatment, or a treatment with proteinase K so as to improve the permeability of the labeled probe. Furthermore, in order to prevent non-specific binding of the probe caused by addition of glycine or acetic acid so as to inactivate the proteinase K, a treatment for neutralizing positive electric charge or the like also may be performed.

Then, the labeled probe is added to the pretreated section slide so as to cause hybridization, and detection of a hybridization signal is performed in a manner appropriate for a labeling substance of the labeled probe.

The amount of the labeled probe to be added is not particularly limited, and can be set as appropriate depending on, for example, the kind of the labeling substance, the proportion of the labeled probe with respect to the probe to be used as a whole, and the like. As a specific example, the amount of the labeled probe to be added may be, but not at all limited to, 1 to 1000 ng per a single slide generally used in an in situ hybridization method, for example.

The conditions of the hybridization are not particularly limited. A specific example is as follows. A thermal denaturation treatment prior to the hybridization preferably is performed at 50°C to 100°C for 1 to 60 minutes, more preferably at 60°C to 95°C for 5 to 10 minutes. Furthermore, the hybridization preferably is performed at 40°C to 80°C for 1 to 36 hours, more preferably at 45°C to 70°C for 4 to 24 hours.

Next, detection of the hybridization signal is performed. The method for detecting the signal is not particularly limited, and can be determined as appropriate depending on the labeled probe and the kind of the labeling substance of the labeled primary antibody or labeled secondary antibody, as described above, for example. When color development or fluorescence is detected, for example, the presence or absence or the amount of the cancer marker miRNA in the sample can be detected based on the presence or absence of color development or fluorescence, or based on the density of the color developed or the intensity of fluorescence. The color development or fluorescence may be checked by visual observation or image processing, for example. When the labeling substance is a radioactive material, an autoradiography may be utilized, for example, and the presence or absence or the amount of the cancer marker miRNA in the sample can be detected based on the presence or absence or the color density of an autoradiograph. The presence or absence or the color density of the autoradiograph may be checked by visual observation or image processing, for example. Image processing is not particularly limited, and can be carried out using a known system or known software.

When a probe is used in the detection of the cancer marker miRNA as described above, the sequence of the probe is not particularly limited. Examples of the probe include probes that can specifically bind to any of the above-described cancer marker miRNAs. A commercially available product may be used as the probe, or the probe may be self-prepared, for example. The sequence of the probe can be designed as appropriate based on, for example, the base sequence of the above-described cancer marker miRNA and common general technical knowledge. Specifically, for example, the probe may be a probe consisting of a sequence complementary to a detection target cancer marker miRNA, or a probe including the complementary sequence. It is preferable that the sequence of the probe is at least about 70% complementary to the target cancer marker miRNA, more preferably at least 90% complementary to the same, and particularly preferably 100% complementary to the same, for example.

The constitutional unit of the probe is not particularly limited, and known constitutional units can be employed, for example. Specific examples of the constitutional units include: nucleotides such as deoxyribonucleotide and ribonucleotide; PNA; and LNA. Examples of the LNA include BNA (Bridged Nucleic Acid) such as 2',4'-bridged nucleic acid. Bases in the constitutional unit are not particularly limited. They may be, for example, natural bases such as adenine, guanine, cytosine, thymine, and uracil, or may be unnatural bases. The length of the probe is not particularly limited, and is, for example, 10 to 100 mer, preferably 15 to 40 mer.

A specific example of the probe is shown below. It is to be noted, however, the present invention is not limited thereto. Other examples of the probe include polynucleotide consisting of a sequence complementary to the base sequence shown in SEQ ID NO: 5.
hsa-miR-92a detection probe (SEQ ID NO: 5)
   5'-acaggccgggacaagtgcaata-3'

The evaluation method of the present invention evaluates, in the evaluation step, the possibility of a cancer in the sample based on the expression level of the cancer marker miRNA detected in the above-described manner.

The expression level of the cancer marker may be, for example, the presence or absence of the expression of the cancer marker miRNA in the sample or the amount of the cancer marker miRNA expressed in the sample. The expression amount may be, for example, the actual amount of miRNA or a value correlated with the actual amount of miRNA. Examples of the latter include a signal value obtained when detecting the cancer marker miRNA. The signal value can be determined as appropriate depending on the method for detecting the miRNA, the kind of a detector for detecting the signal value, and the like, for example. When the detection method is any of nucleic acid amplification methods including PCR methods such as a real-time RT-PCR method, for example, the signal value can be expressed as the number of copies per 1 µl (copies/µl) or the like, for example. Furthermore, as will be described below, when an image having been subjected to miRNA staining (hereinafter referred to as a "miRNA-stained image") is used, the value (lightness) or saturation of the color development or fluorescence corresponds to the signal value, for example.

In the evaluation method of the present invention, when the cancer marker miRNA in the immobilized sample is detected as described above, it is preferable to provide a section slide with the cancer marker miRNA being visualized (hereinafter also referred to as a "cancer marker miRNA visualizing section slide") and a section slide stained with HE (hematoxylin & eosin), and collate them with each other, for example.

Currently, for determination of canceration of a cell or a tissue by pathologists, an image stained with HE (hereinafter referred to as an "HE-stained image") are used. However, HE staining has problems in that, for example: evaluation becomes difficult as to the borderline pathological change; and evaluation becomes difficult when the number of examined cells is 1 or 2. On this account, HE staining and visualization of cancer marker miRNA are performed with respect to section slides derived from the same sample, and the section slides are collated with each other. Then, by collating a tumor area specified based on the HE staining with an area positive for the visualized cancer marker miRNA, it is possible to determine the possibility of a cancer with higher reliability. The term "positive" means that the cancer marker is present, for example, and the term "negative" means that the cancer marker is not present or the cancer marker is present in an amount below the detection limit, for example.

The method for determining the possibility of a cancer by collating the cancer marker miRNA visualizing section slide with the section slide subjected to the HE staining is not particularly limited, and can be carried out in the following manner, for example. First, the HE staining and visualization of miRNA are performed with respect to adjacent section slides in the manner to be described below. Regarding the HE-stained section slide, a tumor area is determined by observation with a microscope or the like, for example. Then, the HE-stained section slide is collated with the cancer marker visualizing section slide. As a result, for example, when the tumor area in the HE-stained section slide agrees with the cancer marker positive area in the cancer marker visualizing section slide, it can be determined that the tumor area and the cancer marker positive area are cancerous.

In the case where not only the visualization of the cancer marker miRNA but also HE staining is performed with respect to the sample, it is preferable that the section slide for the cancer marker miRNA detection and the section slide for the HE staining are adjacent sections cut off from the immobilized and embedded sample. Specifically, it is preferable that the adjacent sections are serial sections. This allows more accurate collation of the images.

The collation between the visualized cancer marker miRNA and the HE staining preferably is performed by collating images, for example. That is, it is preferable to provide an image with the cancer marker being visualized (hereinafter also referred to as a "cancer marker visualization image") and an image stained with HE with regard to the immobilized sample, and collate them with each other. The images can be provided by, for example, converting the immobilized sample visualized by the cancer marker miRNA and the immobilized sample visualized by the HE staining into digital images by a CCD (Charge Coupled Device Image Sensor), a scanner, or the like.

By using the images as described above, collation between the visualized cancer marker miRNA and the HE staining as well as the determination of the possibility of a cancer can be carried out more easily and accurately. Furthermore, by accumulating image data, still more reliable evaluation becomes possible.

The method for determining the possibility of a cancer by collating the cancer marker miRNA visualization image with the HE-stained image is not particularly limited, and can be carried out in the following manner, for example. First, the HE staining and the visualization of the cancer marker miRNA are performed with respect to adjacent section slides as described above, thus providing an HE-stained image and a cancer marker visualization image. Then, a tumor area in the HE-stained image is specified, and the HE-stained image and the cancer marker visualization image are collated each other. As a result, when the tumor area in the HE-stained image and the cancer marker miRNA positive area in the cancer marker visualization image agree with each other, it can be determined that the tumor area and the cancer marker positive area are cancerous. Furthermore, when no tumor area was found in the HE-stained image and the cancer marker visualization image is negative for the cancer marker miRNA, it can be determined that the sample is not cancerous. Still further, when the tumor area in the HE-stained image does not agree with the cancer marker positive area in the cancer marker visualization image, the final judgment may be left to a pathologist, and this data may be stored as accumulation data so as to be prepared for upcoming judgments, for example.

It has been revealed by the present invention that the expression levels of hsa-miR-92a and hsa-miR-494, which are the cancer marker miRNAs, increase in cells and tissues accompanying the canceration, for example. From this fact, it is interpreted that, for example, the expression level of each of the cancer marker miRNAs increases significantly: after the onset of a cancer as compared with before the onset of the cancer; in the preclinical stage as compared with before the preclinical stage; in the clinical stage as compared with before the clinical stage; in the initial stage as compared with before the initial stage; and after the initial stage as compared with in the initial stage. Thus, the evaluation method of the present invention may include, for example, in the evaluation step, the step of determining the possibility of the cancer based on the expression level of the cancer marker miRNA by at least one method selected from the group consisting of the following (1), (2) and (3), for example. In the present invention, the term "normal subject" means, for example, a subject who is not determined as having developed a cancer to be evaluated or having a possibility that he has developed the cancer. On the other hand, the term "patient" means, for example, a subject who is not determined as having developed a cancer to be evaluated.
(1) The expression level of the cancer marker miRNA in the sample of a subject is compared with an expression level of the cancer marker miRNA in a sample of a normal subject, and when the expression level in the subject is higher than the expression level in the normal subject, it is determined that the subject has a high possibility of the cancer.
(2) The expression level of the cancer marker miRNA in the sample of a subject is compared with an expression level of the cancer marker miRNA in a sample of a normal subject, and as the expression level in the subject becomes relatively higher than the expression level in the normal subject, it is determined that the cancer in the subject is relatively advanced.
(3) The expression level of the cancer marker miRNA in the sample of a subject is compared with an expression level of the cancer marker miRNA in a sample of each of cancer patients at different progression stages, and it is determined that the cancer in the subject is in the same progression stage as the cancer in the patient exhibiting the same or a similar expression level.

The expression level of the cancer marker miRNA in the normal subject in the methods (1) and (2) can be determined using a sample collected from the normal subject, for example. Furthermore, the expression levels of the cancer marker miRNA in the cancer patients in the method (3) can be determined by, for example, classifying the patients according to the progression stage and using samples collected from the patients at the respective progression stages. In the methods (1) to (3), the expression levels of the normal subject and the patients may be determined in advance, for example, and it is not necessary to determine them every time evaluation is made. In the methods (1) to (3), the kind of the samples of the normal subject and the patients preferably are the same as the kind of the sample of the subject, for example. Furthermore, the samples of the normal subject and the patients preferably are prepared in the same manner and under the same conditions as the sample of the subject, for example. The expression level of the cancer marker miRNA of the normal subject or each of the patients may be, for example, a value obtained from a single normal subject or a single patient, or may be a value calculated from the expression levels in a plurality of normal subjects or a plurality of patients by a statistical method.

In the method (1), as described above, when the expression level in the subject is higher than the expression level in the normal subject, it can be determined that the subject has a high possibility of the cancer. On the other hand, when the expression level in the subject is equal to or lower than that that in the normal subject, it can be determined that the subject has a low possibility of the cancer. The possibility of a cancer also can be referred to as, for example, the possibility that the canceration has occurred or the possibility that the subject might have the cancer.

In the method (2), as described above, as the expression level in the subject becomes relatively higher than the expression level in the normal subject, it can be determined that the cancer in the subject is relatively advanced. Furthermore, even when the expression level in the subject is higher than the expression level in the normal subject, it can be determined that the cancer is not relatively advanced as the different between the expression levels becomes smaller.

In the method (3), for example, the expression level of each of the cancer patients at different progression stages is determined. Through comparison between the expression level of each patient and the expression level in the subject, not only the possibility of canceration of the subject but also the progression stage of the cancer can be evaluated.

In the methods (1) to (3), when the expression level in the subject is compared with that of the normal subject or that of each patient, the significant difference therebetween can be determined by a statistical method such as a t-test, an F-test, or a chi-square test, for example.

According to such an evaluation method, for example, with regard to a subject having a cancer at a preclinical stage, it is possible to determine that the subject has a high possibility of the cancer with high reliability whereas such determination has been difficult conventionally. Furthermore, for example, the stage of cancer progression also can be determined with high reliability. Thus, in prevention or treatments of cancers, information important in determining strategies for medication, operation, etc. for example, can be obtained with high reliability.

Moreover, the evaluation method of the present invention can evaluate a cancer by calculating the content rate of staining positive cells, for example. The term "staining positive cell" means a cell that has the cancer marker and is stained by the staining of the cancer marker, for example. In this case, in the evaluation method of the present invention, for example, an image having been subjected to cancer marker staining (hereinafter referred to as a "cancer marker-stained image") is obtained regarding the immobilized sample in the cancer marker detecting step. The evaluation method of the present invention preferably further includes:
an HE-stained image obtaining step of obtaining an HE-stained image regarding the immobilized sample;
an information obtaining step of obtaining information of a tumor area in the HE-stained image,
a matching step of calculating a matching position of the HE-stained image obtained in the HE image obtaining step and the cancer marker-stained image obtained in the cancer marker-detecting step;
a specifying step of specifying a tumor area in the cancer marker-stained image based on the information of the tumor area in the HE-stained image obtained in the information obtaining step and information of the matching position calculated in the matching step; and
a staining positive cell detecting step of detecting staining positive cells in the tumor area in the cancer marker-stained image based on information of the tumor area in the cancer marker-stained image specified in the specifying step.

The staining positive cell detecting step may be, for example, a calculating step of calculating a staining positive cell content rate in the tumor area in the cancer marker-stained image based on information of the tumor area in the cancer marker-stained image specified in the specifying step. The cancer marker-stained image may be, for example, an image with the cancer marker miRNA being visualized by color development or light emission.

As described above, evaluation of a cancer including collation with the HE-stained image can be performed by a cancer pathological image diagnosis support method to be described below, for example. This method can be realized by, for example, operating a cancer pathological image diagnosis support system, a cancer pathological image diagnosis support program, or a cancer pathological image diagnosis support device, each of which will be described below. This will be described in detail below.

### <Evaluation Reagent>

The evaluation reagent of the present invention is, as described above, an evaluation reagent to be used in the evaluation method of the present invention and **characterized in that** it contains a reagent for detecting the cancer marker of the present invention, i.e., a miRNA detection reagent for detecting at least one miRNA selected from hsa-miR-92 and hsa-miR-494. According to such an evaluation reagent, it is possible to carry out the evaluation method of the present invention conveniently.

As described above, the present invention is **characterized in that** at least one of hsa-miR-92 and hsa-miR-494 is detected as a cancer marker miRNA, and a method for detecting these miRNAs is by no means limited. It is only necessary that the miRNA detection reagent contained in the evaluation reagent of the present invention can detect either of these miRNAs, and the kind, composition, etc. of the reagent are by no means limited, for example. Furthermore, those skilled in the art can set detection reagents for these cancer marker miRNAs based on common general technical knowledge.

The miRNA detection reagent is not particularly limited, and examples thereof include probes that can hybridize to either of the cancer marker miRNAs, such as those described above. The probe may be a labeled probe as described above. Furthermore, depending on the method for detecting the miRNA, the kind of a labeling substance used in the labeled probe, and the like, the miRNA detection reagent may further contain other reagents.

The evaluation reagent of the present invention further may contain an enzyme, a buffer solution, a washing solution, a dissolving solution, a dispersing solution, a diluent, and the like depending on the method for detecting miRNA, for example. Furthermore, the form of the evaluation reagent of the present invention is not particularly limited. For example, it may be a wet-type reagent in the liquid form or a dry-type reagent in the dry form.

### <Evaluation Kit>

The evaluation kit of the present invention is, as described above, an evaluation kit to be used in the evaluation method of the present invention and **characterized in that** it includes a miRNA detection reagent for detecting at least one miRNA selected from hsa-miR-92 and hsa-miR-494. Examples of the miRNA detection reagent include the evaluation reagent of the present invention, which is as described above. According to such an evaluation kit, the evaluation method of the present invention can be carried out conveniently.

The form of the evaluation kit of the present invention is not particularly limited. It may be a wet-type kit in the liquid form or a dry-type kit in the dry form, for example. The respective reagents in the evaluation kit of the present invention may be provided separately and used together when the kit is used, or may be mixed together before the kit is used, for example. The evaluation kit of the present invention may include instructions for use, for example.

### <Diagnosis Support Based on Cancer Pathological Image>

The present invention provides a system, a program, a method, and a device, each for supporting a diagnosis of a cancer based on a pathological image, and examples thereof include the following first and second embodiments.

### First Embodiment

The present invention provides a cancer pathological image diagnosis support system (hereinafter referred to as an "image diagnosis support system") for supporting a diagnosis of a cancer based on a pathological image, including:
an image obtaining unit that obtains an HE-stained image and a cancer marker-stained image as pathological images to be diagnosed;
an information obtaining unit that obtains information of a tumor area in the HE-stained image;
a matching unit that calculates a matching position of the HE-stained image and the cancer marker-stained image obtained by the image obtaining unit;
a specifying unit that specifies a tumor area in the cancer marker-stained image based on the information of the tumor area in the HE-stained image obtained by the information obtaining unit and information of the matching position calculated by the matching unit; and
a staining positive cell detecting unit that detects staining positive cells in the tumor area in the cancer marker-stained image based on information of the tumor area in the cancer marker-stained image specified by the specifying unit.

The staining positive cell detecting unit may be, for example, a staining positive cell content rate in the tumor area in the cancer marker-stained image based on information of the tumor area in the cancer marker-stained image specified by the specifying unit (hereinafter the same).

It is preferable that the calculating unit calculates staining intensity in addition to the staining positive cell content rate. The staining intensity may be, for example, staining intensity in the tumor area in the cancer marker-stained image.

It is preferable that the image diagnosis support system of the present invention further includes:
an input accepting unit that accepts input of information for specifying a pathological image to be diagnosed and information for specifying a test type; and
a stained image database that stores the HE-stained image and the cancer marker-stained image, wherein
the image obtaining unit obtains the HE-stained image and the cancer marker-stained image from the stained image database based on the specifying information.

In the image diagnosis support system of the present invention, it is preferable that the information for specifying the pathological image to be diagnosed is an image identifier of the HE-stained image, and the image obtaining unit obtains the HE-stained image having the image identifier and the cancer marker-stained image adjacent to the HE-stained image from the stained image database.

In the image diagnosis support system of the present invention, it is preferable that the information for specifying the pathological image to be diagnosed is an image identifier of the cancer marker-stained image, and the image obtaining unit obtains the cancer marker-stained image having the image identifier and the HE-stained image adjacent to the cancer marker-stained image from the stained image database.

In the image diagnosis support system of the present invention, it is preferable that the information for specifying the pathological image to be diagnosed is a subject identifier of a diagnosis target, and the image obtaining unit obtains the HE-stained image and the cancer marker-stained image each having the target identifier from the stained image database.

In the image diagnosis support system of the present invention, it is preferable that the stained image database also stores the information of the tumor area in the HE-stained image, and the information obtaining unit obtains the information of the tumor area in the HE-stained image from the stained image database.

It is preferable that the image diagnosis support system of the present invention further includes: a tumor area calculating unit that calculates the tumor area in the HE-stained image obtained by the image obtaining unit, wherein the information obtaining unit obtains the information of the tumor area in the HE-stained image calculated by the tumor area calculating unit.

It is preferable that the image diagnosis support system of the present invention further includes:
an input accepting unit that accepts input of a slide identifier of a slide to be diagnosed and information for specifying a test type;
a slide database that stores the slide; and
a slide obtaining unit that obtains the slide having the slide identifier from the slide database, wherein
the image obtaining unit obtains the HE-stained image and the cancer marker-stained image by imaging the slides obtained by the slide obtaining unit.

The present invention provides an image diagnosis support system for supporting a diagnosis of a cancer based on a pathological image, including: a terminal; and a server. The terminal and the server can be connected to each other via a communication network provided outside of the system. The terminal includes: a terminal side transmission unit that transmits information in the terminal to the server via the communication network; and a terminal side receiver unit that receives information transmitted from the server via the communication network. The server includes: a server side transmission unit that transmits information in the server to the terminal via the communication network; a server side receiver unit that receives information transmitted from the terminal via the communication network; an image obtaining unit that obtains an HE-stained image and a cancer marker-stained image as pathological images to be diagnosed; an information obtaining unit that obtains information of a tumor area in the HE-stained image; a matching unit that calculates a matching position of the HE-stained image and the cancer marker-stained image obtained by the image obtaining unit; a specifying unit that specifies a tumor area in the cancer marker-stained image based on the information of the tumor area in the HE-stained image obtained by the information obtaining unit and information of the matching position calculated by the matching unit; and a staining positive cell detecting units that detects staining positive cells in the tumor area in the cancer marker-stained image based on information of the tumor area in the cancer marker-stained image specified in the specifying step. In this image diagnosis support system, information of the pathological images is transmitted from the terminal side transmission unit to the server side receiver unit, and information of the staining positive cells detected by the staining positive cell detecting unit of the server is transmitted from the server side transmission unit to the terminal side receiver unit.

In the image diagnosis support system of the present invention, the staining positive cell detecting unit may be, for example, a staining positive cell content rate in the tumor area in the cancer marker-stained image based on information of the tumor area in the cancer marker-stained image specified by the specifying unit. In this case, for example, information of the staining positive cell content rate calculated by the calculating unit of the server is transmitted from the server side transmission unit to the terminal side receiver unit.

The present invention also provides a server for use in the image diagnosis support system of the present invention. The server includes: a server side transmission unit that transmits information in the server to a terminal via a communication network; a server side receiver unit that receives information transmitted from the terminal via the communication network; an image obtaining unit that obtains an HE-stained image and a cancer marker-stained image as pathological images to be diagnosed; an information obtaining unit that obtains information of a tumor area in the HE-stained image; a matching unit that calculates a matching position of the HE-stained image and the cancer marker-stained image obtained by the image obtaining unit; a specifying unit that specifies a tumor area in the cancer marker-stained image based on the information of the tumor area in the HE-stained image obtained by the information obtaining unit and information of the matching position calculated by the matching unit; and a staining positive cell detecting units that detects staining positive cells in the tumor area in the cancer marker-stained image based on information of the tumor area in the cancer marker-stained image specified in the specifying step.

In the server of the present invention, the staining positive cell detecting unit may be, for example, a calculating unit that calculates a staining positive cell content rate in the tumor area in the cancer marker-stained image based on information of the tumor area in the cancer marker-stained image specified by the specifying unit.

The present invention also provides a terminal for use in the image diagnosis support system of the present invention. The terminal includes: a terminal side transmission unit that transmits information in the terminal to a server via a communication network; and a terminal side receiver unit that receives information transmitted from the server via the communication network. In the terminal, information of pathological image is transmitted from the terminal side transmission unit to the server side receiver unit, and information of staining positive cells detected by the staining positive cell detecting unit of the server is transmitted from the server side transmission unit to the terminal side receiver unit.

Furthermore, for example, information of the staining positive cell content rate calculated by the calculating unit of the server may be transmitted from the server side transmission unit to the terminal side receiver unit.

The present invention also provides a cancer pathological image diagnosis support method (hereinafter referred to as an "image diagnosis support method") for supporting a diagnosis of a cancer based on a pathological image, including the steps of:
obtaining an HE-stained image and a cancer marker-stained image as pathological images to be diagnosed;
obtaining information of a tumor area in the HE-stained image;
matching images to calculate a matching position of the HE-stained image and the cancer marker-stained image obtained in the image obtaining step;
specifying a tumor area in the cancer marker-stained image based on the information of the tumor area in the HE-stained image obtained in the information obtaining step and information of the matching position calculated in the matching step; and
detecting staining positive cells in the tumor area in the cancer marker-stained image based on information of the tumor area in the cancer marker-stained image specified in the specifying step.

In the image diagnosis support method of the present invention, the staining positive cell detecting step may be, for example, a calculating step of calculating a staining positive cell content rate in the tumor area in the cancer marker-stained image based on information of the tumor area in the cancer marker-stained image specified in the specifying step (hereinafter the same).

In the image diagnosis support method of the present invention, it is preferable that, in the calculating step, staining intensity is calculated in addition to the staining positive cell content rate. The staining intensity may be, for example, staining intensity in the tumor area in the cancer marker-stained image.

In the image diagnosis support method of the present invention, it is preferable that, in the image obtaining step, for example, the HE-stained image and the cancer marker-stained image are obtained based on information for specifying the pathological image to be diagnosed. It is preferable that the HE-stained image and the cancer marker-stained image are obtained from, for example, the stained image database storing the HE-stained image and the cancer marker-stained image.

In the image diagnosis support method of the present invention, it is preferable that the information for specifying the pathological image to be diagnosed is an image identifier of the HE-stained image. In the image obtaining step, it is preferable that the HE-stained image having the image identifier and the cancer marker-stained image adjacent to the HE-stained image are obtained from the stained image database, for example.

In the image diagnosis support method of the present invention, it is preferable that the information for specifying the pathological image to be diagnosed is an image identifier of the cancer marker-stained image. In the image obtaining step, it is preferable that the cancer marker-stained image having the image identifier and the HE-stained image adjacent to the cancer marker-stained image are obtained from the stained image database, for example.

In the image diagnosis support method of the present invention, it is preferable that the information for specifying the pathological image to be diagnosed is a subject identifier of a diagnosis target. In the image obtaining step, it is preferable that the HE-stained image having the subject identifier and the cancer marker-stained image are obtained from the stained image database, for example.

In the image diagnosis support method of the present invention, it is preferable that the stained image database also stores the information of the tumor area in the HE-stained image. In the information obtaining step, it is preferable that the information of the tumor area in the HE-stained image is obtained from the stained image database, for example.

Preferably, the image diagnosis support method of the present invention further includes the step of calculating the tumor area in the HE-stained image obtained in the image obtaining step. In the information obtaining step, it is preferable to obtain the information of the tumor area in the HE-stained image calculated in the tumor area calculating step.

The present invention also provides a cancer pathological image diagnosis support program (hereinafter referred to as an "image diagnosis support program") for supporting a diagnosis of a cancer based on a pathological image. The image diagnosis support program is **characterized in that** it can cause a computer to execute the image diagnosis method of the present invention.

The image diagnosis support program of the present invention causes a computer to execute, for example:
an image obtaining step of obtaining an HE-stained image and a cancer marker-stained image as pathological images to be diagnosed;
an information obtaining step of obtaining information of a tumor area in the HE-stained image;
a matching step of calculating a matching position of the HE-stained image and the cancer marker-stained image obtained in the image obtaining step;
a specifying step of specifying a tumor area in the cancer marker-stained image based on the information of the tumor area in the HE-stained image obtained in the information obtaining step and information of the matching position calculated at the matching step; and
a staining positive cell detecting step of detecting staining positive cells in the tumor area in the cancer marker-stained image based on information of the tumor area in the cancer marker-stained image specified in the specifying step.

In the image diagnosis support program of the present invention, the staining positive cell detecting step may be, for example, a calculating step of calculating a staining positive cell content rate in the tumor area in the cancer marker-stained image based on information of the tumor area in the cancer marker-stained image specified in the specifying step.

According to the above-described support system, support method, and support program, it is also possible to obtain the staining positive cell content rate as the quantitative value in the end, for example.

In the following, the first embodiment of the present invention will be described specifically with reference to Embodiments 1A to 1D. Hereinafter, the cancer marker-stained image also is referred to as a "miRNA-stained image". It is to be noted that the present invention is by no means limited to these embodiments.

### (Embodiment 1A)

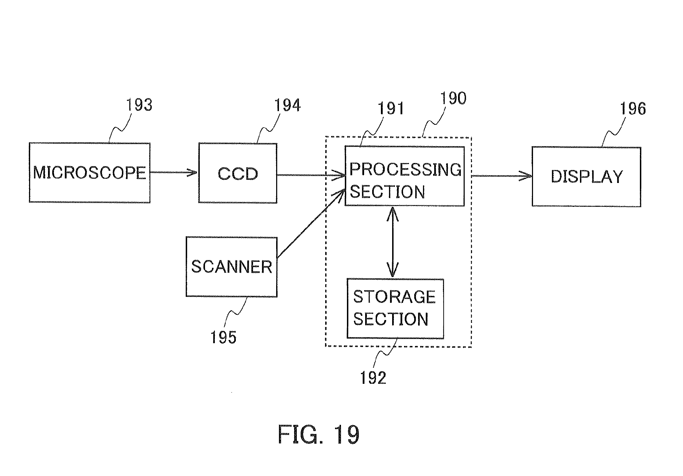

FIG. 19 is a block diagram showing an example of the configuration of an image diagnosis support device provided with the image diagnosis support system according to the present invention. As shown in FIG. 19, an image diagnosis support device 190 includes a processing section 191 and a storage section 192. The image diagnosis support device 190 is configured so that it is connected to a CCD 194 connected a microscope 193 and to a scanner 195 and a display 196 at the processing section 191. The image diagnosis support device 190 includes, for example, a CPU (Central Processing Unit), a RAM (Random Access Memory), an input section, a drive, an input-output interface (I/F), a communication bus, and the like. The CPU controls the entire image diagnosis support device. By installing, for example, a computer program for providing functions of the respective units in the CPU, it is possible to construct the respective units in the image diagnosis support device 190, thus realizing the image diagnosis support device 190. Furthermore, the operation of the image diagnosis support device 190 also can be realized by being equipped with circuit components including hardware components, such as an LSI (Large Scale Integration), in which a computer program for realizing functions of the respective units is installed. Such a computer program may be in the form of a recording medium or the like storing the computer program. Examples of the recording medium include HDD, FD, CD-ROM (CD-R, CD-RW), MO, DVD, and a memory card. Examples of the storage section 192 include ROM, HDD, and HD. HDD controls reading and writing of data with respect to HD under the control of the CPU, for example. HD stores the written data under the control of the HDD, for example.

The display 196 displays various information such as images, data, and documents, for example. Examples of the input section include a keyboard and a mouse. The scanner 195 scans the above-described section slide and optically converts the image into an electrical signal, for example. The CCD 194 converts a microscopic image of the section slide into an electrical signal, for example.

The image diagnosis support device 190 may be accessible to, for example, stained image database for accumulating information regarding stained images, which is provided outside of the image diagnosis support device 190. In this case, the image diagnosis support device 190 may be connected to the stained image database via a communication line, for example.

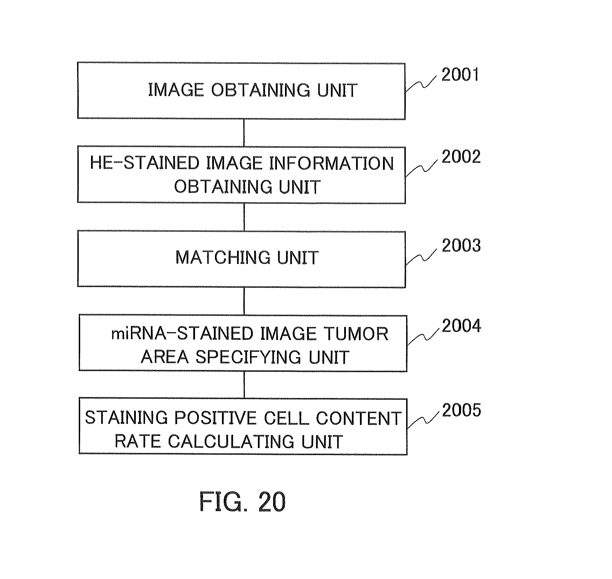

An illustrative example of the image diagnosis support system of the present invention will be given below. FIG. 20 is a block diagram schematically showing the configuration of the image diagnosis support system according to the present embodiment. It is to be noted that the present invention is by no means limited to this embodiment.

As shown in FIG. 20, the image diagnosis support system of the present embodiment includes: an image obtaining unit 2001 that obtains an HE-stained image and a miRNA-stained image; an information obtaining unit 2002 that obtains information of a tumor area in the HE-stained image; a matching unit 2003 that calculates a matching position of the HE-stained image and the miRNA-stained image, which were obtained by the image obtaining unit; a specifying unit 2004 that specifies a tumor area in the miRNA-stained image based on the information of the tumor area in the HE-stained image obtained by the information obtaining unit and information of the matching position calculated by the matching unit; and a calculating unit 2005 that calculates a staining positive cell content rate in the tumor area in the miRNA-stained image based on information of the tumor area in the miRNA-stained image specified by the specifying unit.

The image diagnosis support system may further include a staining level-determining unit that determines the miRNA staining level of the miRNA-stained image. The staining level-determining unit determines the staining level in the specified tumor area in the miRNA-stained image, for example.

Examples of such a system include the image diagnosis support device shown in FIG. 19. The respective constitutional units may be composed of, for example, a functional block realized by execution of a predetermined program by CPU of a computer. Thus, for example, the respective constitutional units are not necessarily provided as hardware components, and they may be provided as a network system. Unless otherwise stated, the image diagnosis support system of the present embodiment is the same as image diagnosis support systems according to Embodiments 1B to 1E to be described below, for example.

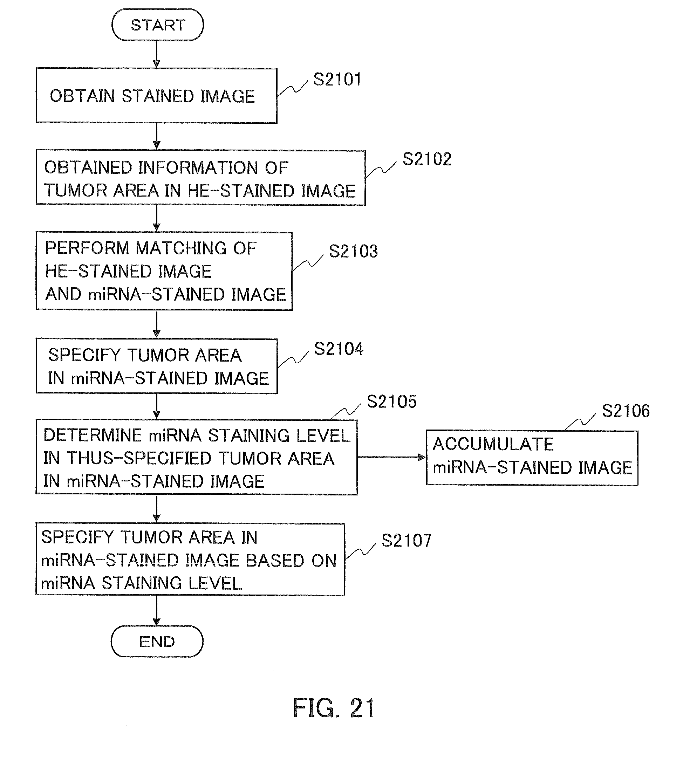

With reference to FIG. 21, one example of a flow of processing in the image diagnosis support system of the present embodiment will be described. FIG. 21 is a flowchart showing the processing flow. This processing is an example of the image diagnosis method according to the present invention, and can be carried out by the image diagnosis support system, image diagnosis support program, or the like of the present invention, for example.

First, an HE-stained image and a miRNA-stained image are obtained (Step S2101). The images can be obtained as electrical signals resulting from the conversion by an image pickup device such as a scanner or CCD, for example.

Next, regarding the HE-stained image, information of a tumor area is obtained (S2102). The information of the tumor area in the HE-stained image may be information determined by a doctor or the like, for example, or may be information calculated by a known method.

Next, matching is performed by overlaying one of the HE-stained image and the miRNA-stained image with the other and calculating a matching position (S2103). Then, based on the information of the tumor area in the HE-stained image and the thus-obtained information of the matching position, a tumor area in the miRNA-stained image is calculated. That is, an area of the miRNA-stained image corresponding to the tumor area in the HE-stained image is calculated, and then the area is specified as a tumor area (S2104). Hereinafter, the tumor area in the miRNA-stained image specified based on the information of the HE-stained image also is referred to as a "tumor area based on the HE-stained image".

Subsequently, the miRNA staining level of the tumor area in the miRNA-stained image determined based on the HE-stained image is determined (S2105). Regarding the miRNA staining level, for example, it is preferable to perform image standardization because the degree of staining differs from slide to slide depending on the staining process, the temperature, the kind of a probe, the kind of a color-developing substance or a fluorescent substance, or the like. At this time, in the miRNA-stained image, the miRNA staining level of an area other than the tumor area based on the HE-stained image also may be determined in the same manner.

Then, the stained image information obtained in this step is accumulated in the stained image database such as described above (S2106).

Next, based on the thus-determined miRNA staining level, a tumor area in the miRNA-stained image is detected again (S2107). That is, regarding the staining level of the miRNA-stained image, whether it reaches the level meaning a tumor cell or is below the level meaning a tumor cell is determined. Then, an area exhibiting the former staining level is specified as a tumor area based on the miRNA staining level. As a result, when the tumor area based on the HE-stained image agrees with the tumor area based on the miRNA staining level, the tumor area based on the miRNA staining level is determined as a detection target area. Also, when the area is not determined as a tumor area in the HE-stained image and the area is determined as a tumor area based on the miRNA staining level, the tumor area based on the miRNA staining level is determined as a detection target area.

The threshold between the staining at the level meaning the tumor and the staining below the level meaning the tumor can be determined by detecting the intensity of the staining in the miRNA-stained image at a plurality of levels, for example. Thus, for example, even when a nontumor cell is stained only slightly, it can be determined that this does not mean the staining indicating a tumor cell. The data regarding the threshold also is accumulated in the stained image database as information regarding the stained images, for example.

The detection target area determined in the above-described manner is outputted as a determined cancer area. Alternatively, regarding the detection target area, the staining positive cell content rate is calculated, and the result of the calculation is outputted.

When information of the miRNA-stained image is accumulated in the stained image database, for example, it is also possible to determine the tumor area in the miRNA-stained image based on the database without conducting the matching with the HE-stained image.

### (Embodiment 1B)

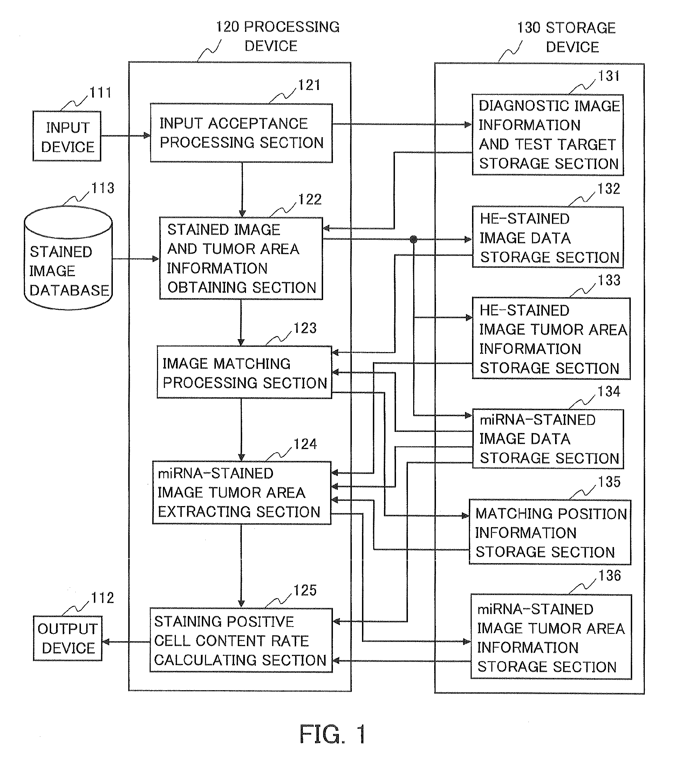

FIG. 1 is a block diagram showing an example of the image diagnosis support system according to the present invention. This system is a system for supporting a diagnosis of a cancer based on a pathological image. The system includes: an image obtaining unit that obtains an HE-stained image and a miRNA-stained image as pathological images to be diagnosed; an information obtaining unit that obtains information of a tumor area in the HE-stained image; a matching unit that calculates a matching position of the HE-stained image and the miRNA-stained image obtained by the image obtaining unit; a specifying unit that specifies a tumor area in the miRNA-stained image based on the information of the tumor area in the HE-stained image obtained by the information obtaining unit and information of the matching position calculated by the matching unit; and a calculating unit that calculates a staining positive cell content rate in the tumor area in the miRNA-stained image based on information of the tumor area in the miRNA-stained image specified by the specifying unit.

In more detail, the system of present embodiment includes an input device 111, an output device 112, a stained image database 113, a processing device 120, and a storage device 130.

The stained image database 113 stores one or more HE-stained images, a miRNA-stained image, which is a specimen of a serial section adjacent to a specimen (a section slide) of the HE-stained image, specimen adjacent information regarding the above-described HE-stained image and the above-described miRNA-stained image, and tumor area information calculated from the above-described HE-stained image or determined by a doctor or the like.

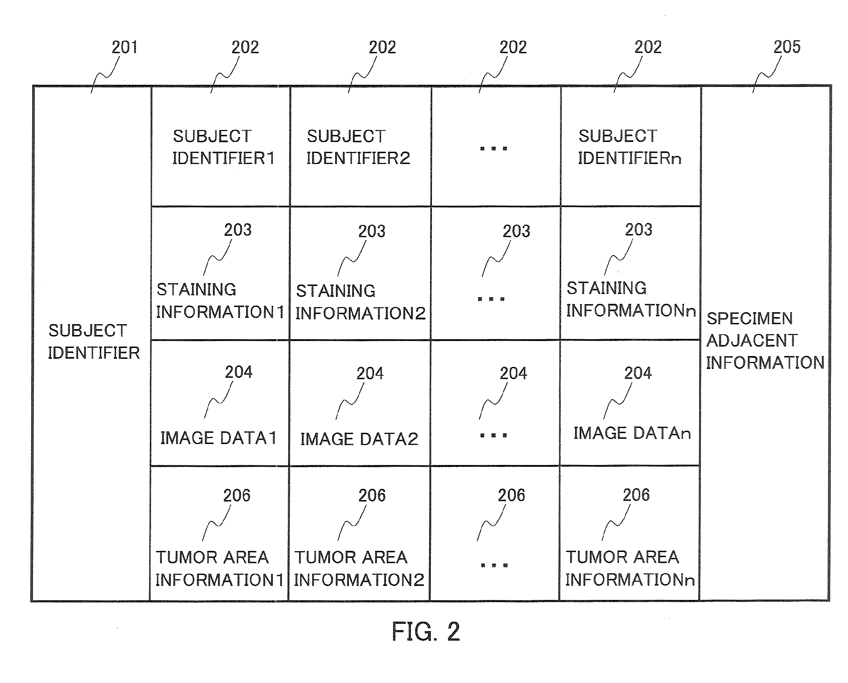

Each image has a subject identifier by which relevant information regarding a subject is related with each image. For example, as shown in FIG. 2, the stained image database 113 includes a subject identifier 201 for uniquely identifying the subject, an image identifier 202, staining information 203, image data 204, specimen adjacent information 205, and HE-stained image tumor area information 206.

The image identifier 202 is an identifier for identifying a plurality of pathological images of each subject. The staining information 203, the image data 204, and the tumor area information 206 are distinguished from those of other images by the image identifier 202. Each staining information 203 indicates the staining information of the image, and examples of the staining information include information regarding HE staining and information regarding cancer marker miRNA staining. Each image data 204 stores an image data. The specimen adjacent information 205 stores the correspondence relationship by using the image identifier 202. The HE-stained image tumor area information 206 stores the tumor area information calculated from the HE-stained image or determined by the doctor or the like. Meanwhile, the HE-stained image tumor area information 206 may be made correspond to the image identifier 202 and stored separately.

As the input device 111 and the output device 112, normal input/output devices provided on a computer may be used, for example. The input device 111 is a keyboard or a mouse, for example. The output device 112 is a display device or a printer, for example. The input device 111 and the output device 112 may be an input file and/or an output file, or may be another computer or the like.

The storage device 130 is composed of a main storage device and an auxiliary storage device provided on a computer, and is used for holding various programs executed in the processing device 120 and data, for example. The processing device 120 includes a CPU of a computer and operates by program control.

The processing device 120 includes an input acceptance processing section 121, a stained image and tumor area information obtaining section 122, an image matching processing section (matching unit) 123, a miRNA-stained image tumor area extracting section (specifying unit) 124, and a staining positive cell content rate calculating section (calculating unit) 125. The stained image and tumor area information obtaining section 122 has functions of both the above-described image obtaining unit and information obtaining unit.

The input acceptance processing section 121 accepts information for specifying the pathological image to be diagnosed and information for specifying the test type from a user or the like by means of the input device 111. Examples of the test type include the type of a cancer marker miRNA to be tested. Further, the input acceptance processing section 121 stores the pieces of information in a diagnostic image information and test target storage section 131 of the storage device 130, and shifts the process to the stained image and tumor area information obtaining section 122. In the case of the present embodiment, the information for specifying the pathological image to be diagnosed is the image identifier 202. The image identifier 202 is the HE-stained image or the miRNA-stained image, and it is possible to specify one or a plurality of them. Also, the information for specifying the test type is an item regarding the miRNA staining, and it is possible to specify, among cancer marker miRNAs of the present invention, any one miRNA or two or more miRNAs.

The stained image and tumor area information obtaining section 122 obtains the HE-stained image and the miRNA-stained image to be diagnosed and the information of the tumor area in the HE-stained image from the stained image database 113, and stores them, respectively, in an HE-stained image data storage section 132, a miRNA-stained image data storage section 134, and an HE-stained image tumor area information storage section 133 in the storage device 130, and shifts the process to the image matching processing section 123.

In the case where the staining information 203 having the image identifier 202 stored in the diagnostic image information and test target storage section 131 is HE staining, the image data 204 having the image identifier 202 is stored in the HE-stained image data storage section 132. Also, by referring to the test type stored in the diagnostic image information and test target storage section 131 and the specimen adjacent information 205, the image data 204 of the miRNA-stained image of the serial section specimen adjacent to the HE image specimen to be diagnosed is stored in the miRNA-stained image data storage section 134. Further, the information of the HE-stained image tumor area information 206 is stored in the HE-stained image tumor area information storage section 133.

On the other hand, in the case where the staining information 203 having the image identifier stored in the diagnostic image information and test target storage section 131 is miRNA staining, the image data 204 having the image identifier 202 is stored in the miRNA-stained image data storage section 134. Also, by referring to the specimen adjacent information 205, the image data 204 of the HE-stained image of the serial section specimen adjacent to the miRNA-stained image specimen to be diagnosed is stored in the HE-stained image data storage section 132. Further, the information of the HE-stained image tumor area information 206 is stored in the HE-stained image tumor area information storage section 133. When a plurality of image identifiers are specified, each of them is searched, associated, and stored.

The image matching processing section 123 reads the HE-stained image and the miRNA-stained image from the HE-stained image data storage section 132 and the miRNA-stained image data storage section 134, respectively, and calculates the matching position of the HE-stored image and the miRNA-stained image. Further, the image matching processing section 123 stores the matching position information in a matching position information storage section 135, and shifts the process to the miRNA-stained image tumor area extracting section 124. Examples of the matching position information include a rotational angle and a horizontal/vertical misalignment width. Since the HE-stained image and the miRNA-stained image are images obtained by staining the serial sections, they may be very similar to each other. The HE staining and the miRNA staining may be performed using colors of the same hue or different hues, for example. However, since the HE-stained image and the miRNA-stained image are subjected to the matching process, it is preferable to stain cells in the miRNA staining in color having a different hue from the color used in the HE staining. Generally, in the HE staining, cell nuclei are stained blue with hematoxylin and cytoplasm is stained pink with eosin. The hue of the color used in the miRNA staining can be set as appropriate by the color-developing substance or fluorescent substance to be used, or the like, for example. In the image matching, each image is binalized, and a phase-only correlation method, a sequential similarity detection algorithm, and a method of using a unique point may be used.

The miRNA-stained image tumor area extracting section 124 reads the HE-stained image tumor area information, the miRNA-stained image data, and the matching position information from the HE-stained image tumor area information storage section 133, the miRNA staining data storage section 134, and the matching position information storage section 135, respectively, and calculates the tumor area in the miRNA-stained image data. Further, the miRNA-stained image tumor area extracting section 124 stores the information of the tumor area in the miRNA-stained image data in the miRNA-stained image tumor area information storage section 136, and shifts the process to the staining positive cell content rate calculating section 125.

The staining positive cell content rate calculating section 125 reads the miRNA-stained image data and the tumor area information from the miRNA-stained image data storage section 134 and the miRNA-stained image tumor area information storage section 136, respectively. It then counts the number of staining positive cell nuclei and the number of staining negative cell nuclei in the tumor area, and calculates the staining positive cell content rate to output from the output device 112.

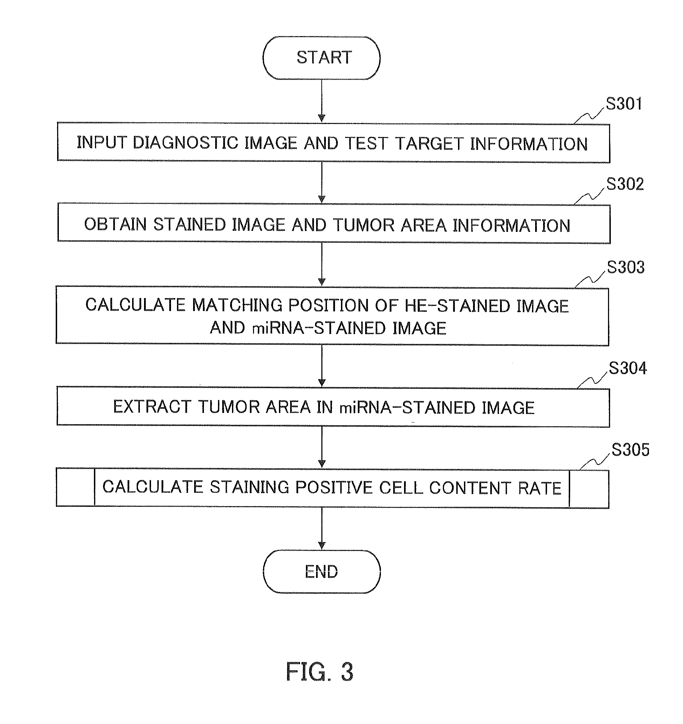

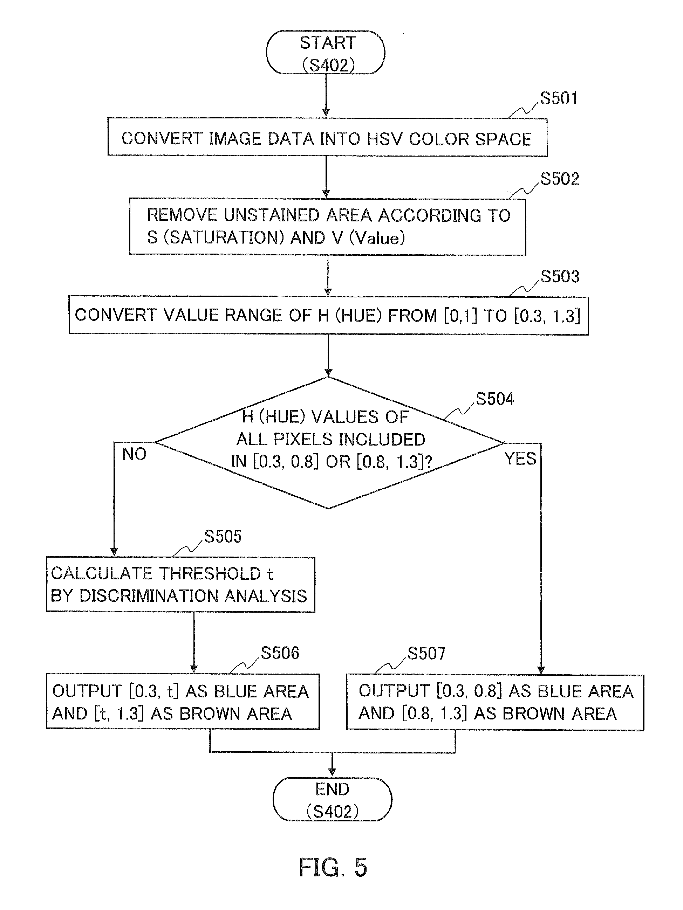

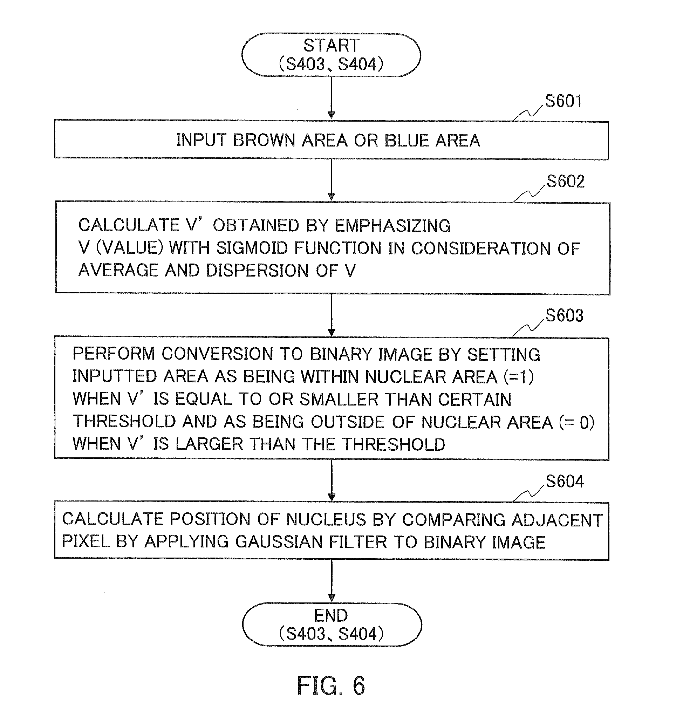

With reference to flowcharts shown in FIGs. 3 to 6, an example of the operation of the system shown in FIG. 1 will be described below as Embodiment 1B of the image diagnosis support method and the image diagnosis support program according to the present invention. In the present embodiment, the description will be made based on the assumption that, in the miRNA staining, positive cell nuclei are stained blue and negative cell nuclei are stained brownish red. It is to be noted, however, that the present invention is not limited thereto, and positive cell nuclei and negative cell nuclei can be counted by a general method for specifying stained cell nuclei, for example. Specifically, for example, regarding a slide to be subjected to the miRNA staining, nuclei may be stained by a general staining method and miRNA staining positive cell nuclei and miRNA staining negative cell nuclei may be counted.

In summary, the method of this embodiment is a method for supporting a diagnosis of a cancer based on a pathological image and includes the following steps (a) to (e). Also, the program of this embodiment is a program for supporting the diagnosis of a cancer based on a pathological image, which causes the computer to execute the steps (a) to (e).
(a) an image obtaining step of obtaining the HE-stained image and the miRNA-stained image as pathological images to be diagnosed
(b) an information obtaining step of obtaining the information of the tumor area in the HE-stained image
(c) a matching step of calculating the matching position of the HE-stained image and the miRNA-stained image obtained in the image obtaining step
(d) a specifying step of specifying the tumor area in the miRNA-stained image based on the information of the tumor area in the HE-stained image obtained in the information obtaining step and the information of the matching position calculated in the matching step
(e) a calculating step of calculating the staining positive cell content rate in the tumor area in the miRNA-stained image based on the information of the tumor area in the miRNA-stained image specified at the specifying step

In more detail, when starting the process, the subject identifier 201, the image identifier 202, the staining information 203, the image data 204, the specimen adjacent information 205, and the HE-stained image tumor area information 206, all of which are a series of data for the subject, are stored in the stained image database 113. The tumor area information 206 is the information obtained by calculating the tumor area from the HE-stained image in advance, or the tumor area information specified by the doctor. When the processing device 120 is activated in such a state, a process shown in FIG. 3 is started.

First, the image identifier of the HE-stained image or the image identifier of the miRNA-stained image, which specifies the diagnostic image, and a miRNA test item request, which specifies a test target cancer marker miRNA, are supplied from the input device 111 to the input acceptance processing section 121 of the processing device 120. The input acceptance processing section 121 passes the information for specifying the diagnostic image and the information for specifying the test target to the stained image and tumor area information obtaining section 122 from the diagnostic image information and test target storage section 131 of the storage section 130. Then, the process is shifted to the stained image and tumor area information obtaining section 122 (step S301).

Next, the stained image and tumor area information obtaining section 122 searches the stained image database 113 for the image identifier in the diagnostic image information and test target storage section 131. When the staining information 203 having the specified image identifier is the HE staining, the stained image and tumor area information obtaining section 122 stores the image data 204 having the image identifier in the HE-stained image data storage section 132. Further, the HE-stained image tumor area information 206 is stored in the HE-stained image tumor area information storage section 133. Also, the miRNA test item in the diagnostic image information and test target storage section 131 is read, the specimen adjacent information 205 in the stained image database 113 is referred to, and the miRNA-stained image data 204, which is the serial section specimen adjacent to the HE-stained image, is stored in the miRNA-stained image data storage section 134.

On the other hand, when the staining information 203 having the specified image identifier is the miRNA staining, the image data 204 having the image identifier is stored in the miRNA-stained image data storage section 134. Also, the specimen adjacent information 205 of the stained image database 113 is referred to, and the HE-stained image data 204, which is the serial section specimen adjacent to the miRNA-stained image, is stored in the HE-stained image data storage section 132. Further, the HE-stained image tumor area information 206 is stored in the HE-stained image tumor area information storage section 133. Then, the process is shifted to the image matching processing section 123 (step S302).

The image matching processing section 123 calculates the matching position of the HE-stained image stored in the HE-stained image data storage section 132 and the miRNA-stained image stored in the miRNA-stained image data storage section 134. The calculation of the matching position is performed by using the phase-only correlation method after adjusting a color scale of both the images, for example. The thus-obtained matching position information is stored in the matching position information storage section 135. Examples of the matching position information include a rotational angle and a horizontal/vertical misalignment width. Then, the process is shifted to the miRNA-stained image tumor area extracting section 124 (step S303).

The miRNA-stained image tumor area extracting section 124 calculates the tumor area in the miRNA-stained image stored in the miRNA-stained image data storage section 134 from the tumor area information of the HE-stained image stored in the HE-stained image tumor area information storage section 133 and the matching position information stored in the matching position information storage section 135. The miRNA-stained image tumor area extracting section 124 stores the thus-obtained tumor area information of the miRNA-stained image in the miRNA-stained image tumor area information storage section 136. Then, the process is shifted to the staining positive cell content rate calculating section 125 (step S304).

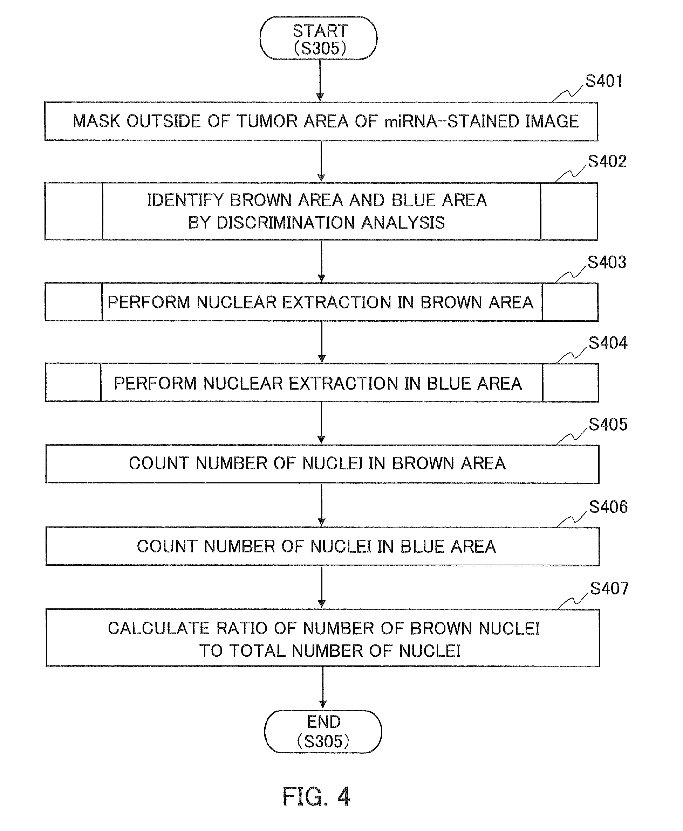

The staining positive cell rate calculating section 125 receives the miRNA-stained image data stored in the miRNA-stained image data storage section 134 and the tumor area information stored in the miRNA-stained image tumor area information storage section 136. Then, the staining positive cell rate calculating section 125 counts the number of staining positive cell nuclei and the number of staining negative cell nuclei in the tumor area, and calculates the staining positive cell content rate to output from the output device 112 (step S305). When the staining positive cell nucleus is stained brownish read and the staining negative cell nucleus is stained blue, the number of nuclei stained brown and the number of nuclei stained blue are counted. This process is performed according to procedures shown in FIGs. 4, 5 and 6 and as will be described below, for example.

First, an outside of the tumor area of the miRNA-stained image data is masked based on the received miRNA-stained image data and tumor area information (step S401). In the tumor area, a brown area, which is an area stained brown, and a blue area, which is an area stained blue, are identified by discrimination analysis (step S402).

In this process, first, the image data is converted into a HSV color space (step S501), an unstained area is removed according to S (Saturation) and V (Value) (step S502), and a value range of H (Hue) is converted from [0, 1] to [0.3,1.3] (step S503). Next, it is checked whether the H (Hue) values of all the pixels are included in either of [0.3, 0.8] range and [0.8, 1.3] range (step S504). When all the pixels are included in one area, [0.3, 0.8] is outputted as the blue area and [0.8, 1.3] is outputted as the brown area (step S507). When the pixels are present in both areas, a threshold t is calculated by the discrimination analysis (step S505), and [0.3, t] is outputted as the blue area and [t, 1.3] is outputted as the brown area (step S506).

Next, nuclear extraction is performed in the brown area (step S403), and subsequently the nuclear extraction is performed in the blue area (step S404). In these steps, first, when the brown area or the blue area is input (step S601), a V' value obtained by emphasizing the V (Value) value with a sigmoid function is calculated in consideration of average and dispersion of the V (value) value (step S602). Then, conversion to a binary image is performed in such a manner that: when the V' value is equal to or smaller than a certain threshold, the inputted area is set as being within a nuclear area (=1); and when the V' value is larger than the threshold, the inputted area is set as being outside of the nuclear area (= 0) (step S603). Next, a position of the nucleus is calculated by performing adjacent pixel comparison by applying a Gaussian filter to the binary image (step S604).

Next, the number of nuclei detected in the brown area is counted (step S405), and the number of nuclei detected in the blue area is counted (step S406). Finally, the ratio of the number of brown nuclei to the total number of nuclei, that is to say, the number of brown nuclei / (the number of brown nuclei + the number of blue nuclei) is calculated (step S407).

Effects of the present embodiment will be described below. In the present embodiment, the HE-stained image and the miRNA-stained image are obtained by the image obtaining unit, and the information of the tumor area in the HE-stained image is obtained by the information obtaining unit. Thereafter, the matching position of the HE-stained image and the miRNA-stained image is calculated by the matching unit. Subsequently, the tumor area in the miRNA-stained image is specified by the specifying unit based on the information of the tumor area in the HE-stained image and the information of the matching position. Then, the staining positive cell content rate in the tumor area in the miRNA-stained image is calculated by the calculating unit based on the information of the tumor area in the miRNA-stained image. Thereby, the staining positive cell content rate can be obtained as a quantitative value. As a result, it becomes possible that the doctor may perform the diagnosis by miRNA staining based on the quantitative value.

Further, since the number of cases of tissue diagnosis and cytological diagnosis is increasing in recent years and the number of pathologists is relatively small, there has been a problem that the pathologists are forced to work for a long time. In this regard, according to the present embodiment, labor burden of doctors and the like can be reduced.

In addition, according to the present embodiment, the tumor area determined in the HE-stained image may be associated with the miRNA-stained image by the matching of the HE-stained image and the miRNA-stained image, which are the serial section specimen images. Also, by applying the discrimination analysis to the H (Hue) value, the brown area and the blue area may be identified, for example. Further, by performing the nuclear extraction in each of the brown area and the blue area, the ratio of the number of brown nuclei to the total number of nuclei may be calculated. Therefore, by presenting the staining positive cell content rate to the doctors or the like, it is possible to provide information helpful to the diagnosis by the doctors, thereby supporting the diagnosis.

### (Embodiment 1C)

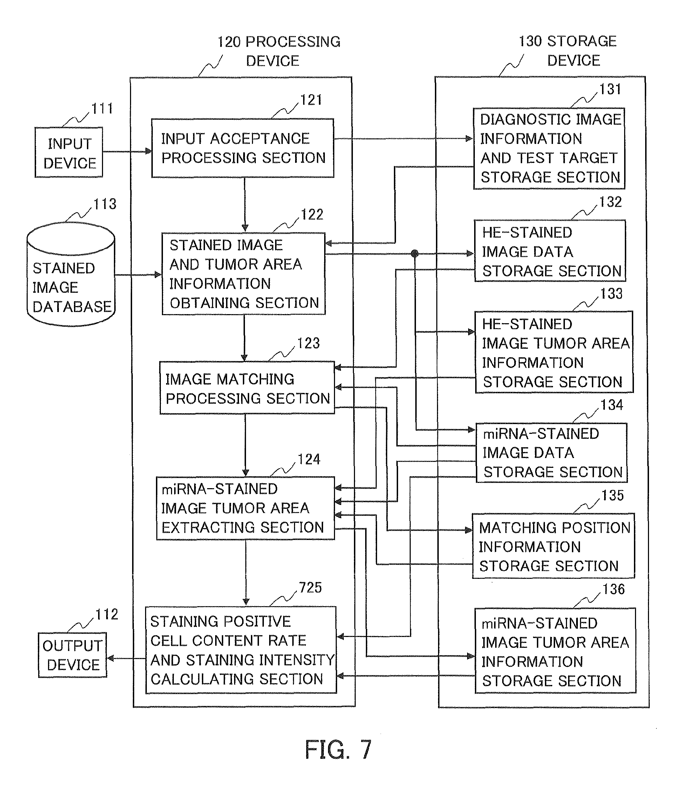

FIG. 7 is a block diagram showing another example of the image diagnosis support system according to the present invention. The system of the present embodiment is different from the system according to Embodiment 1B shown in FIG. 1 in that the staining positive cell content rate calculating section 125 also calculates staining intensity in addition to the staining positive cell content rate. Unless otherwise stated, other configuration and operation are the same as those of Embodiment 1B.

In FIG. 7, a staining positive cell content rate and staining intensity calculating section 725 reads miRNA-stained image data and tumor area information from the miRNA-stained image data storage section 134 and the miRNA-stained image tumor area information storage section 136, respectively. Then, the staining positive cell content rate and staining intensity calculating section 725 counts the number of staining positive cell nuclei and the number of staining negative cell nuclei in the tumor area to calculate the staining positive cell content rate, and further, calculates the staining intensity to output from the output device 112.

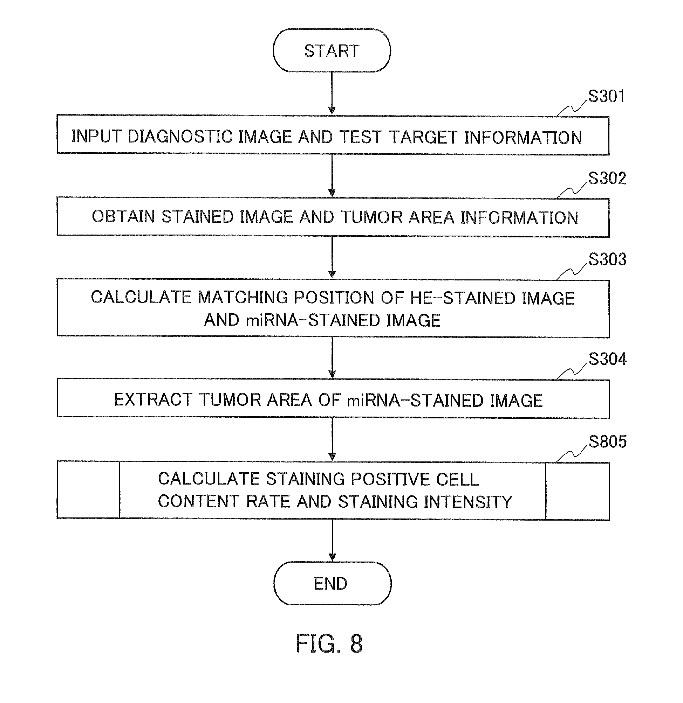

With reference to flowcharts shown in FIGs. 8 to 10, an example of the operation of the system shown in FIG. 7 will be described below as Embodiment 1C of the image diagnosis support method and the image diagnosis support program according to the present invention.

The process of the present embodiment differs from that of Embodiment 1B shown in FIG. 3 in that not only the staining positive cell content rate but also the staining intensity is calculated, and other operations are the same as those in Embodiment 1B.

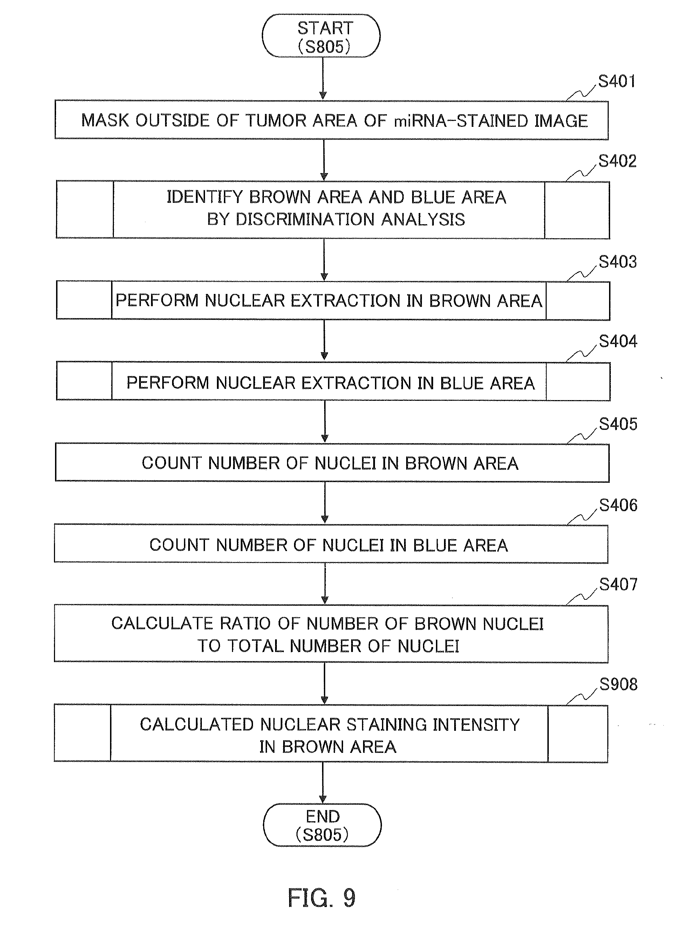

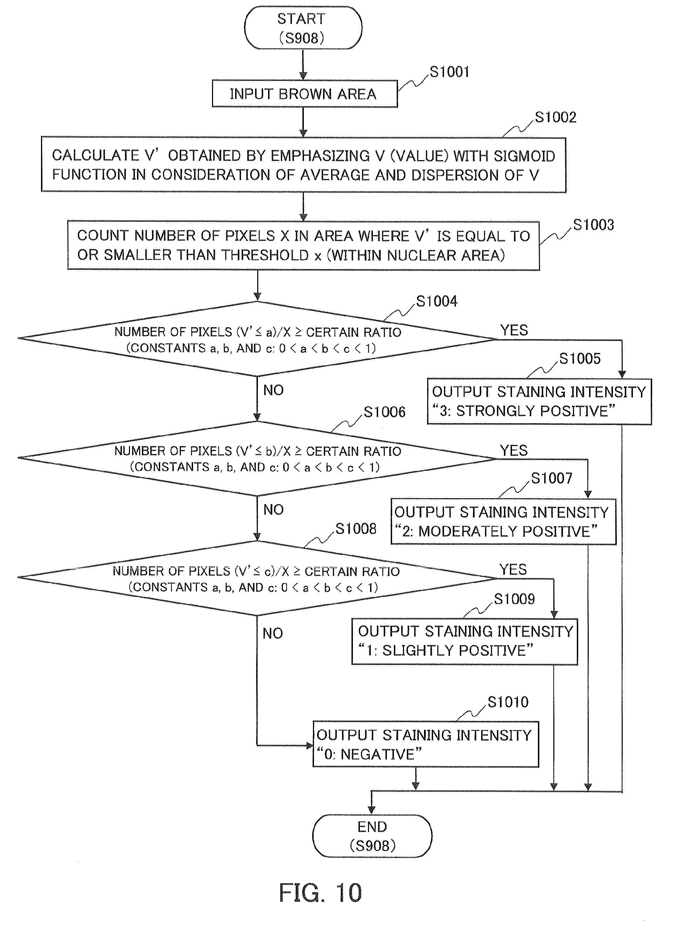

The staining positive cell content rate and staining intensity calculating section 725 receives the miRNA-stained image data stored in the miRNA-stained image data storage section 134 and the tumor area stored in the miRNA-stained image tumor area information storage section 136. Then, the staining positive cell content rate and staining intensity calculating section 725 counts the number of staining positive cell nuclei and the number of staining negative cell nuclei in the tumor area to calculate the staining positive cell content rate, and calculates the staining intensity (0: negative, 1: slightly positive, 2: moderately positive, 3: strongly positive) to output from the output device 112 (step S805). This process is performed according to the procedures shown in FIGs. 9 and 10 and as will be described below, for example.

The process is the same as that of Embodiment 1B shown in FIG. 4 until the step S407 in FIG. 9. Subsequent to the step S407, nuclear staining intensity is calculated in the brown area (step S908).

First, the brown area determined in the step S402 in FIG. 9 is input (step S1001). Then, in consideration of the average and the dispersion of V (Value), V' obtained by emphasizing the V with the sigmoid function is calculated (step S1002). When the V' value is equal to or smaller than a certain threshold x, the inputted area is set as being within a nuclear area and the number of pixels X therein is counted (step S 1003).

Next, constants a, b and c satisfying 0 < a < b < c < 1 are set, and the ratio of the number of pixels satisfying a condition of V ≤ a to the number of pixels in the nuclear area is determined. When the ratio is not smaller than a certain ratio (step S1004), it is outputted as the staining intensity "3: strongly positive" (step S 1005). If it is not the case, the ratio of the number of pixels satisfying a condition of V ≤ b to the number of pixels in the nuclear area is determined. When the ratio is not smaller than a certain ratio (step S1006), it is outputted as the staining intensity "2: moderately positive" (step S1007). If it is not the case, the ratio of the number of pixels satisfying a condition of V ≤ c to the number of pixels in the nuclear area is determined. When the ratio is not smaller than a certain ratio (step S1008), it is outputted as the staining intensity "1: slightly positive" (step S1009). If it is not the case, it is outputted as the staining intensity "0: negative" (step S1010).

Effects of the present embodiment will be described below. Although only the staining positive cell content rate is presented to the doctor in Embodiment 1B, it is possible to present not only the staining positive cell content rate but also the staining intensity to the doctor or the like in Embodiment 1C. Thus, it is possible to provide the information more helpful to the diagnosis by the doctor, thereby supporting the diagnosis. Other effects of the present embodiment are the same as those of Embodiment 1B.

### (Embodiment 1D)

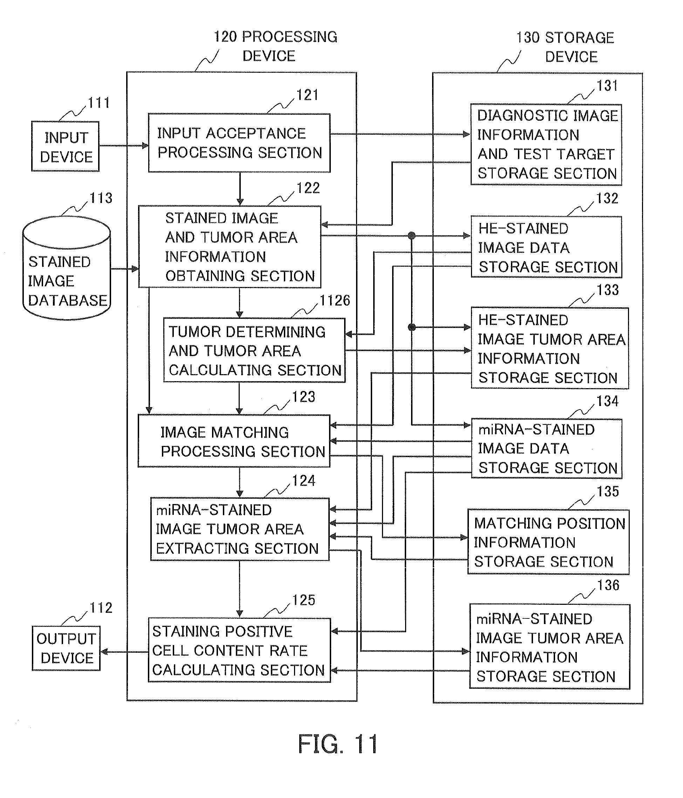

FIG. 11 is a block diagram showing still another example of the image diagnosis support system according to the present invention. The system of the present embodiment differs from the system according to Embodiment 1B shown in FIG. 1 in that the system of the present embodiment is provided with a tumor determining and tumor area calculating section (tumor area calculating unit) 1126. Unless otherwise stated, other configurations and operations are the same as those in Embodiment 1B. In addition, at least one HE-stained image, a miRNA-stained image, which is the specimen of the serial section adjacent to the specimen of the HE-stained image, and specimen adjacent information of the HE-stained image and the miRNA-stained image are accumulated in the stained image database 113. In the present embodiment, the presence of the tumor area information calculated from the miRNA-stained image or determined by the doctor or the like is not imperative.

In FIG. 11, the stained image and tumor area information obtaining section 122 obtains the HE-stained image 204, the miRNA-stained image 204, and the HE-stained image tumor area information 206 from the stained image database 113, and stores them in the HE-stained image data storage section 132, the miRNA-stained image data storage section 134, and the HE-stained image tumor area information storage section 133 of the storage device 130, respectively. Herein, when the tumor area information 206 is present, the process is shifted to the image matching processing section 123; however, when the tumor area information 206 is not present, the process is shifted to the tumor determining and tumor area calculating section 1126.

The tumor determining and tumor area calculating section 1126 reads the HE-stained image data from the HE-stained image data storage section 132, determines the tumor and calculates the tumor area, and shifts the process to the image matching processing section 123. As a method of the tumor determination and a method of the tumor area calculation, those disclosed in Patent Document 1 may be utilized, for example.

Effects of the present embodiment will be described below. In the present embodiment, even when the tumor is not determined in the HE-stained image, a series of processes may be performed by providing the tumor determining and tumor area calculating section. Thus, diagnostic information integrating from a cancer diagnosis to an immunohistochemically-stained image diagnosis can be presented to a doctors or the like. Accordingly, it is possible to provide information helpful to the diagnosis by the doctor, thereby supporting the diagnosis. Other effects of the present embodiment are the same as those in Embodiment 1B.

Meanwhile, in the present embodiment, the staining intensity may be calculated together with the staining positive cell content rate as in Embodiment 1C.

### (Embodiment 1E)

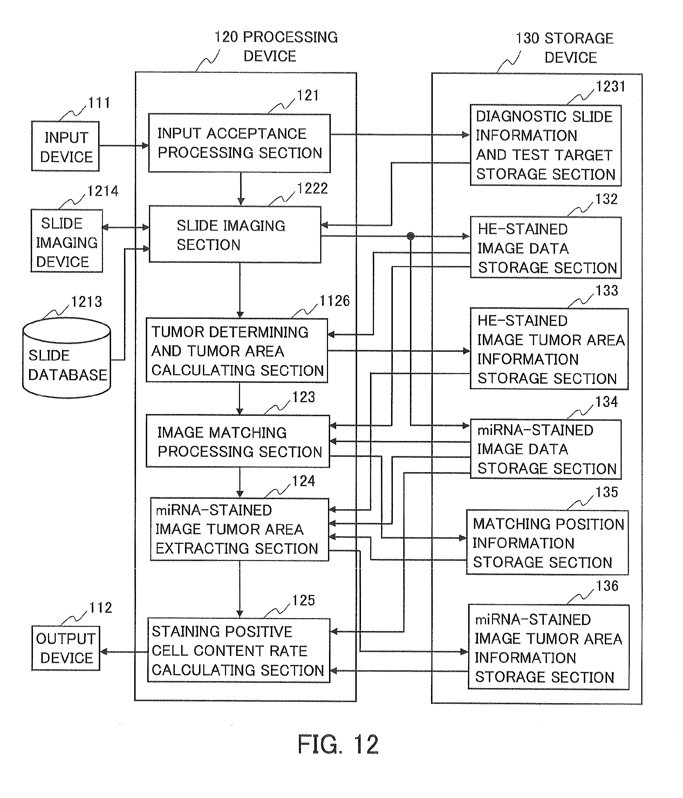

FIG. 12 is a block diagram showing still another example of the image diagnosis support system according to the present invention. The system of the present embodiment includes: a slide imaging section 1222 (slide obtaining unit) that is provided in place of the stained image and tumor area information obtaining section 122 shown in FIG. 1 and the like; a slide database 1213 that is provided in place of the stained image database 113; and a diagnosis slide information and test target storage section 1231 that is provided in place of the diagnostic image information and test target storage section 131. Further, the system is provided with a slide imaging device 1214 and a tumor determining and tumor area calculating section 1126. Unless otherwise stated, other configuration and operation are the same as those in Embodiment 1B.

At least one HE-stained slide, a miRNA-stained slide, which is the specimen of the serial section adjacent to the specimen of the HE-stained slide, and specimen adjacent information of the HE-stained slide and the miRNA-stained slide are accumulated in the slide database 1213. The relevant information regarding the subject is associated to each slide by the subject identifier. The slide imaging device 1214 images a specified slide to convert into digital data.

The input acceptance processing section 121 accepts information for specifying the slide to be diagnosed (slide identifier) and information for specifying the test type from the user and the like through the input device 111. Then, the input acceptance processing section 121 stores them in the diagnostic slide information and test target storage section 1231 of the storage device 130, and shifts the process to the slide imaging section 1222.

The slide imaging section 1222 obtains the HE-stained slide and the miRNA-stained slide, which are the adjacent specimens to be diagnosed, from the slide database 1213. Further, the slide imaging section 1222 obtains the HE-stained image and the miRNA image by imaging the slides obtained by the slide imaging device 1214 and converting them into the digital data. Then, these images are stored in the HE-stained image data storage section 132 and the miRNA-stained image data storage section 134 of the storage device 130, respectively, and the process is shifted to the tumor determining and tumor area calculating section 1126. As described above, in the present embodiment, the slide imaging section 1222 has functions of both the slide obtaining unit and the image obtaining unit.

Effects of the present embodiment will be described below. In the present embodiment, even when a pathological slide is not converted into digital data, a series of processes are performed by providing the slide imaging device, the slide database, and the slide imaging section. Thus, the diagnosis information integrating from the slide imaging to the cancer diagnosis, and further to the immunohistochemically-stained image diagnosis can be presented to the doctor or the like. Accordingly, it is possible to provide the information helpful to the diagnosis by the doctor, thereby supporting the diagnosis. Other effects of the present embodiment are the same as those in Embodiment 1B.

Meanwhile, in the present embodiment, the staining intensity may be calculated together with the staining positive cell content rate as in Embodiment 1C.

The present invention is not limited to the above-described exemplary embodiments, and various modifications may be made. For example, the above-described exemplary embodiments are directed to a case where the input acceptance processing section 121 accepts the image identifier, which specifies the diagnostic image. However, the input acceptance processing section 121 may accept the subject identifier of the diagnostic target instead of the image identifier. In this case, the stained image and tumor area information obtaining section 122 may search the stained image database 113 for the image having the subject identifier and the tumor area information.

### Second Embodiment

The cancer pathological image diagnosis support device according to the present invention (hereinafter referred to as "image diagnosis support device") includes:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and
a diagnosis unit for conducting diagnosis based on the features. The feature determining unit calculates a feature of each of the learning patterns corresponding to each of the feature candidates and determines, as a first feature of the feature set, a feature candidate for which a mutual information quantity with respect to the class information of a set of the learning patterns takes a maximum value; and sequentially determines, under a condition that the determined feature is known, as a subsequent feature of the feature set, a feature candidate for which mutual information quantity between a feature of each learning pattern corresponding to each feature candidate and the class information of an associated one of the learning patterns takes a maximum value. The category table generator unit calculates each feature of each of the learning patterns using the feature set, generates the category table including each feature of the learning patterns and the class information, and classifies the patterns using the category table. The feature extraction unit calculates each feature of the input patterns using the feature set. The diagnosis unit diagnoses the input patterns according to the result of the diagnosis and the category table.

The image diagnosis support device according to the present invention includes:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and a diagnosis unit for conducting diagnosis based on the features. The feature determining unit prepares a predetermined number of sets of the learning patterns to be subjected to a transition according to a value of the feature, calculates the feature of each of the learning patterns corresponding to each of the feature candidates, determines, as a first feature of the feature set, a feature candidate for which a mutual information quantity with respect to the class information of a set of the learning patterns takes a maximum value, distributes the learning pattern with a weight according to the feature thus determined, sequentially causes a transition of the learning pattern to one of the sets corresponding to the feature, and sequentially determines, under a condition that information about the sets respectively containing the learning patterns and the determined feature are known, as a subsequent feature of the feature set, a feature candidate for which mutual information quantity between a feature of each leaning pattern corresponding to each feature candidate and the class information of an associated one of the learning patterns takes a maximum value. The category table generator unit calculates each feature of each of the learning patterns using the feature set, generates the category table including each feature of the learning patterns and the class information, and classifies the patterns using the category table. The feature extraction unit calculates each feature of the input patterns using the feature set. The diagnosis unit causes a transition of each of the input patterns according to each feature of the input patterns and a transition table sequentially having recorded a set to which the learning pattern belongs at determination of each feature of the feature set and diagnoses the input patterns according to a set to which the input pattern belongs as a result of the transition.

The image diagnosis support device according to the present invention includes:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and
a diagnosis unit for conducting diagnosis based on the features. The feature determining unit prepares a predetermined number of sets of the learning patterns to be subjected to a transition according to a value of the feature, calculates the feature of each of the learning patterns corresponding to each of the feature candidates, determines, as a first feature of the feature set, a feature candidate for which a mutual information quantity with respect to the class information of a set of the learning patterns takes a maximum value, distributes the learning pattern with a weight according to the feature thus determined, sequentially causes a transition of the learning pattern to one of the sets corresponding to the feature, and sequentially determines, under a condition that information about the sets respectively containing the learning patterns and the determined feature are known, as a subsequent feature of the feature set, a feature candidate for which mutual information quantity between a feature of each learning pattern corresponding to each feature candidate and the class information of an associated one of the learning patterns takes a maximum value. The category table generator unit calculates each feature of each of the learning patterns using the feature set, and classifies the patterns using the category table including each feature of the learning patterns and the class information. The feature extraction unit calculates, using the feature set, each feature of the input patterns indicating a probability with which the feature at an order takes a predetermined value. The diagnosis unit calculates, according to each feature of the input patterns and a transition table sequentially having recorded a set to which the learning pattern belongs at determination of each feature of the feature set, a probability with which the input pattern includes predetermined class information and then conducts the diagnosis.

In the image diagnosis support device of the present invention, it is preferable that the learning pattern input unit and the pattern input unit select, from R, G, and B values of each pixel in the pathological image stained in advance, pixels belonging to a color region to which a cell nucleus of a predetermined tumor belongs, calculate the distance between the center of distribution of the color region and each pixel belonging to the color region, assign a signal to each pixel according to the distance, detect a peak of distribution of the signals in the pathological image, and input an image centered on the peak as the learning pattern.

In the image diagnosis support device of the present invention, it is preferable that the feature candidates generated by the feature generator unit include a feature candidate obtained from a feature extraction function.

In the image diagnosis support device of the present invention, it is preferable that the feature candidates generated by the feature generator unit include a feature candidate obtained using a feature extraction function obtained by normalizing a complex Gabor function.

In the image diagnosis support device according to the present invention, it is preferable that the feature candidates generated by the feature candidate generator unit includes a feature candidate discriminating a color of the tumor.

In the image diagnosis support device according to the present invention, it is preferable that the feature determining unit compares the signal of each pixel included in the learning patterns calculated by the learning pattern input unit with a predetermined threshold value.

In the image diagnosis support device according to the present invention, it is preferable that the feature determining unit compares the signal of each pixel included in the learning patterns calculated by the learning pattern input unit with a mean value of signals of pixels in the proximity of the pixel.

In the image diagnosis support device according to the present invention, it is preferable that the feature determining unit conducts an operation for each of the learning patterns using a predetermined noise parameter for each of the feature candidates.

In the image diagnosis support device according to the present invention, it is preferable that the feature determining unit calculates, as the feature of each of the learning patterns corresponding to each of the feature candidates, a probability with which the feature of the learning pattern takes a predetermined value.

In the image diagnosis support device according to the present invention, it is preferable that, when the learning patterns can be classified irrespectively of the values of the features, the category table generator unit substitutes a redundant term for the value of the feature at an associated position of the category table.

In the image diagnosis support device according to the present invention, it is preferable that each of the features of the input patterns is a value of a probability with which the feature at an order takes a predetermined value; and the diagnosis unit makes a judgment by calculating, by use of the features, a probability with which each of the feature patterns contained in the category table takes a predetermined value of class information.

Furthermore, the image diagnosis support program according to the present invention is **characterized in that** it can cause a computer to execute the image diagnosis method of the present invention. The image diagnosis support program of the present invention is, for example, an image diagnosis support program for use with an image diagnosis support device including:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and
a diagnosis unit for conducting diagnosis based on the features. The program causes the respective units to execute the following processing: processing in which the feature determining unit calculates a feature of each of the learning patterns corresponding to each of the feature candidates and determines, as a first feature of the feature set, a feature candidate for which a mutual information quantity with respect to the class information of a set of the learning patterns takes a maximum value; and sequentially determines, under a condition that the determined feature is known, as a subsequent feature of the feature set, a feature candidate for which mutual information quantity between a feature of each learning pattern corresponding to each feature candidate and the class information of an associated one of the learning patterns takes a maximum value;
processing in which the category table generator unit calculates each feature of each of the learning patterns using the feature set and classifies the patterns using the category table including each feature of the learning patterns and the class information;
processing in which the feature extraction unit calculates each feature of the input patterns using the feature set; and
processing in which the diagnosis unit diagnoses the input patterns according a result of the calculation and the category table. Specifically, the program according to the present invention is a program for causing the respective units of the image diagnosis support device to execute the above-described processing steps.

The image diagnosis support program of the present invention is an image diagnosis support program for use with an image diagnosis support device including:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and
a diagnosis unit for conducting diagnosis based on the features. The program causes the respective units to execute the following processing: processing in which the feature determining unit prepares a predetermined number of sets of the learning patterns to be subjected to a transition according to a value of the feature, calculates the feature of each of the learning patterns corresponding to each of the feature candidates, determines, as a first feature of the feature set, a feature candidate for which a mutual information quantity with respect to the class information of a set of the learning patterns takes a maximum value, distributes the learning pattern with a weight according to the feature thus determined, sequentially causes a transition of the learning pattern to one of the sets corresponding to the feature, and sequentially determines, under a condition that information about the sets respectively containing the learning patterns and the determined feature are known, as a subsequent feature of the feature set, a feature candidate for which mutual information quantity between a feature of each learning pattern corresponding to each feature candidate and the class information of an associated one of the learning patterns takes a maximum value;
processing in which the category table generator unit calculates each feature of each of the learning patterns using the feature set, generates the category table including each feature of the learning patterns, and the class information and classifies the patterns using the category table; and
processing in which the diagnosis unit causes a transition of each of the input patterns according to each feature of the input patterns and a transition table sequentially having recorded a set to which the learning pattern belongs at determination of each feature of the feature set and diagnoses the input patterns according a set to which the input pattern belongs as a result of the transition. Specifically, the program according to the present invention is a program for causing the respective units of the image diagnosis support device to execute the above-described processing steps.

The image diagnosis support program of the present invention is an image diagnosis support program for use in an image diagnosis support device including:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and
a diagnosis unit for conducting diagnosis based on the features. The program causes the respective units to execute a series of processing as described below: processing in which the feature determining unit prepares a predetermined number of sets of the learning patterns to be subjected to a transition according to a value of the feature, calculates the feature of each of the learning patterns corresponding to each of the feature candidates, determines, as a first feature of the feature set, a feature candidate for which a mutual information quantity with respect to the class information of a set of the learning patterns takes a maximum value, distributes the learning pattern with a weight according to the feature thus determined, sequentially causes a transition of the learning pattern to one of the sets corresponding to the feature, and sequentially determines, under a condition that information about the sets respectively containing the learning patterns and the determined feature are known, as a subsequent feature of the feature set, a feature candidate for which mutual information quantity between a feature of each learning pattern corresponding to each feature candidate and the class information of an associated one of the learning patterns takes a maximum value;
processing in which the category table generator unit calculates each feature of each of the learning patterns using the feature set and classifies the patterns using the category table including each feature of the learning patterns and the class information;
processing in which the feature extracting unit calculates, using the feature set, each feature of the input patterns indicating a probability with which the feature at an order takes a predetermined value; and
processing in which the diagnosis unit calculates, according to each feature of the input patterns and a transition table sequentially having recorded a set to which the learning pattern belongs at determination of each feature of the feature set, a probability with which the input pattern includes predetermined class information and then conducts the diagnosis. Specifically, the program according to the present invention is a program for causing the respective units of the image diagnosis support device to execute the above-described processing steps (hereinafter the same).

In the image diagnosis support program of the present invention, it is preferable that, for example, the learning pattern input unit and the pattern input unit includes processing to select, from R, G, and B values of each pixel in the pathological image stained in advance, pixels belonging to a color region to which a cell nucleus of a predetermined tumor belongs; processing to calculate the distance between a center of distribution of the color region and each pixel belonging to the color region; processing to assign a signal to the pixel according to the distance; processing to detect a peak of distribution of the signals in the pathological image; and processing to input an image centered on the peak as the learning pattern.

In the image diagnosis support program of the present invention, it is preferable that the feature candidates generated by the feature generator unit include a feature candidate obtained from a feature extraction function.

In the image diagnosis support program of the present invention, it is preferable that the feature candidates generated by the feature generator unit include a feature candidate obtained using a feature extraction function obtained by normalizing a complex Gabor function.

In the image diagnosis support program of the present invention, it is preferable that the feature candidates generated by the feature generator unit include a feature candidate discriminating a color of the tumor.

The image diagnosis support program of the present invention preferably includes, for example, the processing in which the feature determining unit compares the signal of each pixel included in the learning patterns calculated by the learning pattern input unit with a predetermined threshold value.

The image diagnosis support program of the present invention preferably includes, for example, processing in which the feature determining unit compares the signal of each pixel included in the learning patterns calculated by the learning pattern input unit with a mean value of signals of pixels in the proximity of the pixel.

The image diagnosis support program of the present invention preferably includes, for example, processing in which the feature determining unit conducts operation for each of the learning patterns using a predetermined noise parameter for each of the feature candidates.

The image diagnosis support program of the present invention preferably includes, for example, processing in which the feature determining unit calculates, as the feature of each of the learning patterns corresponding to each of the feature candidates, a probability with which the feature of the learning pattern takes a predetermined value.

The image diagnosis support program of the present invention preferably includes, for example, processing in which when the learning patterns can be classified irrespectively of the values of the features, the category table generator unit substitutes a redundant term for the value of the feature at an associated position of the category table.

In the image diagnosis support program of the present invention, it is preferable that each of the features of the input patterns is a value of a probability with which the feature at an order takes a predetermined value, and the program further includes processing in which the diagnosis unit calculates, by use of the features, a probability with which each of the feature patterns contained in the category table takes a predetermined value of class information so as to make a determination.

The present invention provides a pathological diagnosis support method using a pathological diagnosis support device including:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and
a diagnosis unit for conducting diagnosis based on the features. The feature determining unit performs the step of calculating a feature of each of the learning patterns corresponding to each of the feature candidates, determining, as a first feature of the feature set, a feature candidate for which a mutual information quantity with respect to the class information of a set of the learning patterns takes a maximum value, and sequentially determining, under a condition that the determined feature is known, as a subsequent feature of the feature set, a feature candidate for which mutual information quantity between a feature of each learning pattern corresponding to each feature candidate and the class information of an associated one of the learning patterns takes a maximum value. The category table generator unit performs the step of calculating each feature of each of the learning patterns using the feature set and classifying the patterns using the category table including each feature of the learning patterns and the class information. The feature extraction unit performs the step of calculating each feature of the input patterns using the feature set. The diagnosis unit performs the step of diagnosing the input patterns according a result of the calculation and the category table. In the method of the present invention, use of the image diagnosis support device is not essential, and it may be a method in which the above-described respective various steps are performed.

The present invention provides an image pathological diagnosis support method using a pathological diagnosis support device including:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and a diagnosis unit for conducting diagnosis based on the features. The feature determining unit performs the step of preparing a predetermined number of learning patterns to be subjected to a transition according to a value of the feature, calculating the feature of each of the learning patterns corresponding to each of the feature candidates, determining, as a first feature of the feature set, a feature candidate for which a mutual information quantity with respect to the class information of a set of the learning patterns takes a maximum value, distributing the learning pattern with a weight according to the feature thus determined, sequentially causing a transition of the learning pattern to one of the sets corresponding to the feature, and sequentially determining, under a condition that the determined feature is known, as a subsequent feature of the feature set, a feature candidate for which mutual information quantity between a feature of each learning pattern corresponding to each feature candidate and the class information of an associated one of the learning patterns takes a maximum value. The category table generator unit performs the step of calculating each feature of each of the learning patterns using the feature set and classifying the patterns using the category table including each feature of the learning patterns and the class information. The feature extraction unit performs the step of calculating each feature of the input patterns using the feature set. The diagnosis unit performs the step of causing a transition of each of the input patterns according to each feature of the input patterns and a transition table sequentially having recorded a set to which the learning pattern belongs at determination of each feature of the feature set and diagnosing the input patterns according a set to which the input pattern belongs as a result of the transition. In the method of the present invention, use of the image diagnosis support device is not essential, and it may be a method in which the above-described respective various steps are performed.

The present invention provides an image pathological diagnosis support method using a pathological diagnosis support device including:
a learning pattern input unit for obtaining, from a pathological image to be used for learning, images centered on a tumor and inputting thereto the images as learning patterns;
a learning pattern storage unit for storing and keeping the learning patterns to which class information is attached;
a feature candidate generator unit for generating a plurality of feature candidates;
a feature determining unit for determining a feature set of features suitable for diagnosis using the feature candidates generated by the feature candidate generator unit;
a feature storage unit for storing and keeping the set of features determined by the feature determining unit;
a category table generator unit for generating a category table;
a pattern input unit for obtaining, from a pathological image to be diagnosed, images centered on a tumor candidate and inputting the images as input patterns;
a feature extraction unit for extracting features from the input patterns; and a diagnosis unit for conducting diagnosis based on the features. The feature determining unit performs the step of preparing a predetermined number of learning patterns to be subjected to a transition according to a value of the feature, calculating the feature of each of the learning patterns corresponding to each of the feature candidates, determining, as a first feature of the feature set, a feature candidate for which a mutual information quantity with respect to the class information of a set of the learning patterns takes a maximum value, distributing the learning pattern with a weight according to the feature thus determined, sequentially causing a transition of the learning pattern to one of the sets corresponding to the feature, and sequentially determining, under a condition that the determined feature is known, as a subsequent feature of the feature set, a feature candidate for which mutual information quantity between a feature of each learning pattern corresponding to each feature candidate and the class information of an associated one of the learning patterns takes a maximum value. The category table generator unit performs the step of calculating each feature of each of the learning patterns using the feature set and classifying the patterns using the category table including each feature of the learning patterns and the class information. The feature extraction unit performs the step of calculating, using the feature set, each feature of the input patterns indicating a probability with which the feature at an order takes a predetermined value. The diagnosis unit performs the step of calculating, according to each feature of the input patterns and a transition table sequentially having recorded a set to which the learning pattern belongs at determination of each feature of the feature set, a probability with which the input pattern includes predetermined class information and then conducting the diagnosis. In the method of the present invention, use of the image diagnosis support device is not essential, and it may be a method in which the above-described respective various steps are performed.

In the image diagnosis support method of the present invention, it is preferable that the learning pattern input unit and the pattern input unit performs the step of selecting, from R, G, and B values of each pixel in the pathological image stained in advance, pixels belonging to a color region to which a cell nucleus of a predetermined tumor belongs; the step of calculating distance between a center of distribution of the color region and each pixel belonging to the color region; the step of assigning a signal to the pixel according to the distance; the step of detecting a peak of distribution of the signals in the pathological image; and the step of inputting an image centered on the peak as the learning pattern.

In the image diagnosis support method of the present invention, it is preferable that the feature candidates generated by the feature generator unit include a feature candidate obtained from a feature extraction function.

In the image diagnosis support method of the present invention, it is preferable that the feature candidates generated by the feature generator unit include a feature candidate obtained using a feature extraction function obtained by normalizing a complex Gabor function.

In the image diagnosis support method of the present invention, it is preferable that the feature candidates generated by the feature generator unit include a feature candidate discriminating a color of the tumor.

In the image diagnosis support method of the present invention, it is preferable that the feature determining unit performs the step of comparing the signal of each pixel included in the learning patterns calculated by the learning pattern input unit with a predetermined threshold value.

In the image diagnosis support method of the present invention, it is preferable that the feature determining unit performs the step of comparing the signal of each pixel included in the learning patterns calculated by the learning pattern input unit with a mean value of signals of pixels in the proximity of the pixel.

In the image diagnosis support method of the present invention, it is preferable that the feature determining unit performs the step of conducting operation for each of the learning patterns using a predetermined noise parameter for each of the feature candidates.

In the image diagnosis support method of the present invention, it is preferable that the feature determining unit performs the step of calculating, as the feature of each of the learning patterns corresponding to each of the feature candidates, a probability with which the feature of the learning pattern takes a predetermined value.

In the image diagnosis support method of the present invention, it is preferable that, when the learning patterns can be classified irrespectively of the values of the features, the category table generator unit performs the step of substituting a redundant term for the value of the feature at an associated position of the category table.

In the image diagnosis support method of the present invention, it is preferable that each of the features of the input patterns is a value of a probability with which the feature at an order takes a predetermined value, and the diagnosis unit performs the step of making a judgment by calculating, by use of the features, a probability with which each of the feature patterns contained in the category table takes a predetermined value of class information.

The present invention provides an image diagnosis support system including:
an information processing terminal for keeping pathological image data including a pathological image and information unique to a patient attached to the image; and
an image diagnosis server for diagnosing the pathological image data.

The image diagnosis server includes:
the image diagnosis support device according to the present invention for diagnosing the pathological image contained in the pathological image data; and
a diagnosis result storage unit for storing a diagnosis result from the image diagnosis support device together with the information unique to the patient. The information processing terminal requests the transmission of the diagnosis result together with the information unique to the patient, and the image diagnosis server compares the information unique to the patient received from the information processing terminal with the information unique to the patient stored together with the diagnosis result and then transmits the diagnosis result to the information processing terminal if the information unique to the patient received from the terminal matches the information unique to the patient stored together with the diagnosis result.,

The image diagnosis support system of the present invention preferably further includes an accounting server for keeping amounts of use charge respectively of the image diagnosis support device and the information processing terminal.

In the image diagnosis support system of the present invention, it is preferable that, when the diagnosis result storage unit stores the diagnosis result, the accounting server accumulates an amount of use charge of the image diagnosis support system.

In the image diagnosis support system of the present invention, it is preferable that, when the information processing terminal receives the diagnosis result, the accounting server accumulates an amount of use charge of the information processing terminal.

The server according to the present invention is a server for use in the image diagnosis support system of the present invention. The server includes: a server side transmission unit that transmits information in the server to the terminal via the communication network; a server side receiver unit that receives information transmitted from the terminal via the communication network; the image diagnosis support device according to the present invention for diagnosing a subject using the pathological image data; and a diagnosis result storage unit for storing a diagnosis result by the image diagnosis support device together with the information unique to the patient. The server compares the information unique to the patient received from the information processing terminal with the information unique to the patient stored together with the diagnosis result and then transmits the diagnosis result to the information processing terminal if the information unique to the patient received from the terminal matches the information, unique to the patient stored together with the diagnosis result.

The terminal of the present invention is a terminal for use in the image diagnosis support system of the present invention. The terminal is an information processing terminal for keeping pathological image data including a pathological image and information unique to a patient attached to the image. The terminal includes: a terminal side transmission unit that transmits information in the terminal to the server via the communication network; and a terminal side receiver unit that receives information transmitted from the server via the communication network. The terminal requests the transmission of the diagnosis result accompanying the information unique to the patient, and receives the diagnosis result transmitted from the server.

In accordance with the image diagnosis support device, the image diagnosis support method, the image diagnosis support program, and the image diagnosis support system according to the present invention, in consideration of importance of changes taking place in a cell nucleus, peripheral tissues thereof, and the like in the discrimination as to whether the property of a tumor is benign or malignant, subimages (image data of subimages) primarily including cell nuclei and interstitium are extracted from a pathological image, and the subimages are stored as learning patterns and input patterns. On the basis of the subimages, the presence or absence of a tumor and benignity or malignity thereof can be determined with high accuracy in a short period of time. In the present invention, the pathological image is the above-described cancer marker-stained image.

In accordance with the second embodiment of the invention as described above, for example, in consideration of importance of changes taking place in a cell nucleus, peripheral tissues thereof, and the like in the discrimination as to whether the property of a tumor is benign or malignant, the subimages are extracted, and the subimages are stored as learning patterns and input patterns. Thus, on the basis of the subimages, the presence or absence of a tumor and benignity or malignity thereof, for example, can be determined with high accuracy in a short period of time

Hereinafter, the present invention will be described with reference to Embodiments 2A and 2B. In the following, a cancer marker-stained image is referred to as a "miRNA-stained image". It is to be noted, however, the present invention is by no means limited to these embodiments.

### (Embodiment 2A)

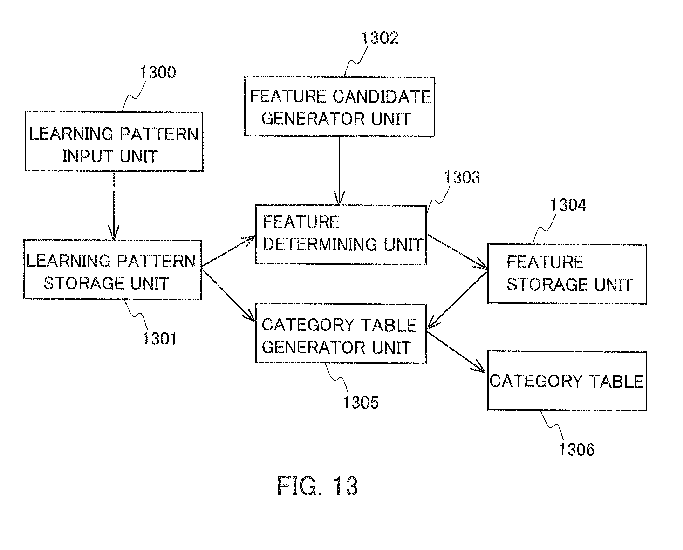

FIG. 13 is a block diagram showing the configuration of an image diagnosis support device according to the present embodiment. As shown in FIG. 13, the image diagnosis support device according to the present embodiment includes a learning pattern input unit 1300, a learning pattern storage unit 1301, a feature candidate generator unit 1302, a feature determining unit 1303, a feature storage unit 1304, a category table generator unit 1305, and a category table 1306.

The learning pattern input unit 1300 extracts subimages including cell nuclei, cytoplasm, and the like from the miRNA-stained image and then stores the subimages in the learning pattern storage unit 1301.

The learning pattern storage unit 1301 is a unit that stores and keeps therein a desired number of subimages used for learning.

The feature candidate generator unit 1302 is a unit that sequentially creates feature candidates using a predetermined number of feature parameter sets.

The feature determining unit 1303 determines an optimal feature set most suitable for the pattern discrimination among the feature candidates produced by the feature candidate generator unit 1302.

The feature storage unit 1304 is a unit that stores and keeps therein the feature set determined by the feature determining unit 1303.

The category table generator unit 1305 is a unit that creates a category table 1306 for diagnosis using the feature set determined by the feature determining unit 1303.

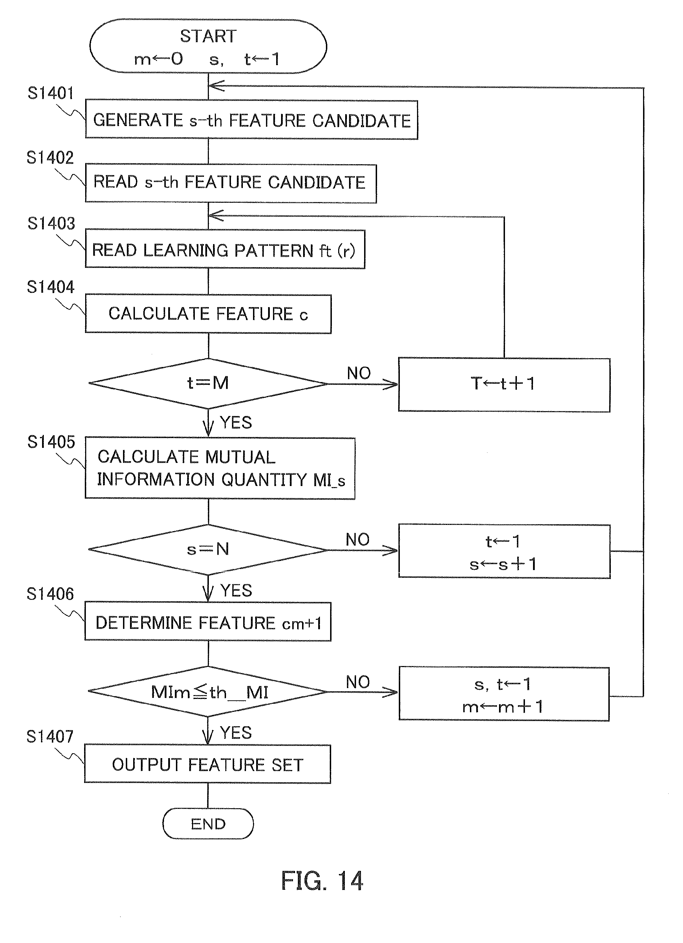

Referring next to FIG. 14, description will be given of a procedure of feature determination processing. Fig. 14 is a flowchart to explain the procedure of the feature determination processing executed in the image diagnosis support device according to the present embodiment.

The feature candidate generator unit 1302 sequentially creates feature candidates according to a large number (e.g., N) of feature parameter sets specified beforehand (S1401). In the present embodiment, among the N feature parameter sets, parameter sets 1 to N_1 are feature candidates regarding textures, parameter sets N_1 + 1 to N_1 + N_2 are feature candidates regarding colors, and parameter sets N_1 + N_2 + 1 to N are feature candidates regarding colors averaged using peripheral pixels. Although the feature candidates regarding textures, colors, and colors averaged using peripheral pixels are designated in this embodiment, the feature candidates are not limited thereto. That is, any element required to determine a feature of each pixel and contained in a pathological image can be generated as a feature candidate.

Next, description will be given of a method for determining features possessed by subimages according to feature candidates generated by the feature candidate generator unit 1302. The features are determined through either one of the following procedures 1 to 3.

### Procedure 1

The feature candidate generator unit 1302 first acquires an s-th feature parameter set. s = 1 to N, and processing starts with s = 1. If s ≤ N_1, the feature candidate generator unit 1302 substitutes the s-th feature parameter set (k_s, r₀_s, σ_s, th_s) for (k, r₀, σ, th) to generate a complex Gabor function Gab and a Gaussian function G as shown in the following expression (1) including parameters k, r₀, and σ. If s ≤ N_1, subimages in colors stored as learning patterns in the learning pattern storage unit 1301 are converted to gray scale images according to a gray scale. A feature c is calculated using the thus-obtained gray scale images.

$\begin{matrix}Gab \left(r:k,{r}_{0},σ\right) \\ = exp \left(ik⁢\left(r-{r}_{0}\right)-{\left|r-{r}_{0}\right|}^{2}/\left(2⁢{σ}^{2}\right)\right) \\ G \left(r:{r}_{0},σ\right) \\ = exp \left(-{\left|r-{r}_{0}\right|}^{2}/\left(2⁢{σ}^{2}\right)/\left(2⁢{πσ}^{2}\right)\right)\end{matrix}$

In expression (1), r = (x, y) indicates a position vector and i² = -1. The feature candidate generator unit 1302 delivers the complex Gabor function Gab and the Gaussian function G in the expression (1) to the feature determining unit 1303 together with a threshold parameter th and an identification number s of the feature candidate (step S 1402).

The learning pattern storage unit 1301 sends to the feature determining unit 1303 pairs of data items each including one of predetermined M subimages I_t (r, i_rgb) (t = 1 to M) and a class qt (t = 1 to M) to which the subimage belongs (step S 1403). In the description of the present embodiment, the device uses two classes (q = 0 or 1) for simplicity of description. The present invention is not limited thereto, and is of course applicable to a case including three classes or more.

Using the subimages sequentially received from the learning pattern storage unit 1301, the feature determining unit 1303 calculates the feature c according to the following expression (2) using the feature candidates (step S1404). The feature candidates are, for example, the complex Gabor function and the Gaussian function of expression (1) as well as other parameters. The calculation is repeatedly carried out for all learning patterns (M patterns) assuming that the t-th learning pattern is I_t (r, i_rgb).

$\begin{matrix}a = | {\sum }_{r} ⁢ I_t \left(r, i_rgb\right) ⁢ Gab \left(r;k,{r}_{0},σ\right) {|}^{2} / {\sum }_{r} I_t ⁢ {\left(r, i_rgb\right)}^{2} ⁢ G \left(r:{r}_{0},σ\right) \\ c = 1 if a ≧ th \\ c = 0 otherwise\end{matrix}$

In the upper row of expression (2), the denominator is a normalization (standardization) factor to suppress variations in the value of "a" due to the size of a learning pattern (luminance of an image). The denominator may be substituted with another normalization factor. The normalization factor is omissible depending on learning patterns to be dealt with.

### Procedure 2

The feature candidate generator unit 1302 first acquires an s-th feature parameter set. s is 1 to N, and the processing starts with s = 1. If N_1 + 1 ≤ s ≤ N_1 + N_2, the feature candidate generator unit 1302 delivers the s-th feature parameter set (x_s, y_s, color_index) and an identification number s of the feature candidate to the feature determining unit 1303 (step S1402).

In this description, (x_s, y_s) indicates a position of a pixel determining feature c in a subimage, and color_index (= 1 to 4) represents a color corresponding to the feature c.

Incidentally, color_index (= 1 to 4) corresponds to colors, for example, as described below.

miRNA staining of a cell by the cancer marker miRNA is an important factor in characterizing a pathological image. Thus, it is preferable to stain a cell nucleus, cytoplasm, interstitium, a pore, and the like with different colors. Regarding the miRNA-stained image resulting from such staining, color_index = 1, 2, 3, 4 are assigned respectively to the colors of the cell nucleus, the cytoplasm, the interstitium, and the pore.

The learning pattern storage unit 1301 delivers to the feature determining unit 1303 data item pairs each of which includes one of the predetermined M learning patterns (color subimages) I_t (r, i_rgb) and a class qt (t = 1 to M) to which the learning pattern belongs (step S1403). r is pixel coordinates and i_rgb = 1 to 3 are parameters designating r, g, b signals of pixels, and t = 1 to M. In the present embodiment, the device handles two classes (q = 0 or 1) for simplicity of description. The present invention is not limited thereto, and is of course applicable to a case including three classes or more.

For the learning patterns received from the learning image storage unit 1301 (step S1403), the feature determining unit 1303 determines a color of a pixel placed at a position (x_s, y_s) in the learning pattern in the following manner. If the color matches a color designated by a parameter color_index, the feature determining unit 1303 sets the value of the feature c to one. Otherwise, the feature determining unit 1303 sets the value to zero (step S1404). Assuming a t-th learning pattern as I_t (r, i_rgb), the operation to determine the value of the feature c is repeatedly conducted for all learning patterns (M patterns).

For the color of a cell nucleus (color_index =1), a hematoxylin signal of each pixel calculated in the subimage detection is compared with a threshold value (e.g., 0.25). If the signal is more than the threshold value, it is determined that the color of the pixel is the color of the cell nucleus. To adaptively determine the threshold value, the values of the hematoxylin signals are added to each other only for the pixels of which the hematoxylin signal value is at least, for example, 0.25 in the vicinity of the pixel under consideration to thereby calculate a mean value of the hematoxylin signal values. The mean value is multiplied by, for example, 0.9 to obtain a threshold value of the pixel under consideration. If the hematoxylin signal value of the pixel under consideration is more than the threshold value, the color of the pixel may be regarded as the color of the nucleus.

For other than the cell nucleus, that is, for a pore, cytoplasm, and interstitium, the colors are classified according to predetermined color regions. For example, an HSV conversion is carried out for the R, G, and B values of each pixel, and these values are converted into values of hue (H = 0 to 1), saturation (S = 0 to 1), and value (V = 0 to 1). If a pixel under consideration is not classified as a pixel having the color of the cell nucleus and has value V > 0.92 and saturation S < 0.2, the color of the pixel is regarded as that of the pore (color_index = 2). If the pixel color is neither that of the cell nucleus nor that of the pore and has hue H < 0.9 and value V < 0.85, it is regarded that the pixel color is the color of the cytoplasm (color_index = 3). In other than the above cases, it is determined that the color of the pixel is the color of the interstitium (color_index = 4).

### Procedure 3

The feature candidate generator unit 1302 first acquires an s-th feature parameter set. s = 1 to N, and processing starts with s = 1. If N_1 + N_2 + 1 ≤ s ≤ N, the feature candidate generator unit 1302 feeds the s-th feature parameter set (x_s, y_s, color_index, th_s) and an identification number s of the feature candidate to the feature determining unit 1303 (step S1402).

In the description, (x_s, y_s) designates a position of a pixel determining the feature c in a subimage, color_index (= 1 to 4) indicates a color corresponding to the feature c, and th represents a threshold parameter.

For the learning patterns received from the learning pattern storage unit 1301 (step S 1403), the feature determining unit 1303 determines the feature c in the following manner (step S 1404). Assuming that a t-th learning pattern is represented as I_t (r, i_rgb), the operation to determine the feature c is repeatedly performed for all learning patterns (M patterns).

Processing to determine that the color of the pixel under consideration corresponds to color_index (= 1 to 4) is almost the same as that of procedure 2, so that the description thereof will be omitted.

First, the feature determining unit 1303 checks whether or not pixels in the proximity of a subimage pixel position x_s, y_s), namely, pixels (x', y') within a range of two pixels apart from the pixel (x_s, y_s) in the x-axis direction and the y-axis direction have a color matching the color designated by color_index. The pixels (x', y') are within a range of two pixels apart from the pixel (x_s, y_s) under consideration, for example, and are represented by |x - x'| ≤ 2 and |y - y'| ≤ 2. The feature determining unit 1303 then counts the pixels existing in the neighbor of the pixel and having the color matching that of color_index. The feature determining unit 1303 then divides the number of pixels by the total number of pixels in the proximity of the pixel to thereby obtain a mean value. If the mean value exceeds the threshold parameter (th_s), the feature c is set to one. Otherwise, the feature c is set to zero.

The feature determining unit 1303 also can determine the feature c of a pixel under consideration using a procedure described below in addition to the above-described three procedures.

First, the feature determining unit 1303 conducts an HSV conversion for the R, G, and B values of each pixel in a subimage including a pixel under consideration, and then converts these values into values of hue (H = 0 to 1), saturation (S = 0 to 1), and value (V = 0 to 1). The feature determining unit 1303 then represents the H, S, and V values in, for example, five levels. If, for example, each of these values of the pixel under consideration is equal to or less than 0.2 (= 1/5), the color of the pixel is represented as (1, 1, 1). If, the H and S values are equal to or less than 0.2 and the V value is 0.2 < V ≤ 0.4, the color of the pixel is expressed as (1, 1, 2). When a pixel at a position (x, y) in the subimage specified by (x, y, H', S', V') received from the feature candidate generator unit 102 as the feature parameter set has a color represented as (H', S', V'), the feature c is "1". Otherwise, the feature c is "0".

As above, when the feature c is calculated for each subimage using the s-th feature candidate (feature parameter set), the feature determining unit 1303 calculates a mutual information quantity MI obtained from the s-th feature candidate according to the following expression (3), and then stores the quantity MI together with the identification number s of the feature candidate (step S1405).

$\begin{matrix}MI \left[Q;C\right] = H \left[Q\right] - < H \left[Q\left|c\right|\right] > c \\ where \\ H \left[Q\right] = - {\sum }_{q} P \left(q\right) logP \left(q\right) , P \left(q\right) = M \left(q\right) / M \\ H \left[Q|c\right] = - {\sum }_{q} P \left(q|c\right) ⁢ logP \left(q|c\right) , P \left(q|c\right) = M \left(q|c\right) / M \left(c\right)\end{matrix}$

In this connection, Q is a set of classes {q = 0, q = 1} and M indicates the total number of subimages. M (q) is the total number of subimages belonging to a class q, M (c) indicates the total number of subimages of which the feature is c, and M (q, c) is the total number of subimages of which the feature is c and which belongs to the class q.

In expression (3), <H[Q| c]>c indicates an averaging operation with respect to c and is calculated using the following expression (4).

$\begin{matrix}< H \left[Q|c\right] ⁢ {>}_{c} = - {\sum }_{c} P \left(c\right) ⁢ H \left[Q|c\right] , \\ P \left(c\right) = M \left(c\right) / M\end{matrix}$

Thereafter, a next feature candidate, i.e., an (s + 1)-th feature candidate is received from the feature candidate generator unit 1302, and is repeatedly processed in the same manner (steps S1402 to S1405). When the calculation of the mutual information quantity MI is completed for all of the feature candidates (N candidates), the feature determining unit 1303 compares the mutual information quantities MI of the respective feature candidates with each other. Then, the feature determining unit 1303 determines the feature candidate having a maximum value of the mutual information quantity Max MI[Q;C] as a first feature of a feature set to be determined (step S1406).

After the determination of the first feature, the feature determining unit 1303 determines a second feature. In the same manner as described above, the feature determining unit 1303 sequentially receives a feature candidate from the feature candidate generator unit 1302 (step S1402) and calculates the feature c according to each subimage (steps S 1403 and S1404). Alternatively, depending on the available storage capacity, a calculation result of the feature c in step S1404 at determination of the first feature may be stored, and the feature determining unit 1303 may read the stored data (feature candidate). When the feature c is calculated for each subimage, the feature determining unit 1303 calculates, using the s-th feature parameter set, a mutual information quantity MI₂ obtained from the s-th feature candidate according to the following expression (5) under a condition that the already determined first feature c₁ is known, and then stores the quantity MI₂ together with an identification number s of the feature candidate (step S1405).

$\begin{matrix}{MI}_{2} \left[Q;C|{C}_{1}\right] = < H \left[Q|{c}_{1}\right] ⁢ {>}_{c ⁢ 1} - < H \left[Q|\left(c, {c}_{1}\right)\right] ⁢ {>}_{c ⁢ 1 - c} \\ where \\ H \left[Q|c⁢1\right] = - {\sum }_{q} P \left(q| {c}_{1}\right) ⁢ logP \left(q|{c}_{1}\right) , P \left(q|{c}_{1}\right) = M \left(q, {c}_{1}\right) / M \left({c}_{1}\right) \\ H \left[Q|c,c⁢1\right] = - {\sum }_{q} P \left(q|c,{c}_{1}\right) ⁢ logP \left(q|c,{c}_{1}\right) , P \left(q|c,{c}_{1}\right) = M \left(q, c, {c}_{1}\right) / M \left(c, {c}_{1}\right)\end{matrix}$

In expression (5), M (c₁) is the total number of subimages of which the first feature is c₁, M (q, c₁) indicates the total number of subimages of which the first feature is c₁ and which belongs to the class q. M (c, c₁) indicates the total number of subimages of which the feature is c and of which the first feature is c₁. M (q, c, c₁) is the total number of subimages of which the feature is c, of which the first feature is c₁, and which belongs to the class q.

Through the above operation, a next feature candidate, namely, an (s + 1)-th feature candidate is received from the feature candidate generating device 1302, and is repeatedly processed in the same manner (steps S1402 to S1405). When the calculation of the mutual information quantity MI is completed for all of the feature candidates (N candidates), the feature determining unit 1303 compares the conditional mutual information quantities MI₂ obtained from the respective feature candidates with each other, and determines the feature candidate having a maximum value of the mutual information quantity as a second feature c₂ of a feature set to be determined (step S 1406).

When an m-th feature is determined in the same manner, a feature candidate that maximizes the evaluation function MIₘ₊₁ of the following expression (6) is adopted as an (m + 1)-st feature c.

$\begin{matrix}{MI}_{m + 1} \left[Q;C|{C}_{1},{C}_{2},⋯,{C}_{m}\right] \\ = < H \left[Q|{c}_{1},{c}_{2},⋯,{c}_{m}\right] > \left(c⁢1,c⁢2,⋯,{c}_{m}\right) \\ - < H \left[Q|\left(c, {c}_{1}, {c}_{2}, ⋯, {c}_{m}\right)\right] > \left(c,c⁢1,c⁢2,⋯,cm\right)\end{matrix}$

MIₘ₊₁ represents an information quantity obtained from the feature c under a condition that the features (c₁, c₂, ..., cₘ) are known. The processing is continuously executed until the obtained information quantity (additional information quantity) is less than a predetermined threshold value MI_th even if a new feature is selected. When the threshold value MI_th is set to, for example, zero, the above-described processing procedure is repeatedly conducted to determine a next feature until the obtained information quantity (additional information quantity) becomes zero, that is, until the end condition is satisfied.

The feature determination processing is terminated when the end condition is satisfied. The parameters of each feature set determined as above are stored in the feature storage unit 1304 (step S1407).

In variations of the feature determination processing, there can be considered a procedure capable of reducing the number of feature candidates created by the feature candidate generator unit 1302 as described below. For example, for each complex Gabor function, the intra-class mean value of the values of "a" calculated using expression (2) is prepared beforehand for a class of q = 0 and a class of q = 1. Then, the threshold value MI_th is set to an intermediate value between the two intra-class mean values. Also, for example, when the mutual information quantity MI is calculated for each complex Gabor function using expression (3) at determination of the first feature, a threshold value MI_th that gives a maximum information quantity MI for each feature candidate is recorded. When the second and subsequent features are determined, the threshold value MI_th is kept immobilized in the processing.

Although a complex Gabor function is employed as a feature extraction function to obtain feature candidates in the present embodiment, another feature extraction function may be additionally employed. Depending on cases, only such feature extraction function is used to obtain feature candidates.

As a favorable variation of the feature determination processing, there may be considered, for example, a variation in which a subspace is formed for each class such that an index indicating the distance to the subspace is added to the feature candidate. Furthermore, it may also be possible to add, to the feature candidates, weighted mean luminance in a neighborhood of a point calculated using a Gaussian function. Also, it may be possible to add to the feature candidates normalized weighted mean luminance where the weighted mean luminance in a neighborhood of a point calculated using a Gaussian function is normalized by mean luminance calculated using a Gaussian function of a wider range, i.e., an index representing whether the neighborhood of the point is brighter or darker than a periphery thereof. Moreover, standard features used in diagnosis can be added to the feature candidates.

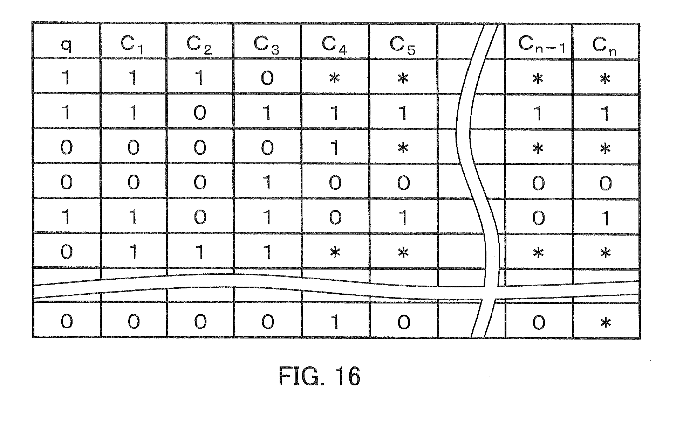

When the feature determination processing is completed and the feature sets thus determined are stored in the feature storage unit 1304, it becomes possible to produce a category table 1306 (shown in FIG. 16) for pattern discrimination. Description will now be given of processing for the category table generator unit 1305 activated by a desired unit to create the category table 1306.

The category table generator unit 1305 first receives each subimage from the learning pattern storage unit 1301 and each parameter of the feature set from the feature storage unit 1304 (hereinafter, it is assumed that the number of determined features is n in total). Then, the category table generator unit 1305 stores each subimage in the category table 1306 together with the feature values (c₁, c₂, ..., cₙ) for each subimage.

The above procedure makes it possible to produce a category table uniquely classifying each subimage. More preferably, it is desirable to use a redundant term (don't care term). For example, when a subimage can be classified using only the feature values (c₁, c₂, ..., cₙ) ranging from the first feature value to the i-th feature value, the values of the (i + 1)-st and subsequent feature vectors are replaced by a symbol indicating "don't care" in the category table.

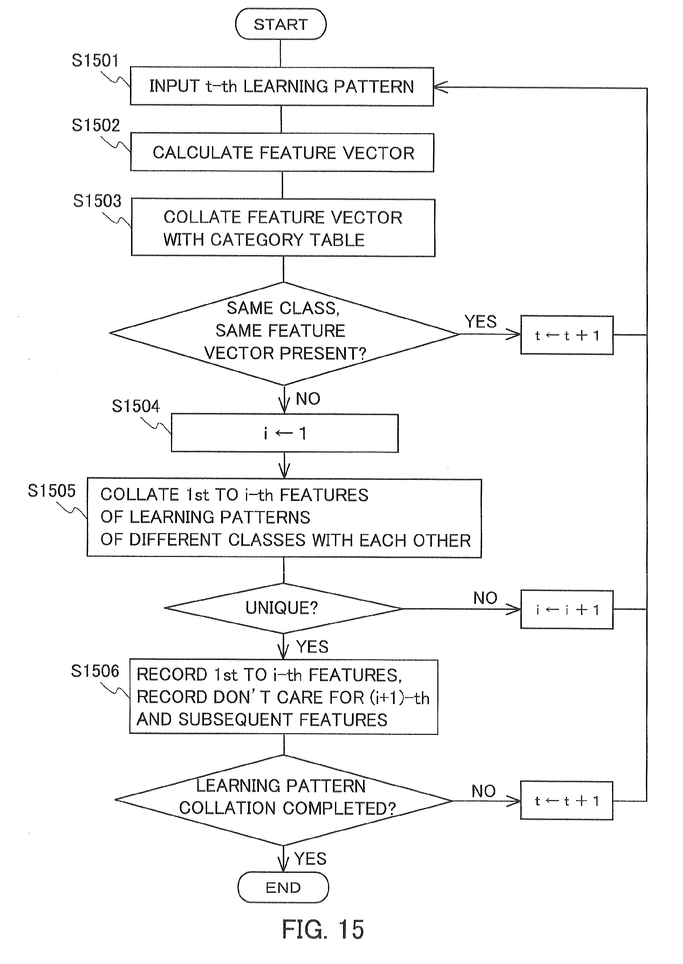

Referring next to FIG. 15, description will be given of an example of a procedure to generate a category table 1306 by use of the redundant term (don't care). FIG. 15 shows an example of the flowchart showing the procedure of creating the category table 1306 in the present embodiment.

The category table generator unit 1305 first calculates a feature vector (c₁, c₂, ..., cₙ) using the parameters of the feature set stored in the feature storage unit 1304 (steps S1501 and S1502) with respect to the subimage inputted thereto.

The category table generator unit 1305 checks whether or not the category table 1306 includes a subimage having a feature vector matching the above feature vector (step S 1503). When a field of the category table 1306 contains the symbol indicating "don't care", any value corresponding thereto is regarded as a matching value.

As a result of the determination, if the category table includes a subimage having a feature vector matching the calculated feature vector, control returns to step S1501 without recording the subimage in the category table 1306 to receive a next subimage.

On the other hand, if the matching subimage is absent, the category table generator unit 1305 sets an increment variable i to "1" (step S1504) to execute the following processing. First, the category table generator unit 1305 checks if subimages that belong to a class (for example, q = 1) different from the class to which the subimage under consideration belongs (for example, q = 0) includes a subimage that include the first to i-th features (c₁,c₂, ...,ci) all matching those of the subimage (step S1505).

As a result, if such subimage is absent, the first to i-th feature values (c₁, c₂, ..., cᵢ) are recorded in the category table together with a sign (e.g., q = 0) of the class to which the subimage belongs. The symbol indicating "don't care" is recorded as the values of the (i + 1)-st and the subsequent feature vectors (step S 1506). Control returns to step S1501 to receive a next inputted subimage.

On the other hand, if such subimage is present, one is added to the increment variable i and control returns to step S1501. That is, the increment of the value of i is successively executed until the inputted subimage becomes distinct from other subimages by the i-th feature value.

The above processing is repeatedly executed for all subimages. In the procedure, there occurs a case in which it is not possible to classify all subimages. For example, in some cases, subimages belonging to mutually different classes possess an equal feature vector. In this case, the processing may be executed in such a manner that, for example, the numbers of the subimages belonging to the respective classes are counted, and the class to which a larger number of subimages belong is determined as a class associated with the feature vector.

There may also be adopted a method in which while incrementing the value of i, patterns for which the features c₁ to cᵢ match each other are classified into a group (to sequentially obtain a group including a smaller number of subimages). When one group includes only one subimage, the (i + 1)-st and subsequent features of the subimage may be set as don't care terms.

FIG. 16 shows an example of the category table 1306 employed in the present invention. FIG. 16 shows a table storing a class identification mark (q) of each subimage and a feature vector (c₁, c₂, ..., cₙ). In FIG. 16, an asterisk "*" indicates "don't care".

Referring next to the drawings, description will be given of a diagnosis method of a pathological image using the category table.

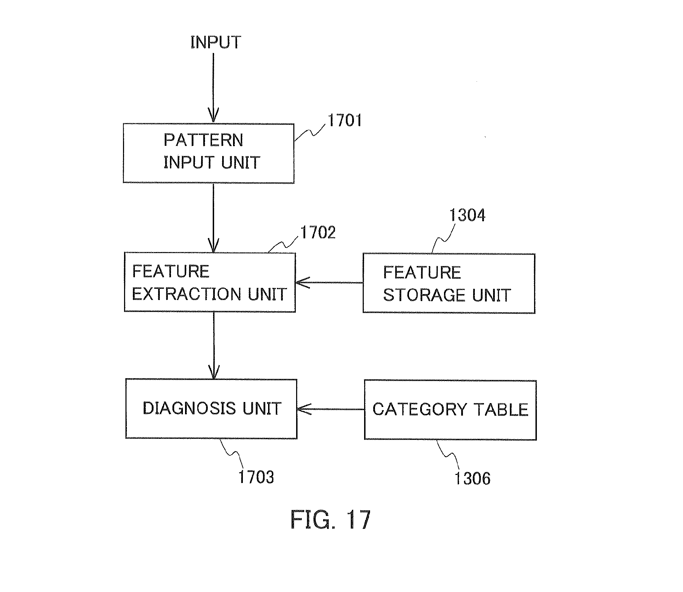

FIG. 17 is a block diagram showing a processing flow of the diagnosis method according to the present invention. FIG. 17 shows a pattern input unit 1701, a feature extraction unit 1702, and a diagnosis unit 1703. FIG. 17 also shows a feature storage unit 1304 that stores a determined feature set to be used for feature extraction by the feature extraction unit 1702 and a category table 1306 prepared in advance for the diagnosis unit 1703 to conduct diagnosis.

The pattern input unit 1701 is a unit to input a subimage from a desired medium. In the present embodiment, the pattern input unit 1701 is a unit to input an image (subimage) of a cell of a pathological tissue centered on a cell nucleus. Although images centered on a cell nucleus are inputted in the present embodiment, images are not limited thereto. For example, images used for pathological judgment or decision by a pathological expert in the diagnosis of pathological tissues, such as images of cell nuclei, pores, cytoplasm, interstitium, and the like, may be inputted as subimages.

The feature extraction unit 1702 is a unit that extracts, according to a subimage transmitted from the pattern input unit 1701, a feature of the subimage using the determined feature set.

The diagnosis unit 1703 is a unit that diagnoses information represented by the subimage, according to the feature obtained by the feature extraction unit 1702.

First, on the basis of the feature obtained by the pattern input unit 1701, a subimage is acquired to be fed to the feature extraction unit 1702.

Subsequently, the feature extraction unit 1702 calculates a feature vector of the subimage inputted thereto, and then delivers the result of the calculation to the diagnosis unit 1703. The feature vector is calculated using a feature set determined through procedures 1, 2, and 3 stored in the feature storage unit 1304, e.g., a feature set determined by the above-described feature determining procedure.

Referring to the category table 1306, the diagnosis unit 1703 retrieves therefrom an entry matching the feature vector and reads a class mark to output the mark as a diagnosis result. If the entry obtained from the category table 1306 contains "don't care", the diagnosis unit 1703 determines that the entry matches the feature vector irrespective of the value of the feature.

To further clarify an advantage of the present invention that determines a feature of a subimage and determines an image through the above-described respective procedures, description will be given of the difference between the present invention and conventional methods using a decision tree (ID3, C4.5).

Procedures of ID3 and the like are similar to those of the present invention in that the classification rule in each node of the decision tree is determined according to a criterion of information quantity maximization. However, in ID3 and C4.5, the classification rule (e.g., feature) is determined for each node. For example, when a second feature is determined after determination of a first feature c₁, the classification rule (feature) varies in the determination between when c₁ is one and when c₁ is zero. In contrast therewith, according to the present invention, if a substantially equal node depth is used, any arbitrary n-th features are determined to be equal to each other. This is a remarkable difference between the present invention and the conventional methods.

In either the present invention or in the conventional methods, the learning patterns are completely classified. However, there appears a considerable difference in generalization performance, i.e., performance of discrimination for subimages not learned yet. Assuming that the tree depths are substantially equal (n) to each other in both methods, while 2<n> features are determined in ID3 or C4.5, only n features are determined in the present invention. That is, the present invention is simpler in structure than the conventional methods. The difference between the numbers of determined features exponentially increases as the problems become more complex and the decision tree required for the procedure becomes deeper.

It has been known as "Occam's rasor" that if two classifying devices have equal performance for learning patterns, the classifying device in a simpler configuration is superior in the generalization performance. This is the reason why the feature determination method and the diagnosis method using the method according to the present invention can improve the generalization performance considerably, as compared with the conventional methods.

Description will now be given of processing to extract a subimage from a pathological image in the learning pattern input unit 1300 and the pattern input unit 1701. Although in the present embodiment, extraction of a subimage centered on a cell nucleus is described, the present invention is not limited thereto. Thus, a morphological feature part to which a pathological expert pays attention in the observation of a pathological image such as a pore, cytoplasm, and interstitium may be extracted as a subimage.

The processing to extract a subimage centered on a cell nucleus includes the step of calculating miRNA staining signals using the R, G, and B values of each pixel in a pathological image and the step of detecting a central position of the cell nucleus according to the distribution of miRNA staining signals of respective pixels in the image. Actually, processing such as processing to smooth the miRNA staining signals also is included. The following example is directed to a case where a pathological image in which the cell nucleus is stained blue by miRNA staining.

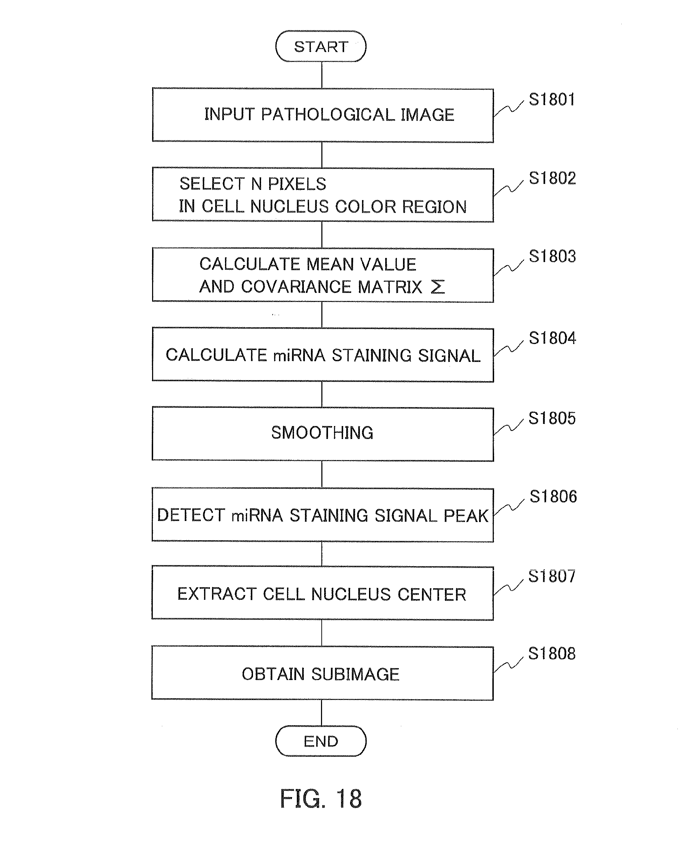

Referring next to FIG. 18, description will be given of processing to extract a subimage in the learning pattern input unit 1300 and the pattern input unit 1701.

When a pathological image is received as an input (step S1801), the learning pattern input unit 1300 and the pattern input unit 1701 assign a miRNA staining signal to each pixel of the pathological image in which the cell nucleus is stained blue.

According to the R, G, and B values of each pixel (R = 0 to 255, G = 0 to 255, B = 0 to 255 when 24 bits are used), there is calculated a miRNA staining signal that takes a value of 1.0 in a region of the blue cell nucleus and a value of 0 in the other regions (regions stained different colors). In this processing, a check is made to determine a color distribution of the cell nucleus in an RGB space represented by R, G, and B values, and the distance between the RGB value of each pixel and a center of the distribution is calculated. Specifically, the RGB value of each pixel is checked. If the value is associated with a position near the center of the color distribution of the cell nucleus in the RGB space, a miRNA staining signal having a large value near one is assigned to the pixel. If the value is at a position apart from the center of the color distribution, a miRNA staining signal having a small value near zero is assigned to the pixel. However, since the result of the staining of the cell nucleus varies between samples depending on the staining operation or the like, the color distribution of the cell nucleus is calculated in an adaptive method.

That is, by referring to the color region of the cell nucleus determined beforehand, only pixels each having the RGB value in the color region of the cell nucleus are selected as pixels representing the color of the cell nucleus.

The color region of the cell nucleus is determined beforehand in the following manner. First, images of cell nuclei in which the stained state thereof vary due to the staining operation or the like are collected. Then, the RGB value is checked for each pixel in the cell nucleus region of each image. At the same time, a check is made to determine the RGB value for pixels in regions stained in colors characteristic to cytoplasm, interstitium, and pores, for example, in the respective images. The processing then determines the color region of the cell nucleus that includes no or few regions stained in colors characteristic to cytoplasm, interstitium, and pores and that includes pixels of the cell nucleus region.

Specifically, the learning pattern input unit 1300 and the pattern input module 1701 assign a miRNA staining signal to each pixel in the following manner.

First, by referring to the color region of the cell nucleus determined beforehand, N pixels each having the RGB value in the color region of the cell nucleus are selected from the pathological image inputted to the learning pattern input unit 1300 and the pattern input unit 1701 (step S1802). It is assumed that the RGB value of each of the selected N pixels includes Ri, Gi, and Bi (i = 1 to N). Next, from Ri, Gi, and Bi of each pixel (i = 1 to N), a mean value (Ro, Go, Bo) and a covariance matrix ∑ of Ri, Gi, and Bi are calculated according to the following expression (7) (step S 1803).

$\begin{matrix}{R}_{0} = 1 / N {\sum }_{i} {R}_{i} , { G}_{0} = 1 / N {\sum }_{i} {G}_{i} , {B}_{0} = 1 / N {\sum }_{i} {B}_{i} \\ \sum = 1 / N {\sum }_{i} {\left({R}_{i}-{R}_{0},{G}_{i}-{G}_{0},{B}_{i}-{B}_{0}\right)}^{T} ⁢ \left({R}_{i}-{R}_{0},{G}_{i}-{G}_{0},{B}_{i}-{B}_{0}\right)\end{matrix}$

In expression (7), T is a symbol indicating transposition of a vector. Using the covariance matrix ∑, the distance L between each pixel (R, G, B) and the mean value (Ro, Go, Bo) and the miRNA staining signal (Hema) are calculated according to the following expression (8) (step S1804).

$\begin{matrix}1 / 2 ⁢ \left(R-{R}_{0},G-{G}_{0},B-{B}_{0}\right) ⁢ {\sum }^{- 1} ⁢ {\left(R-{R}_{0},G-{G}_{0},B-{B}_{0}\right)}^{T} \\ Hema = exp \left(-1/2⁢\left(R-{R}_{0},G-{G}_{0},B-{B}_{0}\right)⁢{\sum }^{- 1}⁢{\left(R-{R}_{0},G-{G}_{0},B-{B}_{0}\right)}^{T}\right)\end{matrix}$

Description will next be given of processing to detect a central position of the cell nucleus using the distribution of miRNA staining signals calculated for the respective pixels.

The miRNA staining signal obtained for each pixel using expression (8) is represented as Hema(-r). In the expression, -r = (x, y) indicates a position vector of a position of a pixel in the pathological image. According to the following expression (9) using a smoothing mask M_{low}, a smoothing operation is conducted for Hema(-r) (step S1805) to obtain a peak value resultant from the smoothing, and the peak value is set as the central position of the cell nucleus (steps S 1806, S 1807).

$Hemaʹ \left(r\right) = {\sum }_{\vec{r}} ⁢ Hema ⁢ \left(\vec{rʹ}-\vec{r}\right) ⁢ {M}_{low} \left(\vec{rʹ}\right)$

The smoothing mask M_{low} may be implemented using, for example, a function of expression (10)

$\begin{matrix}{M}_{low} \left(\vec{rʹ}\right) = {M}_{0} \left(\vec{rʹ}, {s}_{ex}\right) - {M}_{0} \left(\vec{rʹ}, {s}_{in}\right) , \\ {M}_{0} \left(\vec{rʹ}, s\right) = 1 / l \left(when {\left|\vec{rʹ}\right|}^{2}≦{s}^{2}\right) , O \left(otherwise\right)\end{matrix}$

The normalization factor 1/1 in expression (10) is determined according to the following expression (11).

${\sum }_{\vec{rʹ}} ⁢ {M}_{0} \left(\vec{rʹ}, s\right) = 1$

In the expression, Sₑₓ and Sᵢₙ are parameters determined beforehand. Ordinarily, Sₑₓ is set to about a value of typical size (radius) of a cell nucleus and Sᵢₙ is set to about 1.2 times that of Sₑₓ.

After the miRNA staining color signal (miR') is calculated for each pixel, if the value of miR' of a point under consideration is more than a predetermined threshold value (e.g., 0.25) and more than the value of miR' at any point in the neighborhood thereof (e.g., within three pixels from the point along the x and y coordinates), the point is detected as a peak to be set as a central point of the cell nucleus (step S 1807).

In consideration of the fact that there might be a variation in size of the cell nucleus, the miRNA staining color signal smoothing and the peak detection are performed using a plurality (e.g., three kinds) of smoothing masks having mutually different sizes (parameters Sₑₓ and Sᵢₙ). The peak position detected by either of the operations may be set as the center of the cell nucleus.

The learning pattern input unit 1300 and the pattern input unit 1701 detect the center of a cell nucleus by first executing the above-described processing for the pathological image inputted thereto. The units 1300 and 1701 then obtain a large number (equal to the number of the detected cell nucleus's centers) of images (subimages) of a predetermined size from the pathological image, where the cell nuclei are at the center of the images, to extract each of the subimages as a learning pattern or an input pattern (step S 1808).

The image diagnosis support system of the present invention also may include, for example, a unit for evaluating the effectiveness of a miRNA-stained image. By evaluating the effectiveness of a miRNA-stained image, the accuracy of cancer evaluation can be improved still further. The evaluation of the effectiveness of a miRNA-stained image in this context means the same as the evaluation of the effectiveness of a section slide subjected to the miRNA staining. An illustrative example will be given below. It is to be noted, however, that the present invention is not limited thereto.

### (Third embodiment)

The third embodiment is directed to the image diagnosis support system of the first or second embodiment further including: a correction unit that corrects the stained state of a miRNA-stained image; a nontumor cell-detecting unit that detects nontumor cells in the corrected miRNA-stained image; and a determination unit that determines the presence or absence of miRNAs staining in the detected nontumor cells.

The processing by the system of the present embodiment can be carried out in the following manner, for example. First, the stained state of the obtained miRNA-stained image is corrected. The correction is made with respect to the dye, the intensity, or the like in consideration of the state of the section slide being used, the state of another section slide stained in the same manner, the conditions under which the staining was performed, the conditions under which the image data was obtained, and the like, for example.

Then, with respect to the thus-corrected miRNA-stained image, the detection of the nontumor cell is performed. The nontumor cell can be identified based on information such as the shape and size of the cell, the shape and size of the cell nucleus, the site where it is present in a tissue, and the like, for example. This identification can be carried out by, for example, a module that has gone through machine learning based on the above-described conditions.

The detection of the nontumor cell can be carried out by, for example, obtaining a stained image using a counterstaining agent and matching this counterstained image with the miRNA-stained image. This matching may be, for example, the same as the matching of the miRNA-stained image and the HE-stained image as descried above. The counterstaining agent can be determined as appropriate depending on the kind of the sample as a subject, for example. Examples of the counterstaining agent include Kernechtrot. The kind of the nontumor cell to be detected is not particularly limited, and can be determined as appropriate depending on the kind of the sample as a subject, for example. Examples of the nontumor cell include lymphocytes, fibroblasts, and vascular endothelial cells. Depending on whether or not these cells satisfy a specified cell size and/or shape, it is possible to determine whether or not the subject is a nontumor cell.

Then, the presence or absence of miRNA staining of the detected nontumor cell in the miRNA-stained image is determined. As a result, when the nontumor cell is subjected to the miRNA staining, it is determined that this miRNA-stained image is not effective, and the processing is terminated without advancing the flow to the subsequent step. On the other hand, when the nontumor cell is not subjected to the miRNA staining, it is determined that this miRNA-stained image is effective, and the flow goes to the subsequent step, e.g., to the step of detecting a tumor area based on the miRNA staining as described above.

Unless otherwise stated, the above-described respective embodiments can be combined to each other.

### Examples

Hereinafter, the present invention will be described by way of examples. It is to be noted, however, that the present invention is not limited to the following examples.

### [Example 1]

In situ hybridization was performed using a probe, and the expression levels of hsa-miR-92a in leukocytes of acute myeloid leukemia (AML) patients (n = 4) and acute lymphocytic leukemia (ALL) patients (n = 2) were examined.

As the probe, an LNA modified probe (trade name: miRCURY-LNA detection probe, Exiqon) labeled with digoxigenin (DIG) was used. In the following, the sequence of the probe for hsa-miR-92a detection is shown in SEQ ID NO: 5, and the sequence of the probe as a negative control is shown in SEQ ID NO: 6. The sequence of the negative control probe is shown below is a sequence obtained by scrambling the sequence of the hsa-miR-92a detection probe shown below.
hsa-miR-92a detection probe (SEQ ID NO: 5)
   5'-acaggccgggacaagtgcaata-3'
Negative control probe (SEQ ID NO: 6)
   5'-gtgtaacacgtctatacgccca-3'

The in situ hybridization was carried out with a Ventana Discovery automated in situ hybridization instrument (trade name, Ventana Medical Systems) using a RiboMap in situ hybridization kit (trade name, Ventana Medical Systems). Unless otherwise stated, the in situ hybridization was carried out in accordance with the standard protocol supplied by a RiboMap application note available from Ventana Medical Systems (http: //www. ventanamed. com).

First, leukocytes were collected from whole blood of each of the leukemia patients. With respect to the thus collected leukocyte, immobilization using a paraformaldehyde immobilizing solution, paraffin embedding, and preparation of sections were performed according to generally used methods. Then, in situ hybridization was caused after deparaffinizing the section. In the in situ hybridization, first immobilization of the section having undergone the deparaffinization was carried out by incubating the slide having the section using formalin-based RiboPrep (trade name, Ventana Medical Systems) at 37°C for 30 minutes. Subsequently, the slide was incubated in a hydrochloric acid-based RiboClear solution (trade name, Ventana Medical Systems) at 37°C for 10 minutes, and then was treated with ready-to-use protease 2 (trade name, Ventana Medical Systems) at 37°C. Next, the slide was subjected to a prehybridization treatment for denaturation at 70°C for 6 minutes. After the prehybridization, the slide was treated with a RiboHyde hybridization buffer (trade name, Ventana Medical Systems) at 37°C for 6 hours, so as to cause hybridization of 2 ng of the DIG labeled* LNA modified probe per slide. The slide was then subjected to low stringent washing using 2 × RiboWash solution (trade name, Ventana Medical Systems) at 42°C for 6 minutes. Thereafter, the slide was washed using 1 × RiboFix (trade name, Ventana Medical Systems) at 37°C for 20 minutes. Subsequently, the slide was incubated together with 0.1 µg of biotin labeled anti-DIG antibody (Sigma) per slide at 37°C for 30 minutes. Then, the slide was incubated at 37°C for 16 minutes using 0.1 µg of streptavidin-alkaline phosphatase conjugate (Dako) per slide. Thereafter, using a BlueMap NBT/BCIP substrate kit (trade name, Ventana Medical Systems), the signal detection was performed at 37°C for 4 hours. Finally, adjacent slide sections were counterstained with Kernechtrot and HE, and each of the slides was covered with cover glass.

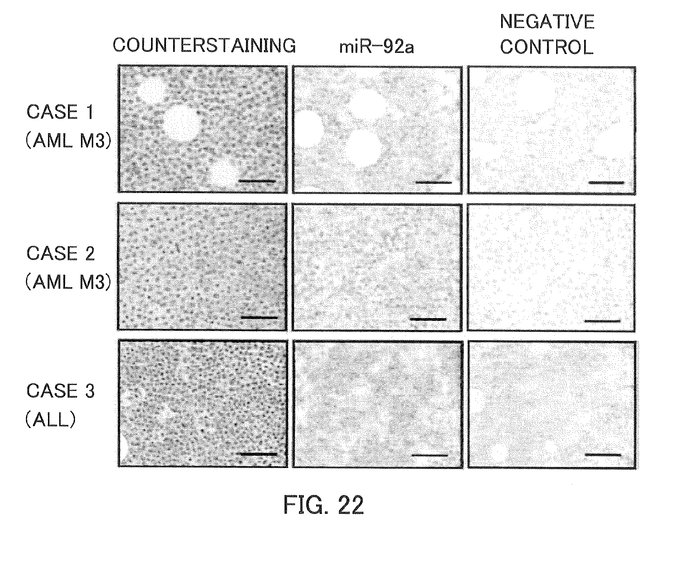

The result thereof is shown in FIG. 22. In FIG. 22, the panels in the upper row and the middle row are photographs showing the results of the staining of the leukocytes derived from the AML patients (FAB classification M3), and the panels in the lower row are photographs showing the results of the staining of the leukocytes derived from the ALL patients. The panels in the left column are photographs showing the results of counterstaining with Kernechtrot; the panels in the middle column are photographs showing the results of staining using the hsa-miR-92a detection probe, and the panels on the right column are photographs showing the results of the staining using the negative control probe. The bar in each panel is 50 µm in length.

As can be seen from FIG. 22, the staining of the cells was observed in both the AML patients and the ALL patients, and the signal intensity of the staining with the hsa-miR-92a detection probe was higher than that of the staining with the negative control probe. Although FIG. 22 shows the results obtained regarding a single AML patient and a single ALL patient, similar results were obtained in the remaining patients. On the other hand, although not shown in the drawing, the expression of hsa-miR-92a was not detected in normal leukocytes. As described above, hsa-miR-92a was expressed strongly in the leukocytes of the AML patients and the ALL patients. This demonstrates that the possibility of a cancer in a cell can be evaluated by detecting hsa-miR-92a in leukocytes.

The region stained using the hsa-miR-92a detection probe was the same as the region stained with Kernechtrot. According to the Kernechtrot staining, a canceration region can be stained. Thus, from this result, it can be said that the possibility of a cancer can be evaluated by detecting hsa-miR-92a.

### [Example 2]

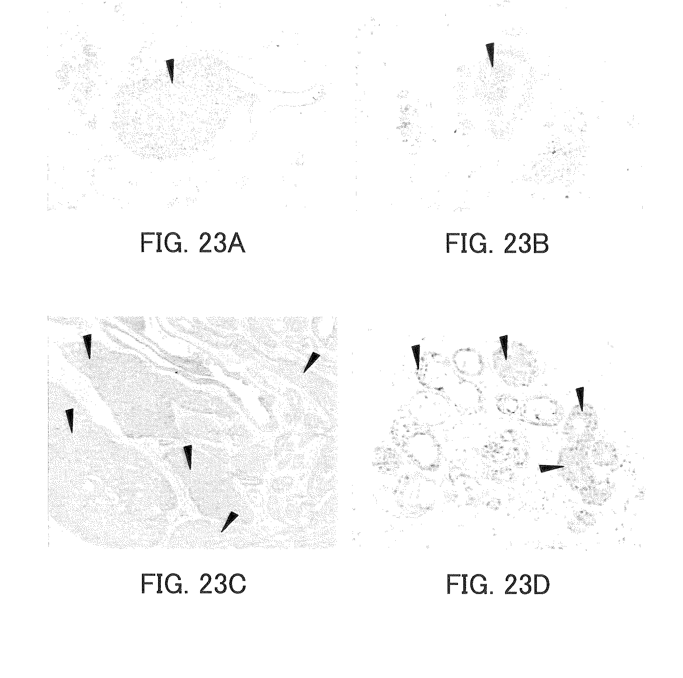

The preparation of sections and detection of hsa-miR-92a by in situ hybridization were carried out in the same manner as in Example 1, except that tissues collected from breasts were used. The results thereof are shown in FIG. 23. FIGs. 23A to 23D are photographs showing the results of miRNA staining with respect to different parts of the tissues collected from the breasts.

In each of FIGs. 23A to 23D, a part(s) of the stained portion is indicated with an arrow. As can be seen from these drawings, the staining of miRNA was observed.

While the present invention has been described above with reference to illustrative embodiments and examples, the present invention is by no means limited thereto. Various changes and modifications that may become apparent to those skilled in the art may be made in the configuration and specifics of the present invention without departing from the scope of the present invention.

This application claims priority from Japanese Patent Application No. 2009-103332 filed on April 21, 2009. The entire disclosure of this Japanese patent application is incorporated herein by reference.

### [Example 3]

Except that tissues collected from hepatocytes were used, the preparation of sections and detection of hsa-miR-92a by in situ hybridization were carried out the in the same manner as in Example 1.

The hepatocytes were collected from hepatocellular carcinoma (HCC) patients (n = 22) and nonneoplastic liver cirrhosis (LC) patients (n = 5). The hepatocytes of the HCC patients were collected from HCC patients with various ages, sexes, kinds of hepatitis virus, clinical stages, and degrees of tumor differentiation.

As a result, in the hepatocytes of nonneoplastic LC patients, the staining with the hsa-miR-92a detection probe substantially was not observed. In contrast, in the hepatocytes of all the HCC patients, remarkable staining with the hsa-miR-92a detection probe was observed.

Among the hepatocytes derived from the 22 HCC patients, FIG. 24 shows the results of the staining regarding the hepatocytes of two representative examples (case 1 and case 2). FIG. 24 shows photographs showing the results of the staining of the hepatocytes derived from the HCC patients. The panels in the upper row and the middle row show the results regarding the hepatocytes of case 1, and the panels in the lower row shows the results regarding the hepatocytes of case 2. The panels in the left column are photographs showing the results of counterstaining with Kernechtrot and HE, the panels in the middle column are photographs showing the results of the staining using the hsa-miR-92a detection probe, and the panels on the right column are photographs showing the results of the staining using the negative control probe. The bar in each panel is 100 µm in length. The panels in the middle row are magnified views of the panels in the upper row. In each panel, strongly colored portions are stained portions, and lightly colored portions are unstained portions.

As can be seen from FIG. 24, the staining with the hsa-miR-92a detection probe was observed in both case 1 and case 2. This demonstrates that the hsa-miR-92a is expressed in the hepatocytes of the HCC patients. Although FIG. 24 only shows the results of the two cases, similar results were obtained in the hepatocyte derived from the remaining HCC patients.

The region stained with the hsa-miR-92a detection probe was the same as the regions stained with Kemechtrot and HE. According to the Kemechtrot staining, a canceration region can be stained. Thus, also from this result, it can be said that the possibility of a cancer can be evaluated by detecting hsa-miR-92a.

Furthermore, RNAs were collected from hepatocytes (n = 5) of the HCC patients and hepatocytes (n = 5) of the LC patients, and the amounts of the hsa-miR-92a expressed was measured by quantitative RT-PCR (qRT-PCR). Furthermore, as an endogenous control, the amount of RNU48 expressed was measured in the same manner. Then, the ratio (hsa-miR-92a/RNU48) of the amount of hsa-miR-92a expressed to the amount of RNU48 expressed was calculated as the expression level of hsa-miR-92a. As a result, it was found that the expression of the hsa-miR-92a was significant in the hepatocytes derived from the HCC patients than in the hepatocytes derived from the LC patients.

As described above, hsa-miR-92a was expressed strongly in the hepatocytes of the HCC patients. This demonstrates that the possibility of a cancer in a cell can be evaluated by detecting hsa-miR-92a in hepatocytes.

### Industrial Applicability

According to the present invention, by detecting the expression level of the cancer marker of the present invention in a sample, it becomes possible to determine the presence or absence of cancer development or cancer progression with excellent reliability, for example. Still further, by associating the evaluation method of the present invention with, for example, cancer evaluation by conventional HE staining or the like, cancer evaluation with still higher reliability becomes possible.

### Explanation of reference numerals

- 111:: input device
- 112:: output device
- 113:: stained image database
- 120:: processing device
- 121:: input acceptance processing section
- 122:: information obtaining section
- 123:: image matching processing section
- 124:: tumor area extracting section
- 125:: staining positive cell content rate calculating section
- 130:: storage device
- 131, 132, 133, 134, 135, 136, 1231:: storage section
- 725:: staining positive cell content rate and staining intensity calculating section
- 1126:: tumor determining and tumor area calculating section
- 1213:: slide database
- 1214:: slide device
- 1222:: slide imaging section
- 1300:: learning pattern input unit
- 1301:: learning pattern storage unit
- 1302:: feature candidate generator unit
- 1303:: feature determining unit
- 1304:: feature storage unit
- 1305:: category table generator unit
- 1306:: category table
- 1701:: pattern input unit
- 1702:: feature extraction unit
- 1703:: diagnosis unit
- 190:: image diagnosis support device
- 191:: processing section
- 192:: storage section
- 193:: microscope
- 194:: CCD
- 195:: scanner
- 196:: display
- 2001:: image obtaining unit
- 2002:: information obtaining unit
- 2003:: matching unit
- 2004:: tumor area specifying unit
- 2005:: calculating unit

### [Sequence Listing]

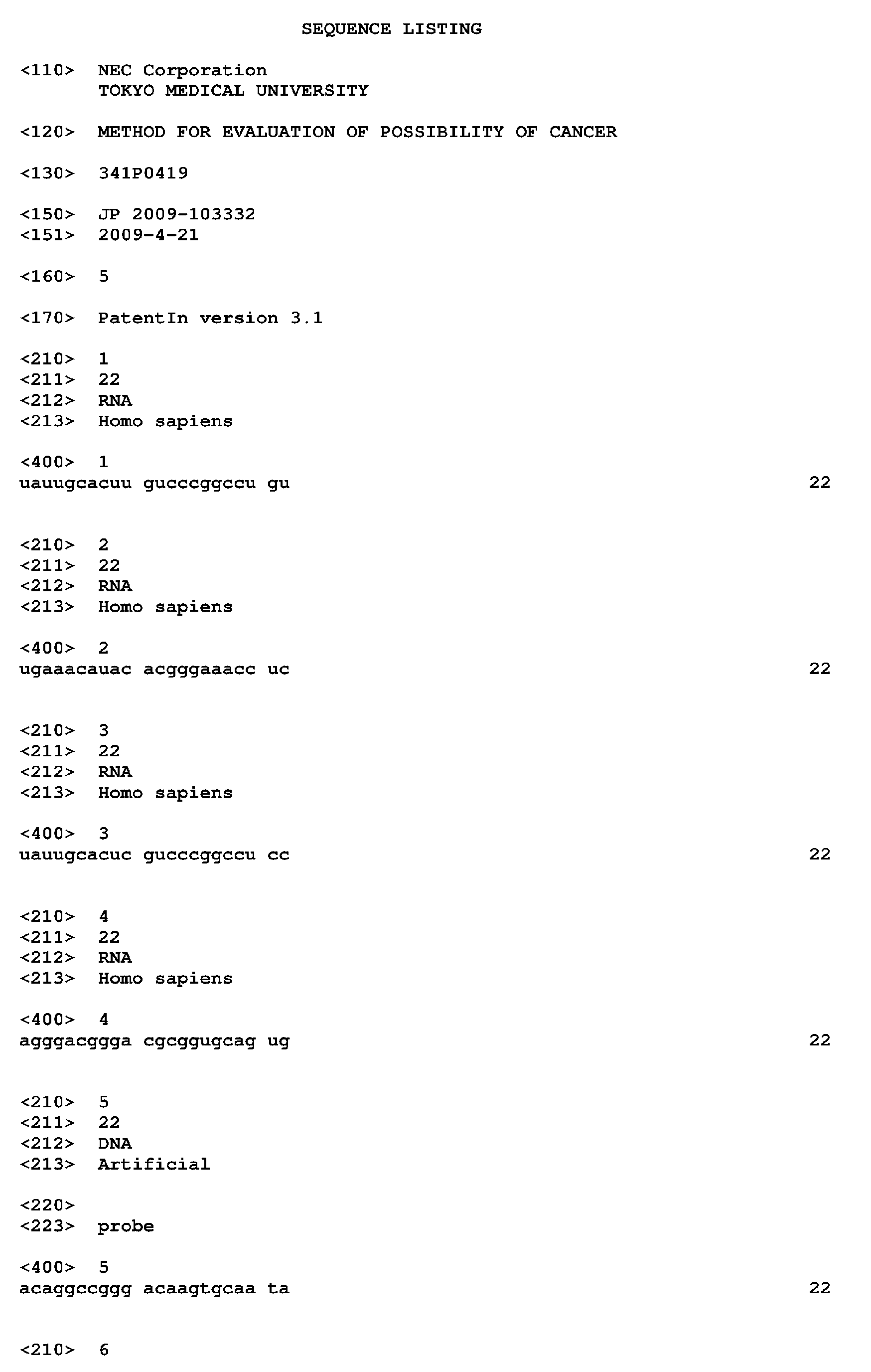

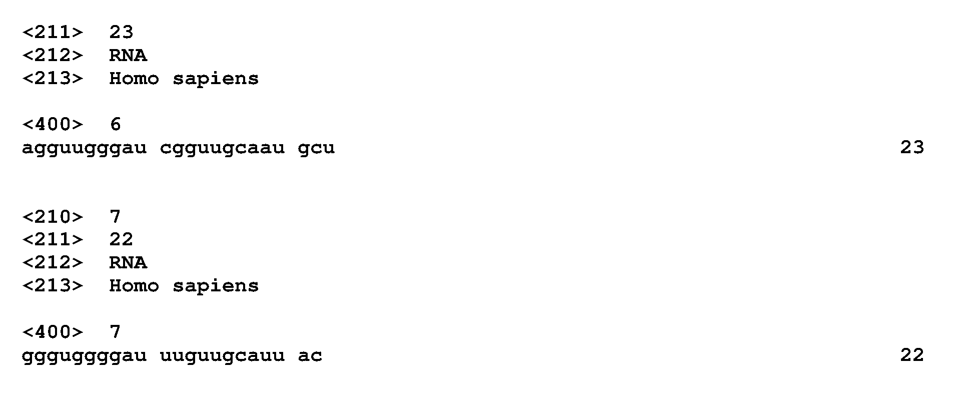

## Claims

1. An evaluation method for evaluating a possibility of a cancer, the evaluation method comprising the steps of:
detecting a cancer marker in a sample; and
evaluating a possibility of a cancer in the sample based on an expression level of the cancer marker, wherein
the sample is a cell or a tissue, and
the cancer marker comprises at least one miRNA selected from hsa-miR-92 and hsa-miR-494.

2. The evaluation method according to claim 1, wherein the hsa-miR-92 is at least one selected from the group consisting of hsa-miR-92a, hsa-miR-92a*, hsa-miR-92b, and hsa-miR-92b*.

3. The evaluation method according to claim 2, wherein the hsa-miR-92a is at least one of hsa-miR-92a-1 and hsa-miR-92a-2.

4. The evaluation method according to claim 2, wherein the hsa-miR-92a* is at least one of hsa-miR-92a-1* and hsa-miR-92a-2*.

5. The evaluation method according to claim 1, wherein the miRNA is hsa-miR-92a.

6. The evaluation method according to any one of claims 1 to 5, wherein the cancer is at least one cancer selected from the group consisting of colon cancer, rectal cancer, gallbladder cancer, stomach cancer, breast cancer, leukemia, pancreas cancer, liver cancer, brain tumor, and osteosarcoma.

7. The evaluation method according to any one of claims 1 to 6, wherein the evaluation method determines the presence or absence of cancer onset, a stage of cancer progression, or a prognosis state.

8. The evaluation method according to any one of claims 1 to 7, wherein, in the cancer marker detecting step, the cancer marker is visualized by at least one selected from the group consisting of color development, fluorescence, and autoradiography.

9. The evaluation method according to any one of claims 1 to 8, wherein the sample is immobilized, and the cancer marker is detected by an in situ hybridization method.

10. The evaluation method according to any one of claims 1 to 9, wherein the expression level of the cancer marker is represented by an amount of the cancer marker expressed in the sample.

11. The evaluation method according to any one of claims 1 to 10, wherein, based on the expression level of the cancer marker detected in the evaluation step, the possibility of the cancer is evaluated by at least one method selected from the group consisting of the following (1), (2), and (3):
(1) the expression level of the cancer marker in the sample of a subj ect is compared with an expression level of the cancer marker in a sample of a normal subject, and when the expression level in the subject is higher than the expression level in the normal subject, it is determined that the subject has a high possibility of the cancer;
(2) the expression level of the cancer marker in the sample of a subject is compared with an expression level of the cancer marker in a sample of a normal subject, and as the expression level in the subject becomes relatively higher than the expression level in the normal subject, it is determined that the cancer in the subject is relatively advanced; and
(3) the expression level of the cancer marker in the sample of a subject is compared with an expression level of the cancer marker in a sample of each of cancer patients at different progression stages, and it is determined that the cancer in the subject is in the same progression stage as the cancer in the patient exhibiting the same or a similar expression level.

12. The evaluation method according to any one of claims 9 to 11, wherein,
in the cancer marker detecting step, a cancer marker-stained image with the cancer marker being stained is obtained regarding the immobilized sample, and
the evaluation method further comprises:
an HE-stained image obtaining step of obtaining an HE-stained image regarding the immobilized sample;
an information obtaining step of obtaining information of a tumor area in the HE-stained image,
a matching step of calculating a matching position of the HE-stained image obtained in the HE image obtaining step and the cancer marker-stained image obtained in the cancer marker detecting step;
a specifying step of specifying a tumor area in the cancer marker-stained image based on the information of the tumor area in the HE-stained image obtained in the information obtaining step and information of the matching position calculated in the matching step; and
a staining positive cell detecting step of detecting a staining positive cell in the tumor area in the cancer marker-stained image based on information of the tumor area in the cancer marker-stained image specified in the specifying step.

13. The evaluation method according to claim 12, wherein the staining positive cell detecting step is a calculating step of calculating a staining positive cell content rate in the tumor area in the cancer marker-stained image based on the information of the tumor area in the cancer marker-stained image specified in the specifying step.
